# EUROPEAN PATENT SPECIFICATION

(11) **EP 1 989 299 B1**
(45) Date of publication and mention of the grant of the patent: **11.04.2012**
(21) Application number: 07734243.4
(22) Date of filing: 13.02.2007
(51) Int. Cl.: C12N 9/22, C12N 15/55, C12N 15/90, A61K 48/00

(54) **MEGANUCLEASE VARIANTS CLEAVING A DNA TARGET SEQUENCE FROM A XERODERMA PIGMENTOSUM GENE AND USES THEREOF**
EINE DNA-ZIELSEQUENZ VON EINEM XERODERMA PIGMENTOSUM-GEN ABSPALTENDE MEGANUKLEASEVARIANTEN UND IHRE VERWENDUNG
VARIANTS DE MÉGANUCLÉASES COUPANT UNE SÉQUENCE D'ADN CIBLE D'UN GÈNE DE XÉRODERMA PIGMENTOSUM ET LEURS UTILISATIONS

(30) Priority: 13.02.2006 WO PCT/IB2006/000589
(43) Date of publication of application: 12.11.2008
(73) Proprietor: CELLECTIS, 93230 Romainville (FR)
(72) Inventor: ARNOULD, Sylvain, F-93200 Saint-denis (FR); PEREZ-MICHAUT, Christophe, F-75016 Paris (FR); SMITH, Julianne, F-92350 Le Plessis Robinson (FR)
(74) Representative: Leblois-Préhaud, Hélène Marthe Georgette
(86) International application number: PCT/IB2007/000924
(87) International publication number: WO 2007/093918

(56) References cited:
- WO-A-2004/067736
- WO-A-2007/057781
- WO-A-2007/093836
- US-A- 6 010 908
- MAGNALDO THIERRY ET AL: "Xeroderma pigmentosum: from symptoms and genetics to gene-based skin therapy." CELLS, TISSUES, ORGANS. 2004, vol. 177, no. 3, 2004, pages 189-198, XP008065613 ISSN: 1422-6405 cited in the application
- HENGGE U R: "Progress and prospects of skin gene therapy: a ten year history" CLINICS IN DERMATOLOGY, J.B. LIPPINCOTT, PHILADELPHIA, PA, US, vol. 23, no. 1, January 2005 (2005-01), pages 107-114, XP004749327 ISSN: 0738-081X cited in the application
- ARNOULD ET AL: "Engineering of Large Numbers of Highly Specific Homing Endonucleases that Induce Recombination on Novel DNA Targets" JOURNAL OF MOLECULAR BIOLOGY, LONDON, GB, vol. 355, no. 3, 20 January 2006 (2006-01-20), pages 443-458, XP005206991 ISSN: 0022-2836 cited in the application
- CHAMES PATRICK ET AL: "In vivo selection of engineered homing endonucleases using double-strand break induced homologous recombination" NUCLEIC ACIDS RESEARCH, vol. 33, no. 20, 2005, XP002378874 ISSN: 0305-1048
- SUSSMAN D ET AL: "Isolation and Characterization of New Homing Endonuclease Specificities at Individual Target Site Positions" JOURNAL OF MOLECULAR BIOLOGY, LONDON, GB, vol. 342, no. 1, 3 September 2004 (2004-09-03), pages 31-41, XP004563015 ISSN: 0022-2836 cited in the application
- SELIGMAN L M ET AL: "Mutations altering the cleavage specificity of a homing endonuclease" NUCLEIC ACIDS RESEARCH, OXFORD UNIVERSITY PRESS, SURREY, GB, vol. 30, no. 17, 1 September 2002 (2002-09-01), pages 3870-3879, XP002282592 ISSN: 0305-1048
- JURICA MELISSA S ET AL: "DNA recognition and cleavage by the LAGLIDADG homing endonuclease I-CreI" MOLECULAR CELL, vol. 2, no. 4, October 1998 (1998-10), pages 469-476, XP007900721 ISSN: 1097-2765 cited in the application
- SMITH JULIANNE ET AL: "A combinatorial approach to create artificial homing endonucleases cleaving chosen sequences." NUCLEIC ACIDS RESEARCH 2006, vol. 34, no. 22, 27 November 2006 (2006-11-27), page e149, XP002457876 ISSN: 1362-4962
- ARNOULD ET AL: "Engineered I-CreI Derivatives Cleaving Sequences from the Human XPC Gene can Induce Highly Efficient Gene Correction in Mammalian Cells" JOURNAL OF MOLECULAR BIOLOGY, LONDON, GB, vol. 371, no. 1, 9 July 2007 (2007-07-09), pages 49-65, XP022145891 ISSN: 0022-2836

### SEQUENCE LISTING

<110> CELLECTIS
   ARNOULD, Sylvain
   PEREZ-MICHAUT, Christophe
   SMITH, Julian
<120> MEGANUCLEASE VARIANTS CLEAVING A DNA TARGET SEQUENCE FROM A
   XERODERMA PIGMENTOSUM GENE AND USES THEREOF
<130> HLPcp1546/11
<160> 228
<170> PatentIn version 3.3
<210> 1
   <211> 22
   <212> DNA
   <213> Artificial
<220>
   <223> 22 pb target sequence
<400> 1
   ccagacgcgg aggcctggtg ga 22
<210> 2
   <211> 22
   <212> DNA
   <213> Artificial
<220>
   <223> 22 pb target sequence
<400> 2
   tcttacttgt cactggggtc ca 22
<210> 3
   <211> 22
   <212> DNA
   <213> Artificial
<220>
   <223> 22 pb target sequence
<400> 3
   caccatctga agagaggggc ta 22
<210> 4
   <211> 22
   <212> DNA
   <213> Artificial
<220>
   <223> 22 pb target sequence
<400> 4
   ctgtctgggt tgtgtgggtt aa 22
<210> 5
   <211> 22
   <212> DNA
   <213> Artificial
<220>
   <223> 22 pb target sequence
<400> 5
   tctgcctgtg aagccagtgg ag 22
<210> 6
   <211> 22
   <212> DNA
   <213> Artificial
<220>
   <223> 22 pb target sequence
<400> 6
   tgagacatat cttcggaggg cg 22
<210> 7
   <211> 22
   <212> DNA
   <213> Artificial
<220>
   <223> 22 pb target sequence
<400> 7
   tcaaacctgg tgaagtggta ag 22
<210> 8
   <211> 22
   <212> DNA
   <213> Artificial
<220>
   <223> 22 pb target sequence
<400> 8
   cgggctgggg aaagtaggac ag 22
<210> 9
   <211> 22
   <212> DNA
   <213> Artificial
<220>
   <223> 22 pb target sequence
<400> 9
   tttaattact ttcttaggat aa 22
<210> 10
   <211> 22
   <212> DNA
   <213> Artificial
<220>
   <223> 22 pb target sequence
<400> 10
   caaaatttct tattctgttt aa 22
<210> 11
<211> 22
   <212> DNA
   <213> Artificial
<220>
   <223> 22 pb target sequence
<400> 11
   caagcccatg acctatgtgg tg 22
<210> 12
   <211> 22
   <212> DNA
   <213> Artificial
<220>
   <223> 22 pb target sequence
<400> 12
   cgagatgtca cacagaggta cg 22
<210> 13
   <211> 22
   <212> DNA
   <213> Artificial
<220>
   <223> 22 pb target sequence
<400> 13
   tgacccgcaa gtgccgggtt ga 22
<210> 14
   <211> 22
   <212> DNA
   <213> Artificial
<220>
   <223> 22 pb target sequence
<400> 14
   ctgtctggcc tttgcagttt ca 22
<210> 15
   <211> 22
   <212> DNA
   <213> Artificial
<220>
   <223> 22 pb target sequence
<400> 15
   tggcctttgc agtttcaggc ta 22
<210> 16
   <211> 22
   <212> DNA
   <213> Artificial
<220>
   <223> 22 pb target sequence
<400> 16
   tttgcccact gccattggct ta 22
<210> 17
   <211> 22
   <212> DNA
   <213> Artificial
<220>
   <223> 22 pb target sequence
<400> 17
   ccatctatcc cgagacagct cg 22
<210> 18
   <211> 22
   <212> DNA
   <213> Artificial
<220>
   <223> 22 pb target sequence
<400> 18
   tgtcccgcat tcccgcagtg gg 22
<210> 19
   <211> 22
   <212> DNA
   <213> Artificial
<220>
   <223> 22 pb target sequence
<400> 19
   ctaaccgtgc tcggaaagcc cg 22
<210> 20
   <211> 22
   <212> DNA
   <213> Artificial
<220>
   <223> 22 pb target sequence
<400> 20
   ttcccctgct ggccaaatgc tg 22
<210> 21
   <211> 22
   <212> DNA
   <213> Artificial
<220>
   <223> 22 pb target sequence
<400> 21
   ctgtcttcca caaactgggg ag 22
<210> 22
   <211> 22
   <212> DNA
   <213> Artificial
<220>
   <223> 22 pb target sequence
<400> 22
   tctgctcatc agggagaggc tg 22
<210> 23
   <211> 22
   <212> DNA
   <213> Artificial
<220>
   <223> 22 pb target sequence
<400> 23
   cccaccactg ccacctgtcc ag 22
<210> 24
   <211> 22
   <212> DNA
   <213> Artificial
<220>
   <223> Xa.1 target
<400> 24
   ctgccttttt tgaaggatcc ta 22
<210> 25
   <211> 24
   <212> DNA
   <213> Artificial
<220>
   <223> C1221 I-CreI site
<400> 25
   tcaaaacgtc gtacgacgtt ttga 24
<210> 26
   <211> 501
   <212> DNA
   <213> Artificial
<220>
   <223> ADNc I-CreI N75 scaffold protein
<400> 26
   atggccaata ccaaatataa caaagagttc ctgctgtacc tggccggctt tgtggacggt    60
   gacggtagca tcatcgctca gattaaacca aaccagtctt ataagtttaa acatcagcta   120
   agcttgacct ttcaggtgac tcaaaagacc cagcgccgtt ggtttctgga caaactagtg   180
   gatgaaattg gcgttggtta cgtacgtgat cgcggatccg tttccaacta catcttaagc   240
   gaaatcaagc cgctgcacaa cttcctgact caactgcagc cgtttctgaa actgaaacag   300
   aaacaggcaa acctggttct gaaaattatc gaacagctgc cgtctgcaaa agaatccccg   360
   gacaaattcc tggaagtttg tacctgggtg gatcagattg cagctctgaa cgattctaag   420
   acgcgtaaaa ccacttctga aaccgttcgt gctgtgctgg acagcctgag cgagaagaag   480
   aaatcctccc cggcggccga c                                              501
<210> 27
   <211> 68
   <212> DNA
   <213> Artificial
<220>
   <223> primer Ulib4rev
<400> 27
   ctgaaaggtc aagcttagmb batgtttaaa cttmbbagac tgmbbtggtt taatctgagc    60
   gatgatgc                                                              68
<210> 28
   <211> 68
   <212> DNA
   <213> Artificial
<220>
   <223> primer Ulib5rev
<400> 28
   ctgaaaggtc aagcttagmb batgtttaaa cttataagac tgmbbtggmb baatctgagc 60
   gatgatgc 68
<210> 29
   <211> 18
   <212> DNA
   <213> Artificial
<220>
   <223> primer Ulib456for
<400> 29
   ctaagcttga cctttcag 18
<210> 30
   <211> 24
   <212> DNA
   <213> Artificial
<220>
   <223> DNA target
<400> 30
   tcgggacgtc gtacgacgtc ccga 24
<210> 31
   <211> 24
   <212> DNA
   <213> Artificial
<220>
   <223> 10TGC_P target
<400> 31
   tctgcacgtc gtacgacgtg caga 24
<210> 32
   <211> 24
   <212> DNA
   <213> Artificial
<220>
   <223> 10AGC_P target
<400> 32 24
   tcaggacgtc gtacgacgtc ctga 24
<210> 33
   <211> 24
   <212> DNA
   <213> Artificial
<220>
   <223> 5TTT_P target
<400> 33 24
   tcaaaacttt gtacaaagtt ttga 24
<210> 34
   <211> 24
   <212> DNA
   <213> Artificial
<220>
   <223> 5CCT_P target
<400> 34 24
   tcaaaaccct gtacagggtt ttga 24
<210> 35
   <211> 22
   <212> DNA
   <213> Artificial
<220>
   <223> Xa.2 target
<400> 35 22
   ctgccttttg tacaggatcc ta 22
<210> 36
   <211> 22
   <212> DNA
   <213> Artificial
<220>
   <223> Xa.3 target
<400> 36 22
   ctgccttttg tacaaaaggc ag 22
<210> 37
   <211> 22
   <212> DNA
   <213> Artificial
<220>
   <223> Xa.4 target
<400> 37
   taggatcctg tacaggatcc ta 22
<210> 38
   <211> 24
   <212> DNA
   <213> Artificial
<220>
   <223> Natural I-CreI site
<400> 38
   tcaaaacgtc gtgagacagt ttgg 24
<210> 39
   <211> 49
   <212> DNA
   <213> Artificial
<220>
   <223> oligonucleotide
<220>
   <221> misc_feature
   <222> (15)..(17)
   <223> n = a, g, c or t
<220>
   <221> misc_feature
   <222> (32)..(34)
   <223> n = a, g, c or t
<400> 39
   ggcatacaag tttcnnnacg tcgtacgacg tnnngacaat cgtctgtca 49
<210> 40
   <211> 26
   <212> DNA
   <213> Artificial
<220>
   <223> primer Gal10F
<400> 40
   gcaactttag tgctgacaca tacagg 26
<210> 41
   <211> 24
   <212> DNA
   <213> Artificial
<220>
   <223> primer Gal10R
<400> 41
   acaaccttga ttgcagactt gacc 24
<210> 42
   <211> 50
   <212> DNA
   <213> Artificial
   <220>
   <223> oligo
<400> 42
   tggcatacaa gtttctgcct tttgtacaaa aggcagacaa tcgtctgtca 50
<210> 43
   <211> 15
   <212> DNA
   <213> Artificial
<220>
   <223> primer assF
<220>
   <221> misc_feature
   <222> (4)..(6)
   <223> nnn code for residue 40 of the I-CreIcoding sequence
<400> 43
   ctannnttga ccttt 15
<210> 44
   <211> 15
   <212> DNA
   <213> Artificial
<220>
   <223> primer assR
<220>
   <221> misc_feature
   <222> (10)..(12)
   <223> nnn code for the residue 40 of the I-creI coding sequence
<400> 44
   aaaggtcaan nntag 15
<210> 45
   <211> 22
   <212> DNA
   <213> Artificial
<220>
   <223> 22 pb target sequence
<400> 45
   ctgcctgcct cggtgcgggc ga 22
<210> 46
   <211> 22
   <212> DNA
   <213> Artificial
<220>
   <223> 22 pb target sequence
<400> 46
   cagactggct cgtgcaagcc ga 22
<210> 47
<211> 22
   <212> DNA
   <213> Artificial
<220>
   <223> 22 pb target sequence
<400> 47
   tttacttact tttgtaggca tg 22
<210> 48
   <211> 22
   <212> DNA
   <213> Artificial
<220>
   <223> 22 pb target sequence
<400> 48
   taggacctgt tatggaattt ga 22
<210> 49
   <211> 22
   <212> DNA
   <213> Artificial
<220>
   <223> 22 pb target sequence
<400> 49
   taatctgttt tcagagatgc tg 22
<210> 50
   <211> 22
   <212> DNA
   <213> Artificial
<220>
   <223> 22 pb target sequence
<400> 50
   tgaaactcta cttaaagtta ca 22
<210> 51
   <211> 22
   <212> DNA
   <213> Artificial
<220>
   <223> 22 pb target sequence
<400> 51
   taagctcaaa cctcacagta cg 22
<210> 52
   <211> 22
   <212> DNA
   <213> Artificial
<220>
   <223> 22 pb target sequence
<400> 52
   tacgacattt ctgaaaagca tg 22
<210> 53
   <211> 22
   <212> DNA
   <213> Artificial
<220>
   <223> 22 pb target sequence
<400> 53
   cagaattgcg gcgagcagta ag 22
<210> 54
   <211> 22
   <212> DNA
   <213> Artificial
<220>
   <223> 22 pb target sequence
<400> 54
   tgacctgttt tatagaattt ta 22
<210> 55
   <211> 22
   <212> DNA
   <213> Artificial
<220>
   <223> 22 pb target sequence
<400> 55
   taatacttca gtaataatta tg 22
<210> 56
   <211> 22
   <212> DNA
   <213> Artificial
<220>
   <223> 22 pb target sequence
<400> 56
   caccactgta ccccaggttc ta 22
<210> 57
   <211> 22
   <212> DNA
   <213> Artificial
<220>
   <223> 22 pb target sequence
<400> 57
   tgtaccccag gttctagtca tg 22
<210> 58
   <211> 22
   <212> DNA
   <213> Artificial
   <220>
   <223> 22 pb target sequence
<400> 58
   cgggcctgtg ggagcggggt ca 22
<210> 59
   <211> 22
   <212> DNA
   <213> Artificial
<220>
   <223> 22 pb target sequence
<400> 59
   tggtatcttg cagacaagaa ga 22
<210> 60
   <211> 22
   <212> DNA
   <213> Artificial
<220>
   <223> 22 pb target sequence
<400> 60
   ccaacccatg tgcatgagta ca 22
<210> 61
   <211> 22
   <212> DNA
   <213> Artificial
<220>
   <223> 22 pb target sequence
<400> 61
   caacccatgt gcatgagtac aa 22
<210> 62
   <211> 22
   <212> DNA
   <213> Artificial
<220>
   <223> 22 pb target sequence
<400> 62
   tggaattatg cagtttatta ag 22
<210> 63
   <211> 22
   <212> DNA
   <213> Artificial
<220>
   <223> 22 pb target sequence
<400> 63
   tgttctcaga aagcagaggc ca 22
<210> 64
   <211> 22
   <212> DNA
   <213> Artificial
<220>
   <223> 22 pb target sequence
<400> 64
   ttaaatcatg gccggcagct ca 22
<210> 65
   <211> 22
   <212> DNA
   <213> Artificial
<220>
   <223> 22 pb target sequence
<400> 65
   tttgcctgct gattagattt ca 22
<210> 66
   <211> 22
   <212> DNA
   <213> Artificial
<220>
   <223> 22 pb target sequence
<400> 66
   ttggacctct tagaagatgt aa 22
<210> 67
   <211> 22
   <212> DNA
   <213> Artificial
<220>
   <223> 22 pb target sequence
<400> 67
   tatgacttcc ggaatgattc tg 22
<210> 68
   <211> 22
   <212> DNA
   <213> Artificial
<220>
   <223> 22 pb target sequence
<400> 68
   ttccctgcgg taagtggtac ca 22
<210> 69
   <211> 22
   <212> DNA
   <213> Artificial
<220>
   <223> 22 pb target sequence
<400> 69
   ccataccagg taagcaggct gg 22
<210> 70
   <211> 22
   <212> DNA
   <213> Artificial
<220>
   <223> 22 pb target sequence
<400> 70
   cgaccctcgt ccgcgaagat ga 22
<210> 71
   <211> 22
   <212> DNA
   <213> Artificial
<220>
   <223> 22 pb target sequence
<400> 71
   ttaaattttc tgattgggcc ta 22
<210> 72
   <211> 22
   <212> DNA
   <213> Artificial
<220>
   <223> 22 pb target sequence
<400> 72
   tgtgctgagg tagctgggcc tg 22
<210> 73
   <211> 22
   <212> DNA
   <213> Artificial
<220>
   <223> 22 pb target sequence
<400> 73
   tcttactgta ttacaggtct gg 22
<210> 74
   <211> 22
   <212> DNA
   <213> Artificial
<220>
   <223> 22 pb target sequence
<400> 74
   caaaaccaag aaacgaatct tg 22
<210> 75
   <211> 22
   <212> DNA
   <213> Artificial
   <220>
   <223> 22 pb target sequence
<400> 75
   tttgccctaa aggaatatgc ca 22
<210> 76
   <211> 22
   <212> DNA
   <213> Artificial
<220>
   <223> 22 pb target sequence
<400> 76
   cggacctacg tctcaggggg aa 22
<210> 77
   <211> 22
   <212> DNA
   <213> Artificial
<220>
   <223> 22 pb target sequence
<400> 77
   ccatcttcat atccaaggtt tg 22
<210> 78
   <211> 22
   <212> DNA
   <213> Artificial
<220>
   <223> 22 pb target sequence
<400> 78
   taaaccactt tagttaggaa ga 22
<210> 79
   <211> 22
   <212> DNA
   <213> Artificial
<220>
   <223> 22 pb target sequence
<400> 79 cttactggct atttttatat tg 22
<210> 80
   <211> 22
   <212> DNA
   <213> Artificial
<220>
   <223> 22 pb target sequence
<400> 80
   ctgcatgaat ctctgagggc ag 22
<210> 81
   <211> 22
   <212> DNA
   <213> Artificial
<220>
   <223> 22 pb target sequence
<400> 81
   tggtctctgg aatgctaggg ag 22
<210> 82
   <211> 22
   <212> DNA
   <213> Artificial
<220>
   <223> 22 pb target sequence
<400> 82
   tagtcctgcc tggatgggcc ca 22
<210> 83
   <211> 22
   <212> DNA
   <213> Artificial
<220>
   <223> 22 pb target sequence
<400> 83
   tgggattttt ttggaaatgt tg 22
<210> 84
   <211> 22
   <212> DNA
   <213> Artificial
<220>
   <223> 22 pb target sequence
<400> 84
   ccttccctcc agcgttggcc aa 22
<210> 85
   <211> 22
   <212> DNA
   <213> Artificial
<220>
   <223> 22 pb target sequence
<400> 85
   ttggctgtgc cttcataggt ca 22
<210> 86
   <211> 22
   <212> DNA
   <213> Artificial
<220>
   <223> 22 pb target sequence
<400> 86
   tgtgccttca taggtcatct ag 22
<210> 87
   <211> 22
   <212> DNA
   <213> Artificial
<220>
   <223> 22 pb target sequence
<400> 87
   tcgaccccgc tgcacagtcc gg 22
<210> 88
   <211> 22
   <212> DNA
   <213> Artificial
<220>
   <223> 22 pb target sequence
<400> 88
   ctatatatca gctactgtgc ca 22
<210> 89
   <211> 22
   <212> DNA
   <213> Artificial
<220>
   <223> 22 pb target sequence
<400> 89
   tggtccccaa catgcagggt ca 22
<210> 90
   <211> 22
   <212> DNA
   <213> Artificial
<220>
   <223> 22 pb target sequence
<400> 90
   cccaacatgc agggtcatgg ag 22
<210> 91
   <211> 22
   <212> DNA
   <213> Artificial
<220>
   <223> 22 pb target sequence
<400> 91
   ctgaacccgt aaaggcagac aa 22
<210> 92
   <211> 22
   <212> DNA
   <213> Artificial
   <220>
   <223> 22 pb target sequence
<400> 92
   cctgcccccc aactttggag ta 22
<210> 93
   <211> 22
   <212> DNA
   <213> Artificial
<220>
   <223> 22 pb target sequence
<400> 93
   ccttctcctt gcccttagcc ca 22
<210> 94
   <211> 22
   <212> DNA
   <213> Artificial
<220>
   <223> 22 pb target sequence
<400> 94
   caggacaggt agcctggggc ag 22
<210> 95
   <211> 22
   <212> DNA
   <213> Artificial
<220>
   <223> 22 pb target sequence
<400> 95
   tgacctgaag gccctggggc gg 22
<210> 96
   <211> 22
   <212> DNA
   <213> Artificial
<220>
   <223> 22 pb target sequence
<400> 96
   cgatactcag tgaggaggct gg 22
<210> 97
   <211> 22
   <212> DNA
   <213> Artificial
<220>
   <223> 22 pb target sequence
<400> 97
   ctccccggcc ccccagatcc tg 22
<210> 98
   <211> 22
   <212> DNA
   <213> Artificial
<220>
   <223> 22 pb target sequence
<400> 98
   cacaacattg gtgagggggg cg 22
<210> 99
   <211> 22
   <212> DNA
   <213> Artificial
<220>
   <223> 22 pb target sequence
<400> 99
   tgggaccctg gcccctgtct ga 22
<210> 100
   <211> 22
   <212> DNA
   <213> Artificial
<220>
   <223> 22 pb target sequence
<400> 100
   cgggacgagt accggcgtct gg 22
<210> 101
   <211> 22
   <212> DNA
   <213> Artificial
<220>
   <223> 22 pb target sequence
<400> 101
   cgtgctgccc gacgaagtgc tg 22
<210> 102
   <211> 22
   <212> DNA
   <213> Artificial
<220>
   <223> 22 pb target sequence
<400> 102
   ctggctccat ccgcacggcc ga 22
<210> 103
   <211> 22
   <212> DNA
   <213> Artificial
<220>
   <223> 22 pb target sequence
<400> 103
   ctccccgcca gattctgtgc tg 22
<210> 104
   <211> 22
   <212> DNA
   <213> Artificial
<220>
   <223> 22 pb target sequence
<400> 104
   cagcacctac gccaaaggta ag 22
<210> 105
   <211> 22
   <212> DNA
   <213> Artificial
<220>
   <223> 22 pb target sequence
<400> 105
   tactcccagg cagtacgggg tg 22
<210> 106
   <211> 22
   <212> DNA
   <213> Artificial
<220>
   <223> 22 pb target sequence
<400> 106
   tgtcatcatc acatctgggg ta 22
<210> 107
   <211> 22
   <212> DNA
   <213> Artificial
<220>
   <223> 22 pb target sequence
<400> 107
   tgagatccct cccactgtcc cg 22
<210> 108
   <211> 22
   <212> DNA
   <213> Artificial
<220>
   <223> 22 pb target sequence
<400> 108
   cagcctgggc aacatggtga ca 22
<210> 109
   <211> 22
   <212> DNA
   <213> Artificial
   <220>
   <223> 22 pb target sequence

<400> 109
   tggtatgctg ccagtggggc tg 22
<210> 110
   <211> 22
   <212> DNA
   <213> Artificial
<220>
   <223> 22 pb target sequence
<400> 110
   cctgacaggc agcctcagtg gg 22
<210> 111
   <211> 22
   <212> DNA
   <213> Artificial
<220>
   <223> 22 pb target sequence
<400> 111
   cagcatgtag gaatggggtg ta 22
<210> 112
   <211> 22
   <212> DNA
   <213> Artificial
<220>
   <223> 22 pb target sequence
<400> 112
   caggctgaga atgcagatat aa 22
<210> 113
   <211> 22
   <212> DNA
   <213> Artificial
<220>
   <223> 22 pb target sequence
<400> 113
   caccacatct cagatgagcc ag 22
<210> 114
   <211> 22
   <212> DNA
   <213> Artificial
<220>
   <223> 22 pb target sequence
<400> 114
   ccatcctgct gtcagtggcc cg 22
<210> 115
   <211> 22
   <212> DNA
   <213> Artificial
<220>
   <223> 22 pb target sequence
<400> 115
   tggcccgggg caaagtgtcc ga 22
<210> 116
   <211> 22
   <212> DNA
   <213> Artificial
<220>
   <223> 22 pb target sequence
<400> 116
   ccaactcaga cacagcatcc tg 22
<210> 117
   <211> 22
   <212> DNA
   <213> Artificial
<220>
   <223> 22 pb target sequence
<400> 117
   cccactcccc accctcagcc tg 22
<210> 118
   <211> 22
   <212> DNA
   <213> Artificial
<220>
   <223> 22 pb target sequence
<400> 118
   ctgtcctcct gccctcagca aa 22
<210> 119
   <211> 22
   <212> DNA
   <213> Artificial
<220>
   <223> 22 pb target sequence
<400> 119
   ccaaattcat tcaaacatcc tg 22
<210> 120
   <211> 22
   <212> DNA
   <213> Artificial
<220>
   <223> 22 pb target sequence
<400> 120
   caagcctcga catgtcgtac aa 22
<210> 121
   <211> 22
   <212> DNA
   <213> Artificial
<220>
   <223> 22 pb target sequence
<400> 121
   caggacactt tacttcggcc ga 22
<210> 122
   <211> 22
   <212> DNA
   <213> Artificial
<220>
   <223> 22 pb target sequence
<400> 122
   taatattggt ttccaggggg ag 22
<210> 123
   <211> 22
   <212> DNA
   <213> Artificial
<220>
   <223> 22 pb target sequence
<400> 123
   tggccttttc aaggttattc ca 22
<210> 124
   <211> 22
   <212> DNA
   <213> Artificial
<220>
   <223> 22 pb target sequence
<400> 124
   ttgtctacca ggtactggat ca 22
<210> 125
   <211> 22
   <212> DNA
   <213> Artificial
<220>
   <223> 22 pb target sequence
<400> 125
   caggaccctc aggggcggca cg 22
<210> 126
   <211> 22
   <212> DNA
   <213> Artificial
   <220>
   <223> 22 pb target sequence
<400> 126
   ttccatggtg atcgcaggtt gg 22
<210> 127
   <211> 22
   <212> DNA
   <213> Artificial
<220>
   <223> 22 pb target sequence
<400> 127
   cccacccata ggaatagtcg aa 22
<210> 128
   <211> 22
   <212> DNA
   <213> Artificial
<220>
   <223> 22 pb target sequence
<400> 128
   tcttactctg tcgctcaggc tg 22
<210> 129
   <211> 22
   <212> DNA
   <213> Artificial
<220>
   <223> 22 pb target sequence
<400> 129
   taaaatggct gggtttggta aa 22
<210> 130
   <211> 22
   <212> DNA
   <213> Artificial
<220>
   <223> 22 pb target sequence
<400> 130
   ctgaattatg gctgcagttg gg 22
<210> 131
   <211> 22
   <212> DNA
   <213> Artificial
<220>
   <223> 22 pb target sequence
<400> 131
   ccagcttgtg aaggtgagaa ga 22
<210> 132
   <211> 22
   <212> DNA
   <213> Artificial
<220>
   <223> 22 pb target sequence
<400> 132
   ccatctcttt tgggtgggtc tg 22
<210> 133
   <211> 22
   <212> DNA
   <213> Artificial
<220>
   <223> 22 pb target sequence
<400> 133
   tggacctgga gaggcagggg ca 22
<210> 134
   <211> 22
   <212> DNA
   <213> Artificial
<220>
   <223> 22 pb target sequence
<400> 134
   tcttctgtgg caacgtggct ca 22
<210> 135
   <211> 22
   <212> DNA
   <213> Artificial
<220>
   <223> 22 pb target sequence
<400> 135
   tctgacccta atcgtgagac tg 22
<210> 136
   <211> 22
   <212> DNA
   <213> Artificial
<220>
   <223> 22 pb target sequence
<400> 136
   tcttctgtgg caacgtggct ca 22
<210> 137
   <211> 22
   <212> DNA
   <213> Artificial
<220>
   <223> 22 pb target sequence
<400> 137
   ttccccacca gctgtaggtt tg 22
<210> 138
   <211> 22
   <212> DNA
   <213> Artificial
<220>
   <223> 22 pb target sequence
<400> 138
   tgccatattc ttctttgttc ag 22
<210> 139
   <211> 22
   <212> DNA
   <213> Artificial
<220>
   <223> 22 pb target sequence
<400> 139
   tggcctctgg acggacatct cg 22
<210> 140
   <211> 22
   <212> DNA
   <213> Artificial
<220>
   <223> 22 pb target sequence
<400> 140
   ttacacagaa ttcttagtcc ca 22
<210> 141
   <211> 22
   <212> DNA
   <213> Artificial
<220>
   <223> 22 pb target sequence
<400> 141
   tgtcattctc tctgtaggtc tg 22
<210> 142
   <211> 22
   <212> DNA
   <213> Artificial
<220>
   <223> 22 pb target sequence
<400> 142
   tgagatggga agtatagtgc ag 22
<210> 143
   <211> 22
   <212> DNA
   <213> Artificial
   <220>
   <223> 22 pb target sequence
<400> 143
   ccagaccaat gaaataagag ta 22
<210> 144
   <211> 22
   <212> DNA
   <213> Artificial
<220>
   <223> 22 pb target sequence
<400> 144
   tggcaccatc gatgagatcc ag 22
<210> 145
   <211> 22
   <212> DNA
   <213> Artificial
<220>
   <223> 22 pb target sequence
<400> 145
   tctgctacca ggaagtgtcc ca 22
<210> 146
   <211> 22
   <212> DNA
   <213> Artificial
<220>
   <223> 22 pb target sequence
<400> 146
   caggctctgt ccagcagtgt aa 22
<210> 147
   <211> 22
   <212> DNA
   <213> Artificial
<220>
   <223> 22 pb target sequence
<400> 147
   ctccctaatc caggctgttc tg 22
<210> 148
   <211> 22
   <212> DNA
   <213> Artificial
<220>
   <223> 22 pb target sequence
<400> 148
   tggtctttca gtattcggat gg 22
<210> 149
   <211> 22
   <212> DNA
   <213> Artificial
<220>
   <223> 22 pb target sequence
<400> 149
   cagtattcgg atggtaagtg gg 22
<210> 150
   <211> 22
   <212> DNA
   <213> Artificial
<220>
   <223> 22 pb target sequence
<400> 150
   tgtactctat ggtggaattt aa 22
<210> 151
   <211> 22
   <212> DNA
   <213> Artificial
<220>
   <223> 22 pb target sequence
<400> 151
   tagcacggtg aggcctggac ca 22
<210> 152
   <211> 22
   <212> DNA
   <213> Artificial
<220>
   <223> 22 pb target sequence
<400> 152
   ccagcccagt attaggggca ga 22
<210> 153
   <211> 22
   <212> DNA
   <213> Artificial
<220>
   <223> 22 pb target sequence
<400> 153
   ccaaatgggc tgcgttggca ga 22
<210> 154
   <211> 22
   <212> DNA

   <213> Artificial
<220>
   <223> 22 pb target sequence
<400> 154
   tggtcttttc cacctgggcg ag 22
<210> 155
   <211> 22
   <212> DNA
   <213> Artificial
<220>
   <223> 22 pb target sequence
<400> 155
   ttccaccccc acacaaggct cg 22
<210> 156
   <211> 22
   <212> DNA
   <213> Artificial
<220>
   <223> 22 pb target sequence
<400> 156
   caacctcctg ctggacatgc ag 22
<210> 157
   <211> 22
   <212> DNA
   <213> Artificial
<220>
   <223> 22 pb target sequence
<400> 157
   tcgactcaat aaagtcatca aa 22
<210> 158
   <211> 22
   <212> DNA
   <213> Artificial
<220>
   <223> 22 pb target sequence
<400> 158
   caagatgcag gaggtggtgg ca 22
<210> 159
   <211> 22
   <212> DNA
   <213> Artificial
<220>
   <223> 22 pb target sequence
<400> 159
   ccatcttctc attgcagtat ga 22
<210> 160
   <211> 22
   <212> DNA
   <213> Artificial
<220>
   <223> 22 pb target sequence
<400> 160
   tatgacgatg gcagcggtat ga 22
<210> 161
   <211> 22
   <212> DNA
   <213> Artificial
<220>
   <223> 22 pb target sequence
<400> 161
   cgacctcatc aaggttgtgg ag 22
<210> 162
   <211> 22
   <212> DNA
   <213> Artificial
<220>
   <223> 22 pb target sequence
<400> 162
   ctaactcgga tccattagcc aa 22
<210> 163
   <211> 22
   <212> DNA
   <213> Artificial
<220>
   <223> 22 pb target sequence
<400> 163
   tcggatccat tagccaaggg ca 22
<210> 164
   <211> 22
   <212> DNA
   <213> Artificial
<220>
   <223> 22 pb target sequence
<400> 164
   tgtcctcttt ttatttagat tg 22
<210> 165
   <211> 22
   <212> DNA
   <213> Artificial
<220>
   <223> 22 pb target sequence
<400> 165
   ctgactgcca agccatgggt ag 22
<210> 166
   <211> 22
   <212> DNA
   <213> Artificial
<220>
   <223> 22 pb target sequence
<400> 166
   ccaaataaag tagaatataa ga 22
<210> 167
   <211> 22
   <212> DNA
   <213> Artificial
<220>
   <223> 22 pb target sequence
<400> 167
   tgacacagag aaggatggca gg 22
<210> 168
   <211> 22
   <212> DNA
   <213> Artificial
<220>
   <223> 22 pb target sequence
<400> 168
   ctgccctgta ttaaatagcc ta 22
<210> 169
   <211> 22
   <212> DNA
   <213> Artificial
<220>
   <223> 22 pb target sequence
<400> 169
   tttgatactg gtttttgtca tg 22
<210> 170
   <211> 22
   <212> DNA
   <213> Artificial
<220>
   <223> 22 pb target sequence
<400> 170
   ctgtatctgt ggccaaggta aa 22
<210> 171
   <211> 22
   <212> DNA
   <213> Artificial
<220>
   <223> 22 pb target sequence
<400> 171
   taacccatcg cttgaagtgg aa 22
<210> 172
   <211> 22
   <212> DNA
   <213> Artificial
<220>
   <223> 22 pb target sequence
<400> 172

   caagactaaa tccttagttc ag 22
<210> 173
   <211> 22
   <212> DNA
   <213> Artificial
<220>
   <223> 22 pb target sequence
<400> 173 22
   ttgaataaag tgttaggttt ta 22
<210> 174
   <211> 22
   <212> DNA
   <213> Artificial
<220>
   <223> 22 pb target sequence
<400> 174 22
   tttaactttt cgtattaggt tg 22
<210> 175
   <211> 22
   <212> DNA
   <213> Artificial
<220>
   <223> 22 pb target sequence
<400> 175 22
   ttaacttttc gtattaggtt gg 22
<210> 176
   <211> 22
   <212> DNA
   <213> Artificial
<220>
   <223> 22 pb target sequence
<400> 176 22
   tgtaatgtat gttgaaagta ta 22
<210> 177
   <211> 22
   <212> DNA
   <213> Artificial
   <220>
   <223> 22 pb target sequence
<400> 177
   tttgcttcca aaatctatca aa 22
<210> 178
   <211> 22
   <212> DNA
   <213> Artificial
<220>
   <223> 22 pb target sequence
<400> 178
   ttagctcttt aaaagtagtt ca 22
<210> 179
   <211> 22
   <212> DNA
   <213> Artificial
<220>
   <223> 22 pb target sequence
<400> 179
   tcatccatcc gcttctgggt tg 22
<210> 180
   <211> 22
   <212> DNA
   <213> Artificial
<220>
   <223> 22 pb target sequence
<400> 180
   ctaacctttg ttcggcagct tg 22
<210> 181
   <211> 22
   <212> DNA
   <213> Artificial
<220>
   <223> 22 pb target sequence
<400> 181
   tttaatatcc gttacgatgc tg 22
<210> 182
   <211> 22
   <212> DNA
   <213> Artificial
<220>
   <223> 22 pb target sequence
<400> 182
   tagtcctgct caggaaggat ag 22
<210> 183
   <211> 22
   <212> DNA
   <213> Artificial
<220>
   <223> 22 pb target sequence
<400> 183
   cctgctcagg aaggataggg ca 22
<210> 184
   <211> 22
   <212> DNA
   <213> Artificial
<220>
   <223> 22 pb target sequence
<400> 184
   ttgtccctga agaaagagaa gg 22
<210> 185
   <211> 22
   <212> DNA
   <213> Artificial
<220>
   <223> 22 pb target sequence
<400> 185
   cactccagaa atgtgcgtgg ag 22
<210> 186
   <211> 22
   <212> DNA
   <213> Artificial
<220>
   <223> 22 pb target sequence
<400> 186 22
   cagcactggc cattacagca ga 22
<210> 187
   <211> 22
   <212> DNA
   <213> Artificial
<220>
   <223> 22 pb target sequence
<400> 187 22
   ctggccatta cagcagattc tg 22
<210> 188
   <213> 22
   <212> DNA
   <213> Artificial
<220>
   <223> 22 pb target sequence
<400> 188
   cagaattagc agccctgtca ca 22
<210> 189
   <211> 22
   <212> DNA
   <213> Artificial
<220>
   <223> 22 pb target sequence
<400> 189
   cgggcctgtg ggagcggggt ca 22
<210> 190
   <211> 22
   <212> DNA
   <213> Artificial
<220>
   <223> 22 pb target sequence
<400> 190
   tggtatcttg cagacaagaa ga 22
<210> 191
   <211> 22
   <212> DNA
   <213> Artificial
<220>
   <223> 22 pb target sequence
<400> 191
   ccaacccatg tgcatgagta ca 22
<210> 192
   <211> 22
   <212> DNA
   <213> Artificial
<220>
   <223> 22 pb target sequence
<400> 192
   caacccatgt gcatgagtac aa 22
<210> 193
   <211> 22
   <212> DNA
   <213> Artificial
<220>
   <223> 22 pb target sequence
<400> 193
   tggaattatg cagtttatta ag 22
<210> 194
   <211> 22
   <212> DNA
   <213> Artificial
<220>

   <223> 22 pb target sequence
<400> 194
   tgttctcaga aagcagaggc ca 22
<210> 195
   <211> 22
   <212> DNA
   <213> Artificial
<220>
   <223> 22 pb target sequence
<400> 195
   ttaaatcatg gccggcagct ca 22
<210> 196
   <211> 22
   <212> DNA
   <213> Artificial
<220>
   <223> 22 pb target sequence
<400> 196
   tttgcctgct gattagattt ca 22
<210> 197
   <211> 22 <212> DNA
   <213> Artificial
<220>
   <223> 22 pb target sequence
<400> 197
   ttggacctct tagaagatgt aa 22
<210> 198
   <211> 22 '
   <212> DNA
   <213> Artificial
<220>
   <223> 22 pb target sequence
<400> 198
   tatgacttcc ggaatgattc tg 22
<210> 199 <211> 22
   <212> DNA
   <213> Artificial
<220>
   <223> 22 pb target sequence
<400> 199
   ttccctgcgg taagtggtac ca 22
<210> 200 .
   <211> 22
   <212> DNA
   <213> Artificial
<220>
   <223> 22 pb target sequence
<400> 200
   ccataccagg taagcaggct gg 22
<210> 201
   <211> 22
   <212> DNA
   <213> Artificial
<220>
   <223> 22 pb target sequence
<400> 201
   cgaccctcgt ccgcgaagat ga 22
<210> 202
   <211> 22
   <212> DNA
   <213> Artificial
<220>
   <223> 22 pb target sequence
<400> 202
   ttaaattttc tgattgggcc ta 22
<210> 203
   <211> 22
   <212> DNA
   <213> Artificial
<220>
   <223> 22 pb target sequence
<400> 203
   tgtgctgagg tagctgggcc tg 22
<210> 204
   <211> 22
   <212> DNA
   <213> Artificial
<220>
   <223> 22 pb target sequence
<400> 204
   tcttactgta ttacaggtct gg 22
<210> 205
   <211> 22
   <212> DNA
   <213> Artificial
<220>
   <223> 22 pb target sequence
<400> 205
   caaaaccaag aaacgaatct tg 22
<210> 206
   <211> 22
   <212> DNA
   <213> Artificial
<220>
   <223> 22 pb target sequence
<400> 206
   tttgccctaa aggaatatgc ca 22
<210> 207
   <211> 22
   <212> DNA
   <213> Artificial
<220>
   <223> 22 pb target sequence
<400> 207
   cggacctacg tctcaggggg aa 22
<210> 208
   <211> 22
   <212> DNA
   <213> Artificial
<220>
   <223> 22 pb target sequence
<400> 208
   ccatcttcat atccaaggtt tg 22
<210> 209
   <211> 22
   <212> DNA
   <213> Artificial
<220>
   <223> 22 pb target sequence
<400> 209
   taaaccactt tagttaggaa ga 22
<210> 210
   <211> 22
   <212> DNA
   <213> Artificial
<220>
   <223> 22 pb target sequence
<400> 210
   cttactggct atttttatat tg 22
<210> 211
   <211> 22
   <212> DNA
   <213> Artificial
<220>
   <223> 22 pb target sequence
<400> 211
   ctgcatgaat ctctgagggc ag 22
<210> 212
   <211> 22
   <212> DNA
   <213> Artificial
<220>
   <223> 22 pb target sequence
<400> 212
   tggtctctgg aatgctaggg ag 22
<210> 213
   <211> 22
   <212> DNA
   <213> Artificial
<220>
   <223> 22 pb target sequence
<400> 213
   tagtcctgcc tggatgggcc ca 22
<210> 214
   <211> 22
   <212> DNA
   <213> Artificial
<220>
   <223> 22 pb target sequence
<400> 214
   tgggattttt ttggaaatgt tg 22
<210> 215
   <211> 22
   <212> DNA
   <213> Artificial
<220>
   <223> 22 pb target sequence
<400> 215
   ccttccctcc agcgttggcc aa 22
<210> 216
   <211> 22
   <212> DNA

   <213> Artificial
<220>
   <223> 22 pb target sequence
<400> 216
   ttggctgtgc cttcataggt ca 22
<210> 217
   <211> 163
   <212> PRT
   <213> Chlamydomonas reinhardtii
<400> 217
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Tyr Lys Phe Lys His Gln Leu Ser Leu Ala Phe Gln Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Arg Gly Ser Val Ser Asp Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 218
   <211> 167
   <212> PRT
   <213> artificial sequence
<220>
   <223> I-CreI N75 scaffold protein
<400> 218
   Met Ala Asn Thr Lys Tyr Asn Lys Glu Phe Leu Leu Tyr Leu Ala Gly
   1 5 10 15
   Phe Val Asp Gly Asp Gly Ser Ile Ile Ala Gln Ile Lys Pro Asn Gln
   20 25 30
   Ser Tyr Lys Phe Lys His Gln Leu Ser Leu Thr Phe Gln Val Thr Gln
           35                  40                  45
   Lys Thr Gln Arg Arg Trp Phe Leu Asp Lys Leu Val Asp Glu Ile Gly
       50                  55                  60
   Val Gly Tyr Val Arg Asp Arg Gly Ser Val Ser Asn Tyr Ile Leu Ser
   65                  70                  75                  80
   Glu Ile Lys Pro Leu His Asn Phe Leu Thr Gln Leu Gln Pro Phe Leu
                   85                  90                  95
   Lys Leu Lys Gln Lys Gln Ala Asn Leu Val Leu Lys Ile Ile Glu Gln
               100                 105                 110
   Leu Pro Ser Ala Lys Glu Ser Pro Asp Lys Phe Leu Glu Val Cys Thr
           115                 120                 125
   Trp Val Asp Gln Ile Ala Ala Leu Asn Asp Ser Lys Thr Arg Lys Thr
       130                 135                 140
   Thr Ser Glu Thr Val Arg Ala Val Leu Asp Ser Leu Ser Glu Lys Lys
   145                 150                 155                 160
   Lys Ser Ser Pro Ala Ala Asp
                   165
<210> 219
   <211> 22
   <212> DNA
   <213> artificial sequence
<220>
   <223> Xb.2 target
<400> 219
   cgggctgggg tacgtaggac ag 22
<210> 220 <211> 22
   <212> DNA
   <213> artificial sequence
<220>
   <223> Xb.3 target
<400> 220
   cgggctgggg taccccagcc cg 22
<210> 221
   <211> 22
   <212> DNA
   <213> artificial sequence
<220>
   <223> Xb.4 target
<400> 221
   ctgtcctacg tacgtaggac ag 22
<210> 222
   <211> 22
   <212> DNA
   <213> artificial sequence
<220>
   <223> xc.2 target
<400> 222
   cgagatgtcg tacagaggta cg 22
<210> 223
   <211> 22
   <212> DNA
   <213> artificial sequence
<220>
   <223> Xc.3 target
<400> 223
   cgagatgtcg tacgacatct cg 22
<210> 224
   <211> 22
   <212> DNA
   <213> artificial sequence
<220>
   <223> xc.4 target
<400> 224
   cgtacctctg tacagaggta cg 22
<210> 225
   <211> 77
   <212> DNA
   <213> artificial sequence
<220>
   <223> primer
<400> 225
   ggggacaagt ttgtacaaaa aagcaggctt cgaaggagat agaaccatgg ccaataccaa 60
   atataacaaa gagttcc 77
<210> 226
   <211> 64
   <212> DNA
   <213> artificial sequence
<220>
   <223> primer
<400> 226
   ggggaccact ttgtacaaga aagctgggtt tagtcggccg ccggggagga tttcttcttc 60
   tcgc 64
<210> 227
   <211> 59
   <212> DNA
   <213> artificial sequence
<220>
   <223> primer
<400> 227
   gcataaatta ctatacttct atagacacgc aaacacaaat acacagcggc cttgccacc 59
<210> 228
   <211> 48
   <212> DNA <213> artificial sequence
<220>
   <223> primer
<400> 228
   ggctcgagga gctcgtctag aggatcgctc gagttatcag tcggccgc 48

## Description

The invention relates to a meganuclease variant cleaving a DNA target sequence from a xeroderma pigmentosum gene (*XP* gene), to a vector encoding said variant, to a cell, an animal or a plant modified by said vector and to the use of said meganuclease variant and derived products for genome therapy, *in vivo* and *ex vivo* (gene cell therapy), and genome engineering.

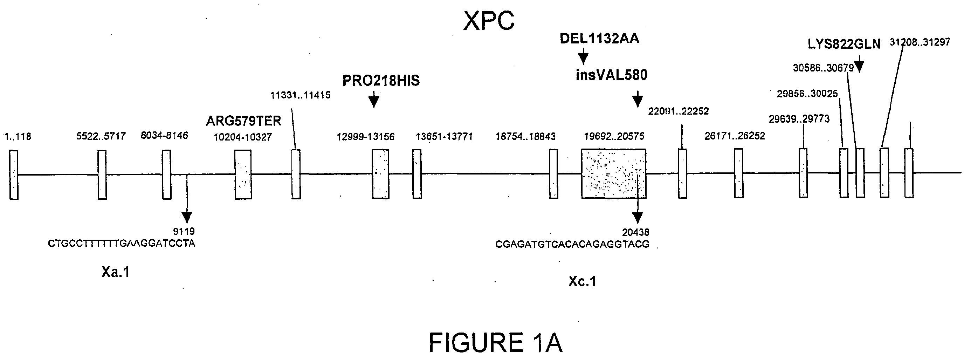

Xeroderma pigmentosum (XP) is a rare autosomal recessive genetic disease characterized by a hypersensitivity to exposure to ultraviolet A (UV) rays, a high predisposition for developing skin cancers on sunlight exposed areas, and in some cases neurological disorders (Hengge, U.R. and W. Bardenheuer, Am. J. Med. Genet. C. Semin. Med. Genet., 2004, 131: 93-100; Magnaldo, T. and A. Sarasin, Cells Tissues Organs, 2004, 177: 189-198; Cleaver, J. E., Nat. Rev. Cancer, 2005, 5: 564-573; Hengge U.R., Clin. Dermatol., 2005, 23: 107-114). Cells of XP patients present a reduced capacity to eliminate UV induced DNA lesions (Cordonnier, A.M. and R.P. Fuchs, Mutat. Res., 1999, 435, 111-119). Such abnormality results from a defect in the Nucleotide Excision Repair (NER) process, a versatile mechanism conserved among eukaryotes and implicated in the correction of the damaged DNA by excision of the damaged nucleotides and re-synthesis. Defect in this process leads to a persistence of UV damage in the DNA, resulting in mutagenesis and tumour development in the UV exposed skin area. The three major types of skin cancers, squamous cell carcinomas, basal cell carcinomas and malignant melanomas already appear in childhood. XP Patients were assigned to 7 complementation groups (XP-A to XP-G) by cell fusion experiments, and each complementation group turned out to result from mutations in a distinct NER gene. The human genes, and the encoded proteins were often named after the complementation group. For example, the *XPC* gene (figure 1A), mutated in the XP-C complementation group, codes for a DNA damage binding protein.

Until now the only treatment available to XP patients is either full protection against sun exposure (as well as against certain common lamps producing long-wavelength UV) or repeated surgery to remove appearing skin cancers. Several attempts of autologous graft have been made to replace such cancerous area with skin from unexposed parts of the patient's body. However, since the grafted cells are also sun-sensitive the benefits for the patients are at best, limited to a few years, and the majority of patients die before reaching adulthood because of metastases. Skin engraftment can be made locally, but with the general limitations of grafts, in term of immunological tolerance.

Thus gene and cell therapy represent a huge hope for this kind of disease. Since cells from the skin lineage can be easily manipulated *in vitro,* a possibility would be to manipulate patient cells and correct their genetic defect, before grafting them back at the site of the tumour. Compared to other XP complementation groups, XP-C seems to be the best candidate for corrective gene transfer. In Europe and North Africa, XP-C is involved in more than half of the XP patients and although XPC expression is ubiquitous, XP-C patients remain free of neurological problems observed in other XP groups. Preliminary studies aimed at tissue therapy of XP patients have shown that *in vitro* retroviral transduction of XP fibroblasts from various complementation groups (XP-A, XP-B, XP-C, XP-D) and of XP-C primary keratinocytes with the XP cloned genes result in the recovery of full DNA repair capacity (Arnaudeau-Begard et al., Hum. Gene Ther., 2003, 14, 983-996; Armelini et al., Cancer Gene Ther., 2005, 12, 389-396). Furthermore, cells from the skin lineage can be easily manipulated, and then used to reconstruct functional skin (Amaudeau-Begard et al., Hum. Gene Ther., 2003, 14, 983-996; Armelini et al., Cancer Gene Ther., 2005, 12, 389-396). Thus, a rationale and promising alternative for long term tissular therapy would then consist in an *ex vivo* gene correction of the XP-C locus in keratinocytes before grafting back a reconstructed skin to the patient.

Homologous recombination is the best way to precisely engineer a given locus. Homologous gene targeting strategies have been used to knock out endogenous genes (Capecchi, M. R., Science, 1989, 244: 1288-1292; Smithies O., Nat. Med., 2001, 7: 1083-1086) or knock-in exogenous sequences in the chromosome. It can as well be used for gene correction, and in principle, for the correction of mutations linked with monogenic diseases, such as XP. However, this application is in fact difficult, due to the low efficiency of the process (10⁻⁶ to 10⁻⁹ of transfected cells). In the last decade, several methods have been developed to enhance this yield. For example, chimeraplasty (De Semir et al. J. Gene Med., 2003, 5: 625-639) and Small Fragment Homologous Replacement (Goncz et al., Gene Ther, 2001, 8: 961-965; Bruscia et al., Gene Ther., 2002, 9: 683-685; Sangiuolo et al., BMC Med. Genet., 2002, 3: 8-; De Semir and Aran, Oligonucleotides, 2003, 13: 261-269; US Patent 6,010,908) have both been used to try to correct CFTR mutations with various levels of success.

Another strategy to enhance the efficiency of recombination is to deliver a DNA double-strand break in the targeted locus, using meganucleases. Meganucleases are by definition sequence-specific endonucleases recognizing large sequences (12 to 45 bp). They can cleave unique sites in living cells, thereby enhancing gene targeting by 1000-fold or more in the vicinity of the cleavage site (Puchta et al., Nucleic Acids Res., 1993, 21: 5034-5040; Rouet et al., Mol. Cell. Biol., 1994, 14, 8096-8106; Choulika et al., Mol. Cell. Biol., 1995, 15, 1968-1973; Puchta et al., Proc. Natl. Acad. Sci. USA, 1996, 93, 5055-5060; Sargent et al., Mol. Cell. Biol., 1997, 17, 267-277; Donoho et al., Mol. Cell. Biol, 1998, 18, 4070-4078; Elliott et al., Mol. Cell. Biol., 1998, 18, 93-101; Cohen-Tannoudji et al., Mol. Cell. Biol., 1998, 18, 1444-1448). Recently, I-*Sce*I was used to stimulate targeted recombination in mouse hepatocytes *in vivo.* Recombination could be observed in up to 1 % of hepatocytes (Gouble *et al.,* J. Gene Med., 2006, 8, 616-622).

However, the use of this technology is limited by the repertoire of natural meganucleases. For example, there is no cleavage site for a known natural meganuclease in human *XP* genes. Therefore, the making of meganucleases with tailored specificities is under intense investigation, and several laboratories have tried to alter the specificity of natural meganucleases or to make artificial endonuclease.

Recently, fusion of Cys2-His2 type Zinc-Finger Proteins (ZFP) with the catalytic domain of the Type IIS *Fok*I endonuclease were used to make functional sequence-specific artificial endonucleases (Smith et al., Nucleic Acids Res., 1999, 27: 674-681; Bibikova et al., Science, 2003, 300: 764; Porteus M.H. and D. Baltimore, Science, 2003, 300: 763). The binding specificity of ZFPs is relatively easy to manipulate, and a repertoire of novel artificial ZFPs, able to bind many (g/a)nn(g/a)nn(g/a)nn sequences is now available (Pabo et al., Annu. Rev. Biochem., 2001, 70, 313-340; Segal, D.J. and C.F. Barbas, Curr. Opin. Biotechnol., 2001, 12, 632-637; Isalan et al., Nat. Biotechnol., 2001, 19, 656-660). This last strategy allowed recently for the engineering of the IL2RG gene *in vitro* (Urnov et al., Nature, 2005, 435, 646-651). Nevertheless, preserving a very narrow specificity is one of the major issues for genome engineering applications, and presently it is unclear whether ZFPs would fulfill the very strict requirements for therapeutic applications.

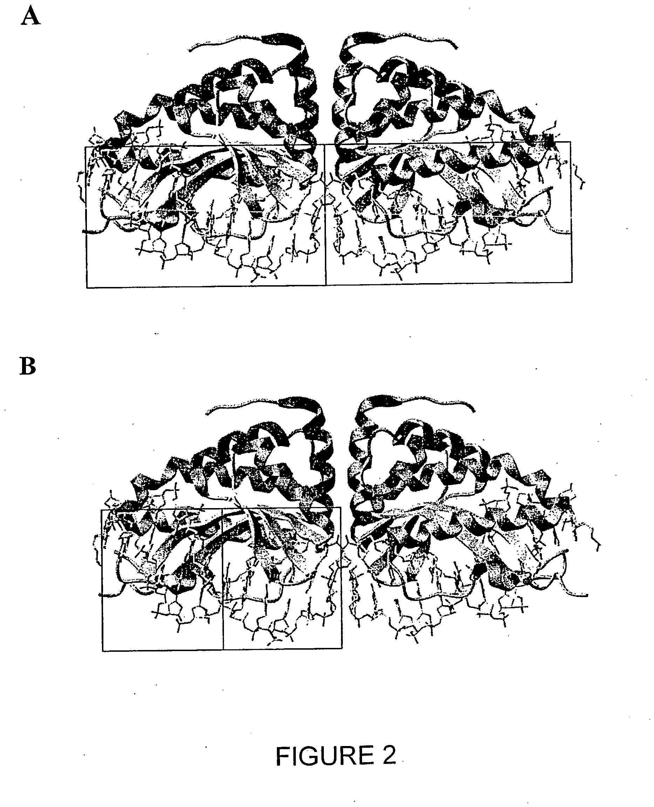

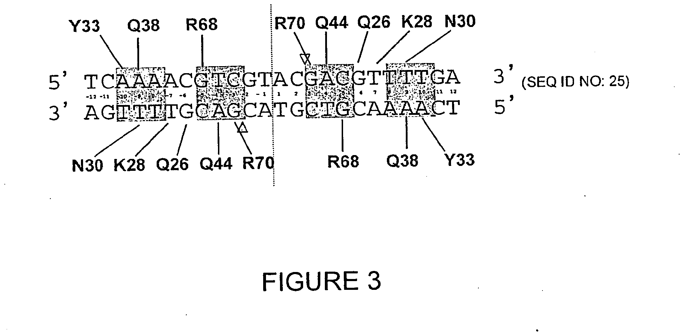

Homing Endonucleases (HEs) are a widespread family of natural meganucleases including hundreds of proteins (Chevalier, B.S. and B.L. Stoddard, Nucleic Acids Res., 2001, 29, 3757-3774). These proteins are encoded by mobile genetic elements which propagate by a process called "homing": the endonuclease cleaves a cognate allele from which the mobile element is absent, thereby stimulating a homologous recombination event that duplicates the mobile DNA into the recipient locus (Kostriken et al., Cell; 1983, 35, 167-174; Jacquier, A. and B. Dujon, Cell, 1985, 41, 383-394). Given their natural function and their exceptional cleavage properties in terms of efficacy and specificity, HEs provide ideal scaffolds to derive novel endonucleases for genome engineering. Data have been accumulated over the last decade, characterizating the LAGLIDADG family, the largest of the four HE families (Chevalier and Stoddard, precited). LAGLIDADG refers to the only sequence actually conserved throughout the family and is found in one or (more often) two copies in the protein. Proteins with a single motif, such as I-*Cre*I, form homodimers and cleave palindromic or pseudo-palindromic DNA sequences, whereas the larger, double motif proteins, such as I-*Sce*I are monomers and cleave non-palindromic targets. Seven different LAGLIDADG proteins have been crystallized, and they exhibit a very striking conservation of the core structure, that contrasts with the lack of similarity at the primary sequence level (Jurica et al., Mol. Cell., 1998, 2, 469-476; Chevalier et al., Nat. Struct. Biol., 2001, 8, 312-316 ; Chevalier et al., J. Mol. Biol., 2003, 329, 253-269; Moure et al., J. Mol. Biol, 2003, 334, 685-695; Moure et al., Nat. Struct. Biol., 2002, 9, 764-770; Ichiyanagi et al., J. Mol. Biol., 2000, 300, 889-901; Duan et al., Cell, 1997, 89, 555-564; Bolduc et al., Genes Dev., 2003, 17, 2875-2888; Silva et al., J. Mol. Biol., 1999, 286, 1123-1136). In this core structure, two characteristic αββαββα folds, also called LAGLIDADG Homing Endonuclease Core Domains, contributed by two monomers, or by two domains in double LAGLIDAG proteins, are facing each other with a two-fold symmetry. DNA binding depends on the four β strands from each domain, folded into an antiparallel β-sheet, and forming a saddle on the DNA helix major groove (figure 2). Analysis of I-*Cre*I structure bound to its natural target shows that in each monomer, eight residues (Y33, Q38, N30, K28, Q26, Q44, R68 and R70) establish direct interactions with seven bases at positions ± 3, 4, 5, 6, 7, 9 and 10 (Jurica *et al.,* 1998, precited; figure 3). In addition, some residues establish water-mediated contact with several bases; for example S40 and N30 with the base pair at position +8 and -8 (Chevalier *et al.,* 2003, precited). The catalytic core is central, with a contribution of both symmetric monomers/domains. In addition to this core structure, other domains can be found: for example, PI*-Sce*I, an intein, has a protein splicing domain, and an additional DNA-binding domain (Moure *et al.,* 2002, precited; Grindl et al., Nucleic Acids Res., 1998, 26, 1857-1862).

Two approaches have been used to derive novel endonucleases with new specificities, from Homing Endonucleases:

### - protein variants

Seligman and co-workers used a rational approach to substitute specific individual residues of the I-*Cre*I αββαββα fold (Sussman et al., J. Mol. Biol., 2004, 342, 31-41; Seligman et al., Genetics, 1997, 147, 1653-64); substantial cleavage was observed for few I-*Cre*I variants (Y33C, Y33H, Y33R, Y33L, Y33S, Y33T, S32K, S32R) and only for a target modified in position ±10.

In a similar way, Gimble *et al.* modified the additional DNA binding domain of PI-*Sce*I (J. Mol. Biol., 2003, 334, 993-1008); they obtained protein variants with altered binding specificity but no altered specificity and most of the variants maintained a lot of affinity for the wild-type target sequence.

The semi-rational approach used in theses studies permits the identification of endonucleases with altered specificity; however, it does not allow the direct production of endonucleases with predicted specificity.

### - hybrid or chimeric single-chain proteins

New meganucleases could be obtained by swapping LAGLIDADG Homing Endonuclease Core Domains of different monomers (Epinat et al., Nucleic Acids Res., 2003, 31, 2952-62; Chevalier et al., Mol. Cell., 2002, 10, 895-905; Steuer et al., Chembiochem., 2004, 5, 206-13; International PCT Applications WO 03/078619 and WO 2004/031346). These single-chain chimeric meganucleases wherein the two LAGLIDADG Homing Endonuclease Core Domains from different meganucleases are linked by a spacer, are able to cleave the hybrid target corresponding to the fusion of the two half parent DNA target sequences.

The construction of chimeric and single chain artificial HEs has suggested that a combinatorial approach could be used to obtain novel meganucleases cleaving novel (non-palindromic) target sequences: different monomers or core domains could be fused in a single protein, to achieve novel specificities. These results mean that the two DNA binding domains of an I-*Cre*I dimer behave independently; each DNA binding domain binds a different half of the DNA target site (figure 2A). Recently, a two steps strategy was used to tailor the specificity of a natural HEs such as I-*Cre*I (Arnould et al., J. Mol. Biol., 2006, 355: 443-458). In a first step, residues Q44, R68 and R70 were mutagenized, and a collection of variants with altered specificity in positions ± 3 to 5 (5NNN DNA target) were identified by screening. In a second step, two different variants were combined and assembled in a functional heterodimeric endonuclease able to cleave a chimeric target resulting from the fusion of a different half of each variant DNA target sequence.

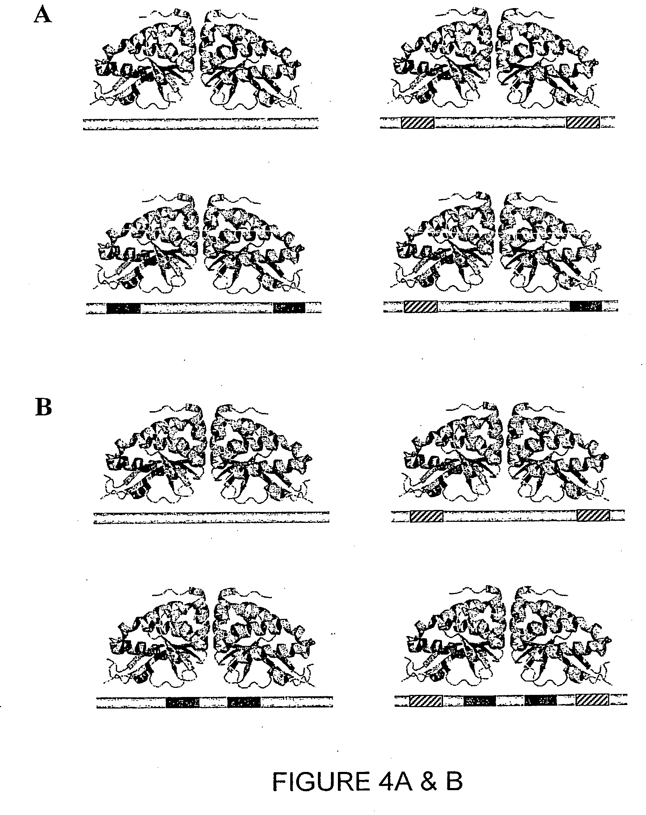

The generation of collections of novel meganucleases, and the ability to combine them by assembling two different monomers/core domains considerably enriches the number of DNA sequences that can be targeted (figure 4A), but does not yet saturate all potential sequences.

To reach a larger number of sequences, it would be extremely valuable to be able to identify smaller independent subdomains that could be combined (figure 2B).

The Patent Application WO2004/067736 discloses a method for making and screening libraries of meganuclease variants able to cleave a desired DNA target sequence which is different from the recognition and cleavage site of the initial meganuclease, including I-*Cre*I variants resulting from the combination of separate libraries; a first wherein randomized residues are I24, Q26, K28, N30, S32, Y33, Q38, S40 and T42 and a second wherein randomized residues are Y66, R68, R70, V73, D75 and I77. Although, Patent Application WO2004/067736 mentions that meganuclease variants can be used to introduce double strand DNA breaks in gene therapy of genetic diseases, including XP, said Patent Application does not disclose any variant able to cleave a desired DNA target sequence, which were obtained using this method.

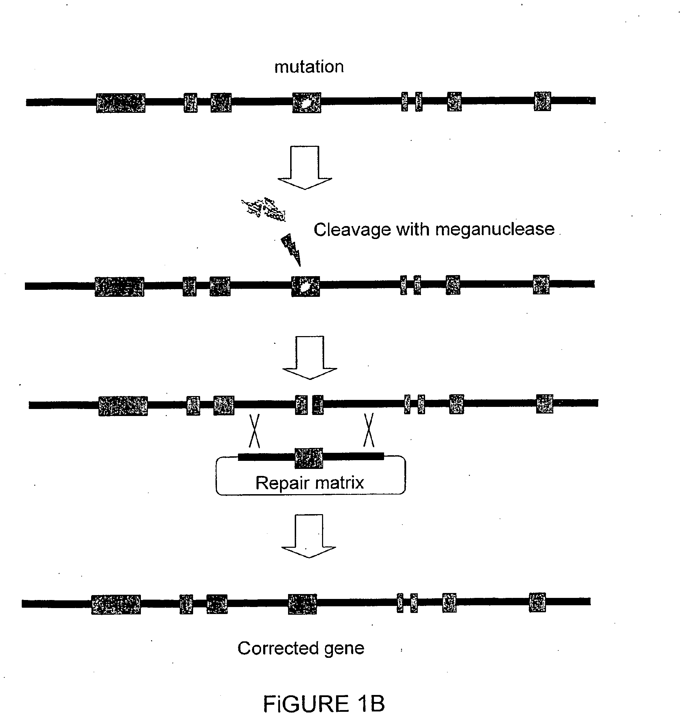

A combinatorial approach is much more difficult to apply within a single monomer or domain than between monomers since the structure of the binding interface is very compact and the two different ββ hairpins which are responsible for virtually all base-specific interactions do not constitute separate subdomains, but are part of a single fold. For example, in the internal part of the DNA binding regions of I-*Cre*I, the gtc triplet is bound by one residue from the first hairpin (Q44), and two residues from the second hairpin (R68 and R70; see figure 1B of Chevalier *et al.,* 2003, precited).

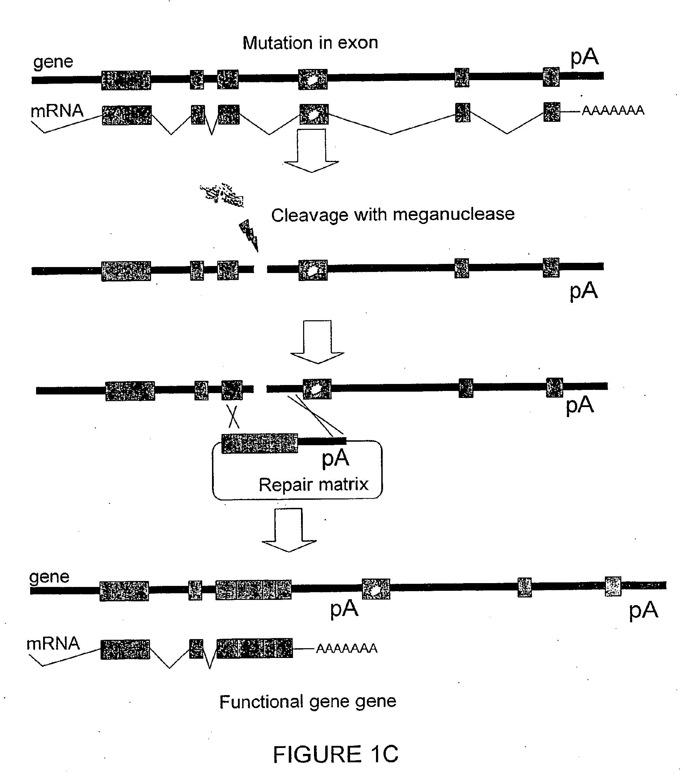

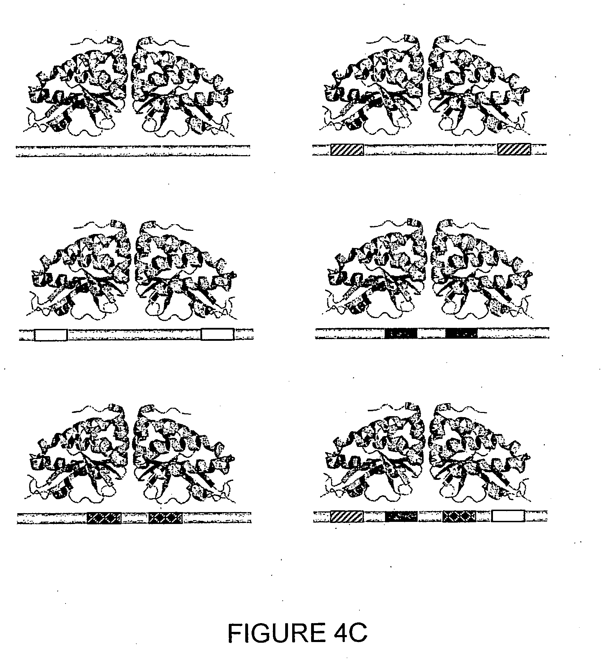

A semi rational design assisted by yeast high throughput screening method allowed the Inventors, to identify and isolate thousands of I-*Cre*I variants in positions 28, 30, 33, 38 and 40 with altered specificities in positions ± 8 to 10 (10NNN DNA target). These new proteins were designed to cleave one of the 64 targets degenerate at nucleotides ±10, ±9, ±8 (10NNN DNA target) of the I-*Cre*I original target site (figure 3). Furthermore, in spite of the lack of apparent modularity at the structural level, residues 28 to 40 binding to positions ± 8 to 10 and residues 44 to 77 binding to positions ± 3 to 5 of the I-*Cre*I site, were revealed to form two separable functional subdomains, able to bind distinct parts of an I-*Cre*I homing endonuclease half-site (figure 3 and figure 4B). By assembling two subdomains from different monomers or core domains within the same monomer, the Inventors have engineered functional homing endonuclease (homodimeric) variants, which are able to cleave palindromic chimeric targets (figure 4B) having the nucleotides in positions ± 3 to 5 and ± 8 to 10 of each parent monomer/core domain. Furthermore, a larger combinatorial approach is allowed by assembling four different subdomains (figure 4C: top right, middle left and right, bottom left) to form new heterodimeric molecules which are able to cleave non-palindromic chimeric targets (bottom right). The different subdomains can be modified separately and combine in one meganuclease variant (heterodimer or single-chain molecule) which is able to cleave a target from a gene of interest. The engineered variant can be used for gene correction via double-strand break induced recombination (figure 1B and 1C).

The capacity to combine four sub-domains considerably increases the number of DNA sequences that can be targeted (figure 4C). However, it is still difficult to fully appreciate the range of sequences that can be reached with this combinatorial approach. One of the most elusive factors is the impact of the four central nucleotides of the I-*Cre*I target site (gtac in the palindromic I-*Cre*I site C1221, figure 3). Even though the base-pairs ±1 and ±2 do not display any contact with the protein, it has been shown that these positions are not devoid of content information (Chevalier et al., J. Mol. Biol., 2003, 329, 253-269), especially for the base-pair ±1 and could be a source of additional substrate specificity (Argast et al., J. Mol. Biol., 1998, 280, 345-353; Jurica et al., Mol. Cell., 1998, 2, 469-476; Chevalier, B.S. and B.L. Stoddard, Nucleic Acids Res., 2001, 29, 3757-3774). *In vitro* selection of cleavable I-*Cre*I target (Argast *et al.,* precited) randomly mutagenized, revealed the importance of these four base-pairs on protein binding and cleavage activity. It has been suggested that the network of ordered water molecules found in the active site was important for positioning the DNA target (Chevalier et al., Biochemistry, 2004, 43, 14015-14026). In addition, the extensive conformational changes that appear in this region upon I-*Cre*I binding suggest that the four central nucleotides could contribute to the substrate specificity, possibly by sequence dependent conformational preferences (Chevalier *et al.,* 2003, precited).

Thus, it was not clear if mutants identified on 10NNN and 5NNN DNA targets as homodimers cleaving a palindromic sequence with the four central nucleotides being gtac, would allow the design of new endonucleases that would cleave targets containing changes in the four central nucleotides.

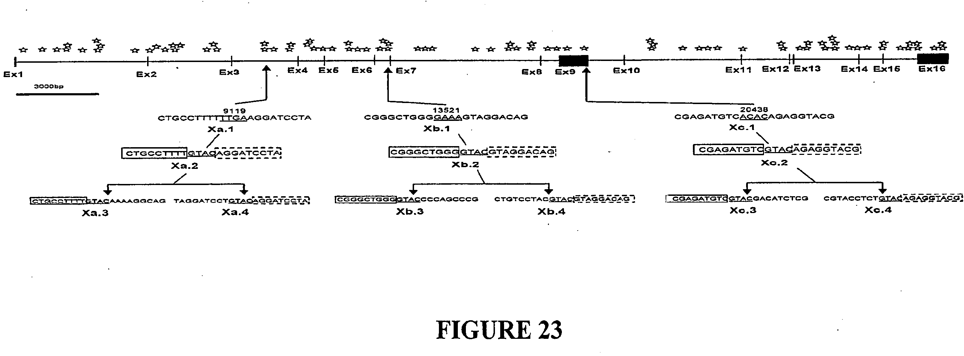

The Inventors have identified hundreds of DNA targets in the *XP* genes that could be cleaved by I-*Cre*I variants. The combinatorial strategy described in figure 4 was used to extensively redesign the DNA binding domain of the *I-CreI* protein and thereby engineer novel meganucleases with fully engineered specificity, to cleave two DNA targets from the *XPC* gene (Xa.1 and Xc.1) which differ from the I-*Cre*I C1221 22 bp palindromic site by 17 nucleotides including the four central nucleotides in positions ±1 to 2 (Xa.1, Figures 3,9 and 23) or 11 nucleotides including two (positions - 1 and -2) of the four central nucleotides (Xc.1, Figure 23).

Even though the combined variants were initially identified towards nucleotides 10NNN and 5NNN respectively, and a strong impact of the four central nucleotides of the target on the activity of the engineered meganuclease was observed, functional meganucleases with a profound change in specificity regarding the other base-pairs of the target were selected. Furthermore, the activity of the engineered protein could be significantly improved by two successive rounds of random mutagenesis and screening, to compare with the activity of the I-*Cre*I protein. Finally, the extensive redesign of the DNA binding domain is not made at the expense of the level of specificity, the novel endonucleases keeping a very narrow numbers of cleavable cognate targets.

These I-*Cre*I variants which are able to cleave a DNA target sequence from a *XP* gene can be used for repairing the mutations associated with Xeroderma pigmentosum. Other potential applications include genome engineering at the *XP* genes loci.

The invention relates to an I-*Cre*I variant which has at least two substitutions, one in each of the two functional subdomains of the LAGLIDADG core domain situated from positions 28 to 40 and 44 to 70 of I-*Cre*I amino acid sequence SEQ ID NO: 217, said variant being able to cleave a DNA target sequence selected from the group consisting of the sequences SEQ ID NO: 1 to 24 and 45 to 216 from a human xeroderma pigmentosum gene, and being obtainable by a method comprising at least the steps of:
(a) constructing a first series of I-*Cre*I variants having at least one substitution in positions 28, 30, 32, 33, 38 and/or 40 of a first functional subdomain of the LAGLIDADG core domain situated from positions 28 to 40 of I-*Cre*I,
(b) constructing a second series of I-*Cre*I variants having at least one substitution in positions 44, 68 and/or 70 of a second functional subdomain of the LAGLIDADG core domain situated from positions 44 to 70 of I-*Cre*I,
(c) selecting the variants from the first series of step (a) which are able to cleave a mutant I-*Cre*I site wherein (i) the nucleotide triplet in positions -10 to -8 of the I-*Cre*I site has been replaced with the nucleotide triplet which is present in positions -10 to -8 of a genomic DNA target selected from the group consisting of the sequences SEQ ID NO: 1 to 24 and 45 to 216 from a human xeroderma pigmentosum gene and (ii) the nucleotide triplet in positions +8 to +10 has been replaced with the reverse complementary sequence of the nucleotide triplet which is present in positions -10 to -8 of said genomic target,
(d) selecting the variants from the second series of step (b) which are able to cleave a mutant I-*Cre*I site wherein (i) the nucleotide triplet in positions -5 to -3 of the I-*Cre*I site has been replaced with the nucleotide triplet which is present in positions -5 to -3 of said genomic target in (c) and (ii) the nucleotide triplet in positions +3 to +5 has been replaced with the reverse complementary sequence of the nucleotide triplet which is present in positions -5 to - 3 of said genomic target,
(e) selecting the variants from the first series of step (a) which are able to cleave a mutant I-*Cre*I site wherein (i) the nucleotide triplet in positions +8 to +10 of the I-*Cre*I site has been replaced with the nucleotide triplet which is present in positions +8 to +10 of said genomic target in (c) and (ii) the nucleotide triplet in positions -10 to -8 has been replaced with the reverse complementary sequence of the nucleotide triplet which is present in position +8 to +10 of said genomic target,
(f) selecting the variants from the second series of step (b) which are able to cleave a mutant I-*Cre*I site wherein (i) the nucleotide triplet in positions +3 to +5 of the I-*Cre*I site has been replaced with the nucleotide triplet which is present in positions +3 to +5 of said genomic target in (c) and (ii) the nucleotide triplet in positions -5 to -3 has been replaced with the reverse complementary sequence of the nucleotide triplet which is present in positions +3 to +5 of said genomic target,
(g) combining in a single variant, the mutation(s) in positions 28 to 40 and 44 to 70 of two variants from step (c) and step (d), to obtain a novel homodimeric I-*Cre*I variant which cleaves a sequence wherein (i) the nucleotide triplet in positions -10 to -8 is identical to the nucleotide triplet which is present in positions -10 to -8 of said genomic target in (c), (ii) the nucleotide triplet in positions +8 to +10 is identical to the reverse complementary sequence of the nucleotide triplet which is present in positions -10 to -8 of said genomic target, (iii) the nucleotide triplet in positions -5 to -3 is identical to the nucleotide triplet which is present in positions -5 to -3 of said genomic target and (iv) the nucleotide triplet at positions +3 to +5 is identical to the reverse complementary sequence of the nucleotide triplet which is present at positions -5 to -3 of said genomic target,
(h) combining in a single variant, the mutation(s) in positions 28 to 40 and 44 to 70 of two variants from step (e) and step (f), to obtain a novel homodimeric I-*Cre*I variant which cleaves a sequence wherein (i) the nucleotide triplet in positions +3 to +5 is identical to the nucleotide triplet which is present in positions +3 to +5 of said genomic target in (c), (ii) the nucleotide triplet in positions - 5 to -3 is identical to the reverse complementary sequence of the nucleotide triplet which is present in positions +3 to +5 of said genomic target, (iii) the nucleotide triplet in positions +8 to +10 of the I-*Cre*I site has been replaced with the nucleotide triplet which is present in positions +8 to +10 of said genomic target and (iv) the nucleotide triplet in positions -10 to -8 is identical to the reverse complementary sequence of the nucleotide triplet in positions +8 to +10 of said genomic target,
(i) combining the variants obtained in steps (g) and (h) to form heterodimers, and
(j) selecting the heterodimers from step (i) which are able to cleave said DNA target sequence selected from the group consisting of the sequences SEQ ID NO: 1 to 24 and 45 to 216 from a human xeroderma pigmentosum gene.

The cleavage activity of the variant according to the invention may be measured by any well-known, *in vitro* or *in vivo* cleavage assay, such as those described in the International PCT Application WO 2004/067736 or in Arnould et al., J. Mol. Biol., 2006, 355: 443-458. For example, the cleavage activity of the variant of the invention may be measured by a direct repeat recombination assay, in yeast or mammalian cells, using a reporter vector. The reporter vector comprises two truncated, non-functional copies of a reporter gene (direct repeats) and the genomic DNA target sequence within the intervening sequence, cloned in a yeast or a mammalian expression vector. Expression of the variant results in a functional endonuclease which is able to cleave the genomic DNA target sequence. This cleavage induces homologous recombination between the direct repeats, resulting in a functional reporter gene, whose expression can be monitored by appropriate assay.

### Definitions

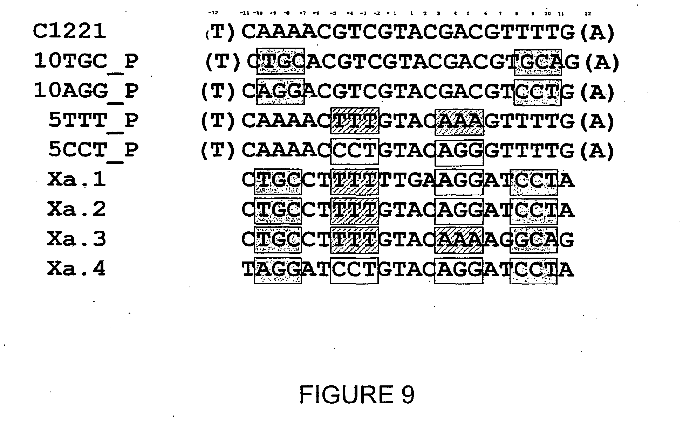

- Amino acid residues in a polypeptide sequence are designated herein according to the one-letter code, in which, for example, Q means Gln or Glutamine residue, R means Arg or Arginine residue and D means Asp or Aspartic acid residue.
- Nucleotides are designated as follows: one-letter code is used for designating the base of a nucleoside: a is adenine, t is thymine, c is cytosine, and g is guanine. For the degenerated nucleotides, r represents g or a (purine nucleotides), k represents g or t, s represents g or c, w represents a or t, m represents a or c, y represents t or c (pyrimidine nucleotides), d represents g, a or t, v represents g, a or c, b represents g, t or c, h represents a, t or c, and n represents g, a, t or c.
- by "I*-Cre*I" is intended the wild-type I-*Cre*I having the sequence SWISSPROT P05725, corresponding to SEQ ID NO: 217 in the sequence listing, or pdb accession code 1 g9y.
- by "I-*Cre*I variant" or "variant" is intended a protein obtained by replacement of at least one amino acid of I-*Cre*I with a different amino acid.
- by "functional I-*Cre*I variant" is intended a I-*Cre*I variant which is able to cleave a DNA target, preferably a DNA target which is not cleaved by I-*Cre*I. For example, such variants have amino acid variation at positions contacting the DNA target sequence or interacting directly or indirectly with said DNA target.
- by "I-*Cre*I variant with novel specificity" is intended a variant having a pattern of cleaved targets different from that of the parent meganuclease. The terms "novel specificity", "modified specificity", "novel cleavage specificity", "novel substrate specificity" which are equivalent and used indifferently, refer to the specificity of the variant towards the nucleotides of the DNA target sequence.
- by "I-*Cre*I site" is intended a 22 to 24 bp double-stranded DNA sequence which is cleaved by I-*Cre*I. I-*Cre*I sites include the wild-type (natural) non-palindromic I-*Cre*I homing site and the derived palindromic sequences such as the sequence 5'- t₁₂c-₁₁a-₁₀a-₉a-₈a-₇c-₆g-₅t-₄c-₃g-₂t-₁a+-₁c+₂g+₃a+₄c+₅g+₆t+₇t+-₈t+₉t+₁₀g+₁₁a+₁₂ (SEQ ID NO :25), also called C1221 (figures 3 and 9).
- by "domain" or "core domain" is intended the "LAGLIDADG Homing Endonuclease Core Domain" which is the characteristic α₁β₁β₂α₂β₃β₄α₃ fold of the homing endonucleases of the LAGLIDADG family, corresponding to a sequence of about one hundred amino acid residues. Said domain comprises four beta-strands (β₁, β₂, β₃, β₄) folded in an antiparallel beta-sheet which interacts with one half of the DNA target. This domain is able to associate with another LAGLIDADG Homing Endonuclease Core Domain which interacts with the other half of the DNA target to form a functional endonuclease able to cleave said DNA target. For example, in the case of the dimeric homing endonuclease I-*Cre*I (163 amino acids), the LAGLIDADG Homing Endonuclease Core Domain corresponds to the residues 6 to 94.
- by "subdomain" is intended the region of a LAGLIDADG Homing Endonuclease Core Domain which interacts with a distinct part of a homing endonuclease DNA target half-site. Two different subdomains behave independently and the mutation in one subdomain does not alter the binding and cleavage properties of the other subdomain. Therefore, two subdomains bind distinct part of a homing endonuclease DNA target half-site.
- by "beta-hairpin" is intended two consecutive beta-strands of the antiparallel beta-sheet of a LAGLIDADG homing endonuclease core domain (β₁β₂ or, β₃β₄) which are connected by a loop or a turn,
- by "single-chain meganuclease", "single-chain chimeric meganuclease", "single-chain meganuclease derivative", "single-chain chimeric meganuclease derivative" or "single-chain derivative", is intended a meganuclease comprising two LAGLIDADG homing endonuclease domains or core domains linked by a peptidic spacer. The single-chain meganuclease is able to cleave a chimeric DNA target sequence comprising one different half of each parent meganuclease target sequence.
- by "DNA target", "DNA target sequence", "target sequence", "target-site", "target" , "site"; "site of interest"; "recognition site", "recognition sequence", "homing recognition site", "homing site", "cleavage site" is intended a 20 to 24 bp double-stranded palindromic, partially palindromic (pseudo-palindromic) or non-palindromic polynucleotide sequence that is recognized and cleaved by a LAGLIDADG homing endonuclease. These terms refer to a distinct DNA location, preferably a genomic location, at which a double stranded break (cleavage) is to be induced by the endonuclease. The DNA target is defined by the 5' to 3' sequence of one strand of the double-stranded polynucleotide, as indicated above for C1221. Cleavage of the DNA target occurs at the nucleotides in positions +2 and -2, respectively for the sense and the antisense strand. Unless otherwiwe indicated, the position at which cleavage of the DNA target by an I-*Cre* I meganuclease variant occurs, corresponds to the cleavage site on the sense strand of the DNA target.
- by "DNA target half-site", "half cleavage site" or half-site" is intended the portion of the DNA target which is bound by each LAGLIDADG homing endonuclease core domain.
- by "chimeric DNA target" or "hybrid DNA target" is intended the fusion of a different half of two parent meganucleases target sequences. In addition at least one half of said target may comprise the combination of nucleotides which are bound by at least two separate subdomains (combined DNA target)..
- by *"XP* gene" is intended a gene of one the xeroderma pigmentosum complementation groups (*XP-A, XP-B, XP-C, XP-D, XP-E, XP-F*) of a mammal. For example, the human *XP* genes are available in the NCBI database, under the indicated accession numbers: XPA: GeneID:7507, ACCESSION NC_000009, REGION: complement (97516747..97539194); XPB: GeneID:2071, ACCESSION NC_000002, REGION: complement (127731096..127767982); XPC: GeneID:7508, ACCESSION NC_000003, REGION: complement (14161651..14195087); XPD: GeneID:2068, ACCESSION NC_000019, REGION: complement(50546686..50565669); XPE: GeneID:1642, ACCESSION NC_000011, REGION: complement (60823502..60857125); XPF: GeneID:2072, ACCESSION NC_000016, REGION: 13921524..13949705; XPG: GeneID:2073, ACCESSION NC_000013, REGION: 102296421..102326346.
- by "DNA target sequence from a *XP* gene" "genomic DNA target sequence", " genomic DNA cleavage site", "genomic DNA target" or "genomic target" is intended a 20 to 24 bp sequence of a *XP* gene of a mammal which is recognized and cleaved by a meganuclease variant or a single-chain chimeric meganuclease derivative.
- by "vector" is intended a nucleic acid molecule capable of transporting another nucleic acid to which it has been linked.
- by "homologous" is intended a sequence with enough identity to another one to lead to a homologous recombination between sequences, more particularly having at least 95 % identity, preferably 97 % identity and more preferably 99%.
- "Identy" refers to sequence identity between two nucleic acid molecules or polypeptides. Identity can be determined by comparing a position in each sequence which may be aligned for purposes of comparison. When a position in the compared sequence is occupied by the same base, then the molecules are identical at that position. A degree of similarity or identity between nucleic acid or amino acid sequences is a function of the number of identical or matching nucleotides at positions shared by the nucleic acid sequences. Various alignment algorithms and/or programs may be used to calculate the identity between two sequences, including FASTA, or BLAST which are available as a part of the GCG sequence analysis package (University of Wisconsin, Madison, Wis.), and can be used with, e.g., default settings.
- "individual" includes mammals, as well as other vertebrates (e.g., birds, fish and reptiles). The terms "mammal" and "mammalian", as used herein, refer to any vertebrate animal, including monotremes, marsupials and placental, that suckle their young and either give birth to living young (eutharian or placental mammals) or are egg-laying (metatharian or nonplacental mammals). Examples of mammalian species include humans and other primates (e.g., monkeys, chimpanzees), rodents (e.g., rats, mice, guinea pigs) and ruminants (e.g., cows, pigs, horses).
- by mutation is intended the substitution, deletion, addition of one or more nucleotides/amino acids in a polynucleotide (cDNA, gene) or a polypeptide sequence. Said mutation can affect the coding sequence of a gene or its regulatory sequence. It may also affect the structure of the genomic sequence or the structure/stability of the encoded mRNA.

According to the present invention, the positions of the mutations are indicated by reference to the I-*Cre*I amino acid sequence SEQ ID NO: 217.

In a preferred embodiment of said variant, it comprises the substitution of the aspartic acid in position 75 by an uncharged amino acid, preferably an asparagine (D75N) or a valine (D75V).

In another preferred embodiment of said variant, it comprises one or more additional substitutions in positions 19, 24, 26, 42, 69, 75, 77, 80, 85, 87, 87, 109, 133 and 161 of I*-Cre*I. The substitutions may improve the binding and/or the cleavage properties of the variant towards the DNA target sequence of the human *XP* gene. The additional residues which are mutated may be on the entire I-*Cre*I sequence. The mutations may affect the active site (position 19), the protein-DNA interface (for example, position 69), the hydrophobic core (for example, positions 85, 87 or 109) or the C-terminal part (for example, position 161). The substitutions may also be at additional positions contacting the DNA target sequence or interacting directly or indirectly with said DNA target. The I-*Cre*I interacting residues are well-known in the art. The residues which are mutated may interact with the DNA backbone or with the nucleotide bases, directly or via water molecule.

In yet another preferred embodiment of said variant, said substitutions are replacement of the initial amino acids with amino acids selected from the group consisting of: A, D, E, G, H, K, N, P, Q, R, S, T, Y, L and V.

The variant according to the present invention is an heterodimer, resulting from the association of a first and a second monomer having different mutations in positions 28 to 40 and 44 to 70 of I-*Cre*I, said heterodimer being able to cleave a non-palindromic DNA target sequence selected from the group consisting of the sequences SEQ ID NO: 1 to 24 and 45 to 216 from a human *XP* gene (*XPA* to *XPG* genes).

The DNA target sequence which is cleaved by said variant may be in an exon or in an intron of the human *XP* gene. Preferably, it is located, either in the vicinity of a mutation, preferably within 500 bp of the mutation, or upstream of a mutation, preferably upstream of all the mutations of said *XP* gene.

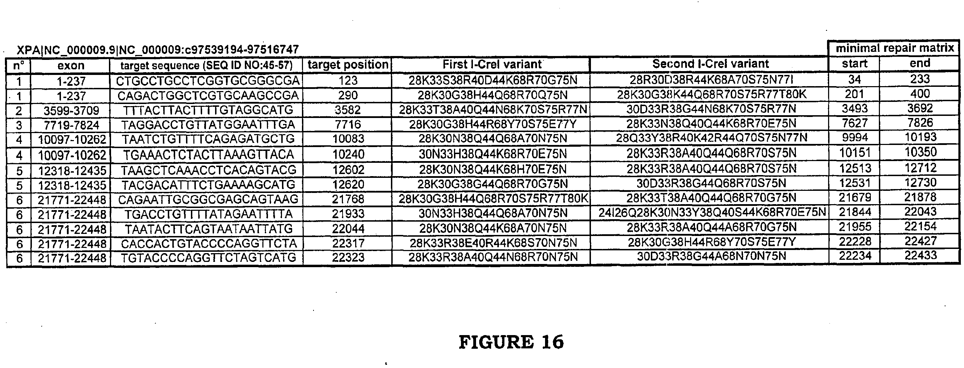

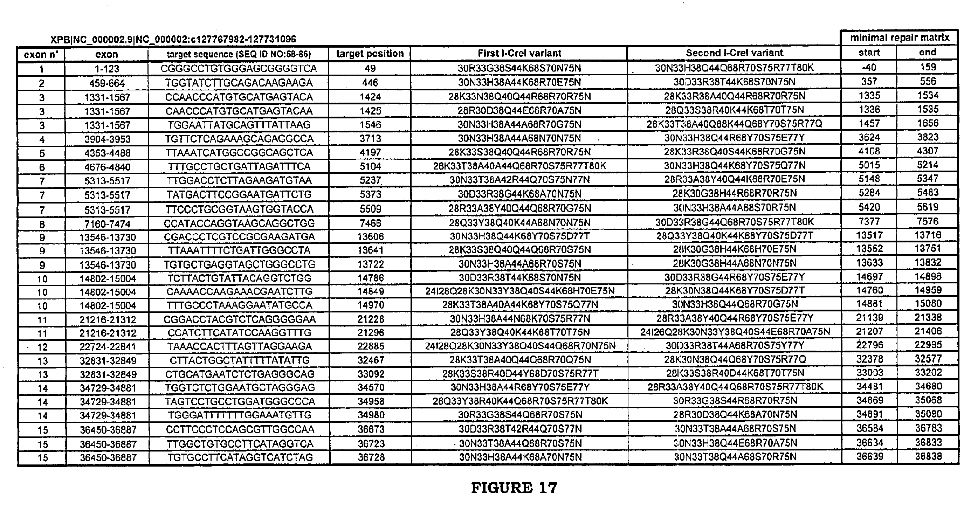

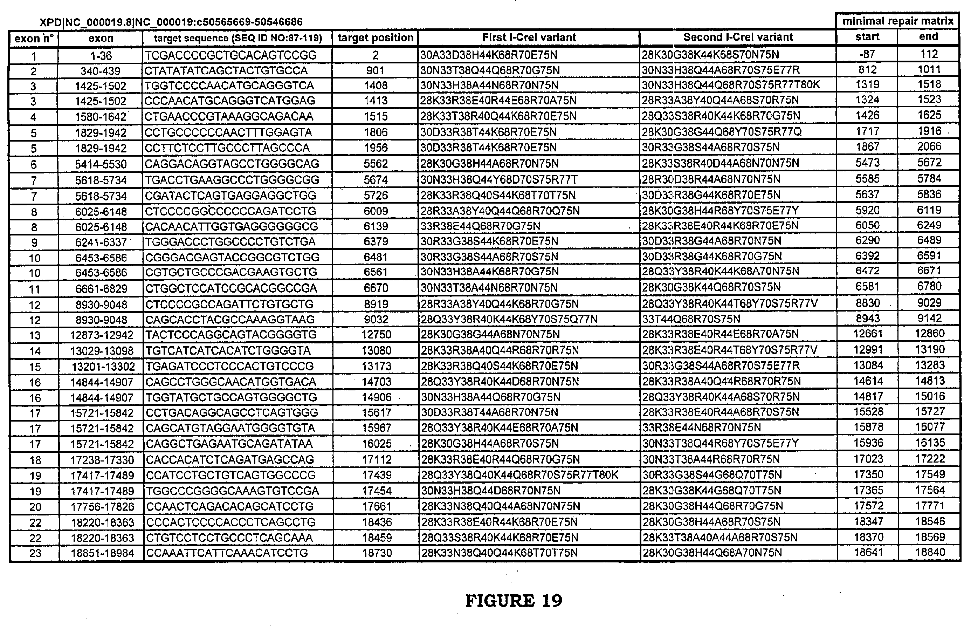

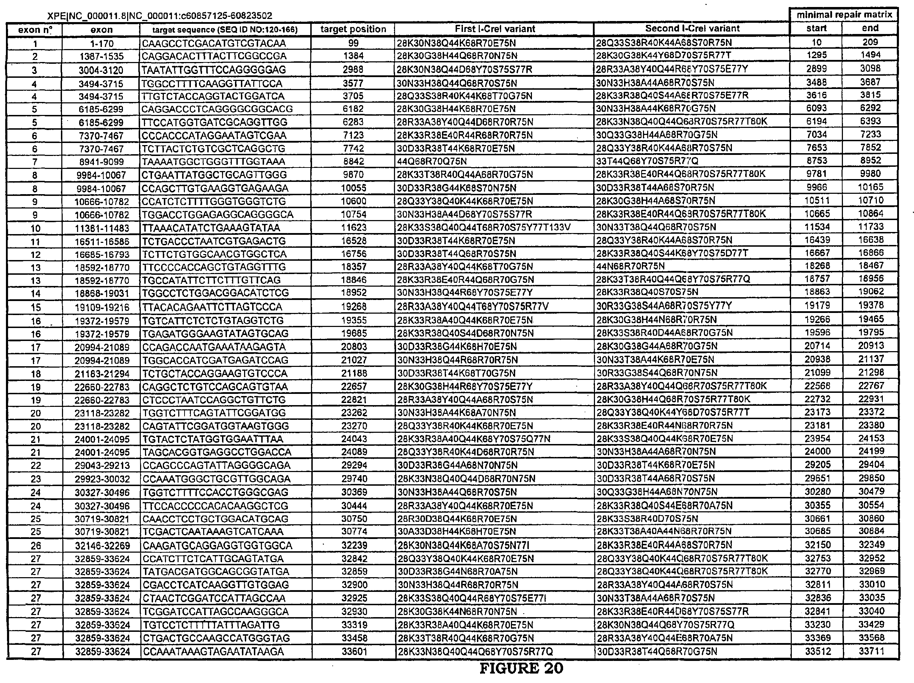

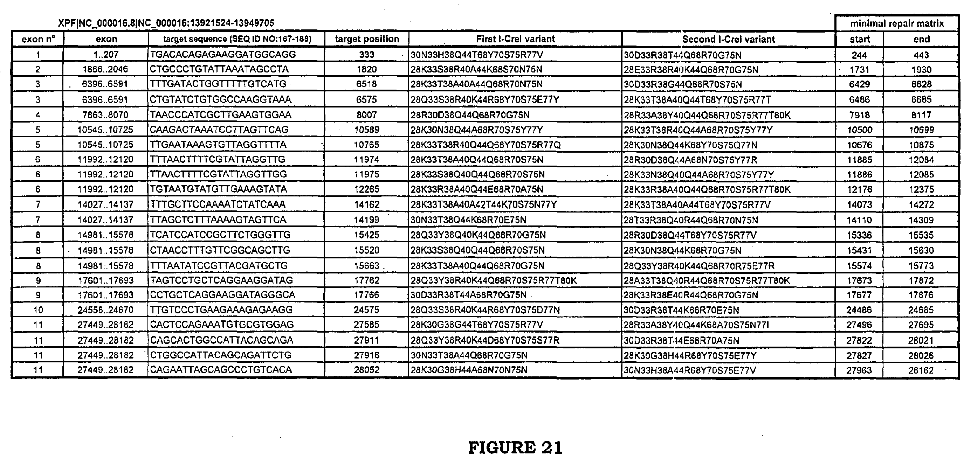

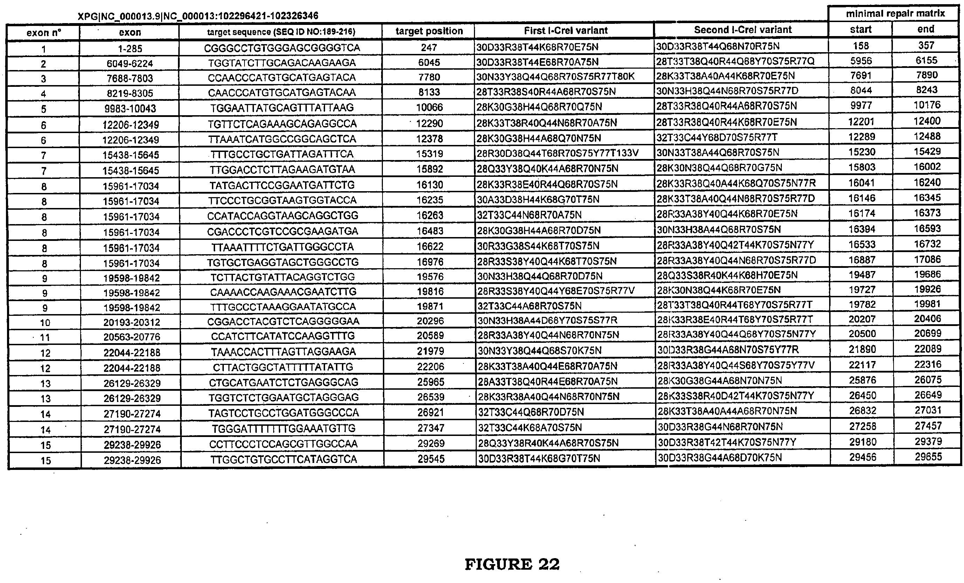

DNA targets from each human *XP* gene are presented in Tables IX to XV and figures 16 to 22.

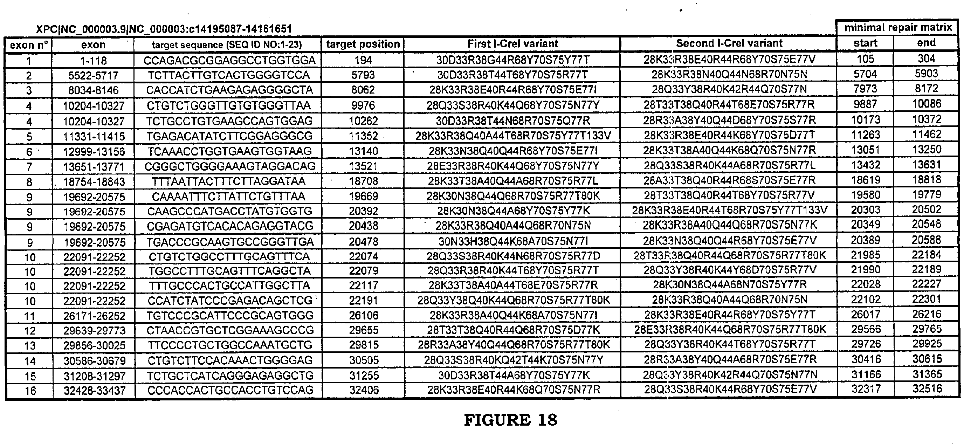

For example, the sequences SEQ ID NO: 1 to 24 are DNA targets from the *XPC* gene ; SEQ ID NO: 1 to 23 are situated in or close to one of the exons and these sequences cover all the exons of the *XP* gene (Table XI and figure 18). The target sequence SEQ ID NO: 24 (Xa.1) is situated in the third intron, upstream of the mutations (figure 1A). The target sequence SEQ ID NO: 12 (Xc.1) is situated in Exon 9, in the vicinity of the deletion 1132AA and the insertion insVAL580 (Figure 1A).

Hererodimeric variants which cleave each DNA target are presented in Tables I to VIII and figures 16 to 22.

The sequence of each variant is defined by its amino acid residues at the indicated positions. For example, the first heterodimeric variant of Table I consists of a first monomer having K, S, R, D, K, R, G and N in positions 28, 33, 38, 40, 44, 68, 70 and 75, respectively and a second monomer having R, D, R, K, A, S, N and I in positions 28, 30, 38, 44, 68, 70, 75 and 77, respectively. The positions are indicated by reference to I-*Cre*I sequence SWISSPROT P05725, SEQ ID NO: 217 or pdb accession code 1g9y; I-*Cre*I has G, I, Q, K, N, S, Y, Q, S, A, Q, R,D, R, D, I, E, H, F, I, A and S, in positions 19, 24, 26, 28, 30, 32, 33, 3 8, 40, 42, 44, 68, 69, 70, 75, 77, 80, 85, 87, 109, 133 and 161, respectively. The variant may consist of an I-*Cre*I sequence having the amino acid residues as indicated in the Table. In this case, the positions which are not indicated are not mutated and thus correspond to the wild-type I-*Cre*I sequence. Alternatively, the variant may comprise an I-*Cre*I sequence having the amino acid residues as indicated in the Table. In the latter cause, the positions which are not indicated may comprise mutations as defined above, or may not be mutated. For example, the variant may be derived from an I-*Cre*I scaffold protein encoded by SEQ ID NO: 26, said I-*Cre*I scaffold protein (SEQ ID NO: 218) differing from the *Cre*-I sequence SWISSPROT P05725 or SEQ ID NO:217 by the insertion of an alanine at position 2, the substitutions A42T, D75N, W110E and R111Q and three additional amino acids (A, A and D) at the C-terminus. In addition, said variant, derived from wild-type I-*Cre*I or an I-*Cre*I scaffold protein, may comprise additional mutations, as defined above.

The target which is cleaved by each heterodimeric variant is indicated in the last column of the Table.

**Table I: Sequence of heterodimeric I-CreI variants having a DNA target site in or close to one exon of the XPA gene**

| **First monomer** | **Second monomer** | **Exon closest to the target sequence (SEQ ID NO: 45 to 57)** |
|---|---|---|
| 28K33S38R40D44K68R70G75N | 28R30D38R44K68A70S75N771 | Exon 1 |
| 28K30G38H44Q68R70Q75N | 28K30G38K44Q68R70S75R77T80K | Exon 1 |
| 28K33T38A40Q44N68K70S75R77N | 30D33R38G44N68K70S75R77N | Exon 2 |
| 28K30G38H44R68Y70S75E77Y | 28K33N38Q40Q44K68R70E75N | Exon 3 |
| 28K30N38Q44Q68A70N75N | 28Q33Y38R40K42R44Q70S75N77N | Exon 4 |
| 30N33H38Q44K68R70E75N | 28K33R38A40Q44Q68R70S75N | Exon 4 |
| 28K30N38Q44K68H70E75N | 28K33R38A40Q44Q68R70S75N | Exon 5 |
| 28K30G38G44Q68R70G75N | 30D33R38G44Q68R70S75N | Exon 5 |
| 28K30G38H44Q68R70S75R77T80K | 28K33T38A40Q44Q68R70G75N | Exon 6 |
| 30N33H38Q44Q68A70N75N | 24126Q28K30N33Y38Q40S44K68R70E75N | Exon 6 |
| 28K30N38Q44K68A70N75N | 28K33R38A40Q44A68R70G75N | Exon 6 |
| 28K33R38E40R44K68S70N75N | 28K30G38H44R68Y70S75E77Y | Exon 6 |
| 28K33R38A40Q44N68R70N75N | 30D33R38G44A68N70N75N | Exon 6 |

**Table II: Sequence of heterodimeric I-CreI variants having a DNA target site in or close to one exon of the XPB gene**

| **First monomer** | **Second monomer** | **Exon closest to the target sequence (SEQ ID NO: 58 to 86)** |
|---|---|---|
| 30R33G38S44K68S70N75N | 30N33H38Q44Q68R70S75R77T80K | exon 1 |
| 30N33H38A44K68R70E75N | 30D33R38T44K68S70N75N | exon 2 |
| 28K33N38Q40Q44R68R70R75N | 28K33R38A40Q44R68R70R75N | exon 3 |
| 28R30D38Q44E68R70A75N | 28Q33S38R40K44K68T70T75N | exon 3 |
| 30N33H38A44A68R70G75N | 28K33T38A40Q44Q68Y70S75R77Q | exon 3 |
| 30N33H38A44A68R70N75N | 30N33H38A44A68Y70S75E77Y | exon 4 |
| 28K33S38Q40Q44R68R70R75N | 28K33R38Q40S44K68R70G75N | exon 5 |
| 28K33T38A40A44Q68R70S75R77T80K | 30N33H38Q44K68Y70S75Q77N | exon 6 |
| 30N33T38A42R44Q70S75N77N | 28R33A38Y40Q44K68R70E75N | exon 7 |
| 30D33R38G44K68A70N75N | 28K30G38H44R68R70R75N | exon 7 |
| 28R33A38Y40Q44Q68R70G75N | 30N33H38A44A68S70R75N | exon 7 |
| 28Q33Y38Q40K44A68N70N75N | 30D33R38G44Q68R70S75R77T80K | exon 8 |
| 30N33H38Q44K68Y70S75D77T | 28Q33Y38Q40K44K68Y70S75D77T | exon 9 |
| 28K33S38Q40Q44Q68R70S75N | 28K30G38H44K68H70E75N | exon 9 |
| 30N33H38A44A68R70S75N | 28K30G38H44A68N70N75N | exon 9 |
| 30D33R38T44K68S70N75N | 30D33R38G44R68Y70S75E77Y | exon 10 |
| 24126028K30N33Y38040S44K68H70E75N | 28K30N38Q44K68Y70S75D77T | exon 10 |
| 28K33T38A40A44K68Y70S75Q77N | 30N33H38Q44Q68R70G75N | exon 10 |
| 30N33H38A44N68K70S75R77N | 28R33A38Y40Q44R68Y70S75E77Y | exon 11 |
| 28Q33Y38Q40K44K68T70T75N | 24126Q28K30N33Y38Q40S44E68R70A75N | exon 11 |
| 24126Q28K30N33Y38Q40S44Q68R70N75N | 30D33R38T44A68R70S75Y77Y | exon 12 |
| 28K33T38A40Q44Q68R70Q75N | 28K30N38044Q68Y70S75R77Q | exon 13 |
| 28K33S38R40D44Y68D70S75R77T | 28K33S38R40D44K68T70T75N | exon 13 |
| 30N33H38A44R68Y70S75E77Y | 28R33A38Y40Q44Q68R70S75R77T80K | exon 14 |
| 28Q33Y38R40K44Q68R70S75R77T80K | 30R33G38S44R68R70R75N | exon 14 |
| 30R33G38S44Q68R70S75N | 28R30D38Q44K68A70N75N | exon 14 |
| 30D33R38T42R44Q70S77N | 30N33T38A44A68R70S75N | exon 15 |
| 30N33T38A44Q68R70S75N | 30N33H38Q44E68R70A75N | exon 15 |
| 30N33H38A44K68A70N75N | 30N33T38Q44A68S70R75N | exon 15 |

**Table III: Sequence of heterodimeric I-CreI variants having a DNA target site in or close to one exon of the XPC gene**

| **First monomer** | **Second monomer** | **Exon closest to the target sequence (SEQ ID NO: 1 to 23)** |
|---|---|---|
| 30D33R38G44R68Y70S75Y77T | 28K33R38E40R44R68Y70S75E77V | exon 1 |
| 30D33R38T44T68Y70S75R77T | 28K33R38N40Q44N68R70N75N | exon 2 |
| 28K33R38E40R44R68Y70S75E771 | 28Q33Y38R40K42R44Q70S77N | exon 3 |
| 28Q33S38R40K44Q68Y70S75N77Y | 28T33T38Q40R44T68E70S75R77R | exon 4 |
| 30D33R38T44N68R70S75Q77R | 28R33A38Y40Q44D68Y70S75S77R | exon 4 |
| 28K33R38Q40A44T68R70S75Y77T133V | 28K33R38E40R44K68Y70S75D77T | exon 5 |
| 28K33N38Q40Q44R68Y70S75E771 | 28K33T38A40Q44K68Q70S75N77R | exon 6 |
| 28E33R38R40K44Q68Y70S75N77Y | 28Q33S38R40K44A68R70S75R77L | exon 7 |
| 28K33T38A40Q44A68R70S75R77L | 28A33T38Q40R44R68S70S75E77R | exon 8 |
| 28K30N38Q44Q68R70S75R77T80K | 28T33T38Q40R44T68Y70S75R77V | exon 9 |
| 28K30N38Q44A68Y70S75Y77K | 28K33R38E40R44T68R70S75Y77T133V | exon 9 |
| 28K33R38Q40A44Q68R70N75N | 28K33R38A40Q44Q68R70S75N77K | exon 9 |
| 33H75N | 33R38A40Q44K70N75N | exon 9 |
| 30N33H38Q44K68A70S75N771 | 28K33N38Q40Q44R68Y70S75E77V | exon 9 |
| 28Q33S38R40K44N68R70S75R77D | 28T33R38Q40R44Q68R70S75R77T80K | exon 10 |
| 28Q33R38R40K44T68Y70S75R77T | 28Q33Y38R40K44Y68D70S75R77V | exon 10 |
| 28K33T38A40A44T68E70S75R77R | 28K30N38Q44A68N70S75Y77R | exon 10 |
| 28Q33Y38Q40K44Q68R70S75R77T80K | 28K33R38Q40A44Q68R70N75N | exon 10 |
| 28K33R38A40Q44K68A70S75N771 | 28K33R38E40R44R68Y70S75Y77T | exon 11 |
| 28T33T38Q40R44Q68R70S75D77K | 28E33R38R4OK44Q68R7OS75R77T80K | exon 12 |
| 28R33A38Y40Q44Q68R70S75R77T80K | 28Q33Y38R40K44T68Y70S75R77T | exon 13 |
| 28Q33S38R40K42T44K70S75N77Y | 28R33A38Y40Q44A68R70S75E77R | exon 14 |
| 30D33R38T44A68Y70S75Y77K | 28Q33Y38R40K42R44Q70S75N77N | exon 15 |
| 28K33R38E40R44K68Q70S75N77R | 28Q33S38R40K44R68Y70S75E77V | exon 16 |

**Table IV: Sequence of heterodimeric I-CreI variants having a DNA target site in or close to one exon of the XPD gene**

| **First monomer** | **Second monomer** | **Exon closest to the target sequence (SEQ ID NO: 87 to 119)** |
|---|---|---|
| 30A33D38H44K68R70E75N | 28K30G38K44K68S70N75N | exon 1 |
| 30N33T38Q44Q68R70G75N | 30N33H38Q44A68R70S75E77R | exon 2 |
| 30N33H38A44N68R70N75N | 30N33H38Q44Q68R70S75R77T80K | exon 3 |
| 28K33R38E40R44E68R70A75N | 28R33A38Y40Q44A68S70R75N | exon 3 |
| 28K33T38R40Q44K68R70E75N | 28Q33S38R40K44K68R70G75N | exon 4 |
| 30D33R38T44K68R70E75N | 28K30G38G44Q68Y70S75R77Q | exon 5 |
| 30D33R38T44K68R70E75N | 30R33G38S44A68R70S75N | exon 5 |
| 28K30G38H44A68R70N75N | 28K33S38R40D44A68N70N75N | exon 6 |
| 30N33H38Q44Y68D70S75R77T | 28R30D38R44A68N70N75N | exon 7 |
| 28K33R38Q40S44K68T70T75N | 30D33R38G44K68R70E75N | exon 7 |
| 28R33A38Y40Q44Q68R70Q75N | 28K30G38H44R68Y70S75E77Y | exon 8 |
| 33R38E44Q68R70G75N | 28K33R38E40R44K68R70E75N | exon 8 |
| 30R33G38S44K68R70E75N | 30D33R38G44A68R70N75N | exon 9 |
| 30R33G38S44A68R70S75N | 30D33R38G44K68R70G75N | exon 10 |
| 30N33H38A44K68R70G75N | 28Q33Y38R40K44K68A70N75N | exon 10 |
| 30N33T38A44N68R70N75N | 28K30G38K44Q68R70S75N | exon 11 |
| 28R33A38Y40Q44K68R70G75N | 28Q33Y38R40K44T68Y70S75R77V | exon 12 |
| 28Q33Y38R40K44K68Y70S75Q77N | 33T44Q68R70S75N | exon 12 |
| 28K30G38G44A68N70N75N | 28K33R38E40R44E68R70A75N | exon 13 |
| 28K33R38A40Q44R68R70R75N | 28K33R38E40R44T68Y70S75R77V | exon 14 |
| 28K33R38Q40S44K68R70E75N | 30R33G38S44A68R70S75E77R | exon 15 |
| 28Q33Y38R40K44D68R70N75N | 28K33R38A40Q44R68R70R75N | exon 16 |
| 30N33H38A44Q68R70G75N | 28Q33Y38R40K44A68S70R75N | exon 16 |
| 30D33R38T44A68R70N75N | 28K33R38E40R44A68R70S75N | exon 17 |
| 28Q33Y38R40K44E68R70A75N | 33R38E44N68R70N75N | exon 17 |
| 28K30G38H44A68R70S75N | 30N33T38Q44R68Y70S75E77Y | exon 17 |
| 28K33R38E40R44Q68R70G75N | 30N33T38A44R68R70R75N | exon 18 |
| 28Q33Y38Q40K44Q68R70S75R77T80K | 30R33G38S44G68Q70T75N | exon 19 |
| 30N33H38Q44D68R70N75N | 28K30G38K44G68Q70T75N | exon 19 |
| 28K33N38Q40Q44A68N70N75N | 28K30G38H44Q68R70G75N | exon 20 |
| 28K33R38E40R44K68R70E75N | 28K30G38H44A68R70S75N | exon 22 |
| 28Q33S38R40K44K68R70E75N | 28K33T38A40A44A68R70S75N | exon 22 |
| 28K33N38Q40Q44K68T70T75N | 28K30G38H44Q68A70N75N | exon 23 |

**Table V: Sequence of heterodimeric I-CreI variants having a DNA target site in or close to one exon of the XPE gene**

| **First monomer** | **Second monomer** | **Exon closest to the target sequence (SEQ ID NO: 120 to 166)** |
|---|---|---|
| 28K30N38Q44K68R70E75N | 28Q33S38R40K44A68S70R75N | exon 1 |
| 28K30G38H44Q68R70N75N | 28K30G38K44Y68D70S75R77T | exon 2 |
| 28K30N38Q44D68Y70S75S77R | 28R33A38Y40Q44R68Y70S75E77Y | exon 3 |
| 30N33H38Q44Q68R70S75N | 30N33H38A44A68R70S75N | exon 4 , |
| 28Q33S38R40K44K68T70G75N | 28K33R38Q40S44A68R70S75E77R | exon 4 |
| 28K30G38H44K68R70E75N | 30N33H38A44K68R70G75N | exon 5 |
| 28R33A38Y40Q44D68R70R75N | 28K33N38Q40Q44Q68R70S75R77T80K | exon 5 |
| 28K33R38E40R44R68R70R75N | 30Q33G38H44A68R70G75N | exon 6 |
| 30D33R38T44K68R70E75N | 28Q33Y38R40K44A68R70S75N | exon 6 |
| 44Q68R70Q75N | 33T44Q68Y70S75R77Q | exon 7 |
| 28K33T38R40Q44A68R70G75N | 28K33R38E40R44Q68R70S75R77T80K | exon 8 |
| 30D33R38G44K6BS70N75N | 30D33R38T44A68S70R75N | exon 8 |
| 28Q33Y38Q40K44K68R70E75N | 28K30G38H44A68S70R75N | exon 9 |
| 30N33H38A44D68Y70S75S77R | 28K33R38E40R44Q68R70S75R77T80K | exon 9 |
| 28K33S38Q40Q44T68R70S75Y77T133V | 30N33T38Q44Q68R70S75N | exon 10 |
| 30D33R38T44K68R70E75N | 28Q33Y38R40K44A68S70R75N | exon 11 |
| 30D33R38T44Q68R70S75N | 28K33R38Q40S44K68Y70S75D77T | exon 12 |
| 28R33A38Y40Q44K68T70G75N | 44N68R70R75N | exon 13 |
| 28K33R38E40R44Q68R70G75N | 28K33T38R40Q44Q68Y70S75R77Q | exon 13 |
| 30N33H38Q44R68Y70S75E77Y | 28K33R38Q40S70S75N | exon 14 |
| 28R33A38Y40Q44T68Y70S75R77V | 30R33G38S44A68R70S75Y77Y | exon 15 |
| 28K33R38A40Q44K68R70E75N | 28K30G38H44N68R70R75N | exon 16 |
| 28K33R38Q4OS44D68R7ON75N | 28K33S38R40D44A68R70G75N | exon 16 |
| 30D33R38G44K68H70E75N | 28K30G38G44A68R70G75N | exon 17 |
| 30N33H38Q44R68R70R75N | 30N33T38A44K68R70E75N | exon 17 |
| 30D33R38T44K68T70G75N | 30R33G38S44Q68R70N75N | exon 18 |
| 28K30G38H44R68Y70S75E77Y | 28R33A38Y40Q44Q68R70S75R77T80K | exon 19 |
| 28R33A38Y40Q44A68R70S75N | 28K30G38H44Q68R70S75R77T80K | exon 19 |
| 30N33H38A44K68A70N75N | 28Q33Y38Q40K44Y68D70S75R77T | exon 20 |
| 28Q33Y38R40K44K68R70E75N | 28K33R38E40R44N68R70R75N | exon 20 |
| 28K33R38A4OQ44K68Y7OS75Q77N | 28K33S38Q40Q44K68R70E75N | exon 21 |
| 28Q33Y38R40K44D68R70R75N | 30N33H38A44A68R70N75N | exon 21 |
| 30D33R38G44A68N70N75N | 30D33R38T44K68R70E75N | exon 22 |
| 28K33N38Q40Q44D68R70N75N | 30D33R38T44A68R70S75N | exon 23 |
| 30N33H38A44Q68R70S75N | 30Q33G38H44A68N70N75N | exon 24 |
| 28R33A38Y40Q44K68R70E75N | 28K33R38Q40S44E68R70A75N | exon 24 |
| 28R30D38Q44K68R70E75N | 28K33S38R40D70S75N | exon 25 |
| 30A33D38H44K68H70E75N | 28K33T38A40A44N68R70R75N | exon 25 |
| 28K30N38Q44K68A70S75N771 | 28K33R3BE40R44A6BS70R75N | exon 26 |
| 28Q33Y38Q40K44K68R70E75N | 28Q33Y38Q40K44Q68R70S75R77T80K | exon 27 |
| 30D33R38G44N68R70A75N | 28Q33Y38Q40K44Q68R70S75R77T80K | exon 27 |
| 30N33H38Q44R68R70R75N | 28R33A38Y40Q44A68R70S75N | exon 27 |
| 28K33S38Q40Q44R68Y70S75E771 | 30N33T38A44A68R70S75N | exon 27 |
| 28K30G38K44N68R70N75N | 28K33R38E40R44D68Y70S75S77R | exon 27 |
| 28K33R38A40Q44K68R70E75N | 28K30N38Q44Q68Y70S75R77Q | exon 27 |
| 28K33T38R40Q44K68R70G75N | 28R33A38Y40Q44E68R70A75N | exon 27 |
| 28K33N38040Q44Q68Y70S75R770 | 30D33R38T44Q68R70G75N | exon 27 |

**Table VI: Sequence of heterodimeric I-CreI variants having a DNA target site in or close to one exon of the XPF gene**

| **First monomer** | **Second monomer** | **Exon closest to the target sequence (SEQ ID NO: 167 to 188)** |
|---|---|---|
| 30N33H38Q44T68Y70S75R77V | 30D33R38T44Q68R70G75N | exon 1 |
| 28K33S38R40A44K68S70N75N | 28E33R38R40K44Q68R70G75N | exon 2 |
| 28K33T38A40A44Q68R70N75N | 30D33R38G44Q68R70S75N | exon 3 |
| 28Q33S38R40K44R68Y70S75E77Y | 28K33T38A40Q44T68Y70S75R77T | exon 3 |
| 28R30D38Q44Q68R70G75N | 28R33A38Y40Q44Q68R70S75R77T80K | exon 4 |
| 28K30N38Q44A68R70S75Y77Y | 28K33T38R40Q44A68R70S75Y77Y | exon 5 |
| 28K33T38R40Q44Q68Y70S75R77Q | 28K30N38Q44K68Y70S75Q77N | exon 5 |
| 28K33T38A40Q44Q68R70S75N | 28R30D38Q44A68N70S75Y77R | exon 6 |
| 28K33S38Q40Q44Q68R70S75N | 28K33N38Q40Q44A68R70S75Y77Y | exon 6 |
| 28K33R38A40Q44E68R70A75N | 28K33R38A40Q44Q68R70S75R77T80K | exon 6 |
| 28K33T38A40A42T44K70S75N77Y | 28K33T38A40A44T68Y70S75R77V | exon 7 |
| 30N33T38Q44K68R70E75N | 28T33R38Q40R44Q68R70N75N | exon 7 |
| 28Q33Y38Q40K44Q68R70G75N | 28R30D38Q44T68Y70S75R77V | exon 8 |
| 28K33S38Q40Q44Q68R70S75N | 28K30N38Q44K68R70G75N | exon 8 |
| 28K33T38A40Q44Q68R70G75N | 28Q33Y38R40K44Q68R70R75E77R | exon 8 |
| 28Q33Y38R40K44Q68R70S75R77T80K | 28A33T38Q40R44Q68R70S75R77T80K | exon 9 |
| 30D33R38T44A68R70G75N | 28K33R38E40R44Q68R70G75N | exon 9 |
| 28Q33S38R40K44R68Y70S75D77N | 30D33R38T44K68R70E75N | exon 10 |
| 28K30G38G44T68Y70S75R77V | 28R33A38Y40Q44K68A70S75N77I | exon 11 |
| 28Q33Y38R40K44D68Y70S75S77R | 30D33R38T44E68R70A75N | exon 11 |
| 30N33T38A44Q68R70G75N | 28K30G38H44R68Y70S75E77Y | exon 11 |
| 28K30G38H44A68N70N75N | 30N33H38A44R68Y70S75E77V | exon 11 |

**Table VII: Sequence of heterodimeric I-CreI variants having a DNA target site in or close to one exon of the XPG gene**

| **First monomer** | **Second monomer** | **Exon closest to the target sequence (SEQ ID NO: 189 to 216)** |
|---|---|---|
| 30D33R38T44K68R70E75N | 30D33R38T44Q68N70R75N | exon 1 |
| 30D33R38T44E68R70A75N | 28T33T38Q40R44Q68Y70S75R77Q | exon 2 |
| 30N33Y38Q44Q68R70S75R77T80K | 28K33T38A40A44K68R70E75N | exon 3 |
| 28T33R38S40R44A68R70S75N | 30N33H38Q44N68R70S75R77D | exon 4 |
| 28K30G38H44Q68R70Q75N | 28T33R38Q40R44A68R70S75N | exon 5 |
| 28K33T38R40Q44N68R70A75N | 28T33R38Q40R44K68R70E75N | exon 6 |
| 28K30G38H44A68Q70N75N | 32T33C44Y68D70S75R77T | exon 6 |
| 28R30D38Q44T68R70S75Y77T133V | 30N33T38A44Q68R70S75N | exon 7 |
| 28Q33Y38Q40K44A68R70N75N | 28K30N38Q44Q68R70G75N | exon 7 |
| 28K33R38E40R44Q68R70S75N | 28K33R38Q40A44K68Q70S75N77R | exon 8 |
| 30A33D38H44K68G70T75N | 28K33T38A40Q44N68R70S75R77D | exon 8 |
| 32T33C44N68R70A75N | 28R33A38Y40Q44K68R70E75N | exon 8 |
| 28K30G38H44A68R70D75N | 30N33H38A44Q68R70S75N | exon 8 |
| 30R33G38S44K68T70S75N | 28R33A38Y40Q42T44K70S75N77Y | exon 8 |
| 28R33S38Y40Q44K68T70S75N | 28R33A38Y40Q44N68R70S75R77D | exon 8 |
| 30N33H38Q44Q68R70D75N | 28Q33S38R40K44K68H70E75N | exon 9 |
| 28R33S38Y40Q44Y68E70S75R77V | 28K30N38Q44K68R70E75N | exon 9 |
| 32T33C44A68R70S75N | 28T33T38Q40R44T68Y70S75R77T | exon 9 |
| 30N33H38A44D68Y70S75S77R | 28K33R38E40R44T68Y70S75R77T | exon 10 |
| 28R33A38Y40Q44N68R70N75N | 28R33A38Y40Q44Q68Y70S75N77Y | exon 11 |
| 30N33Y38Q44Q68S70K75N | 30D33R38G44A68N70S75Y77R | exon 12 |
| 28K33T38A40Q44E68R70A75N | 28R33A38Y40Q44S68Y70S75Y77V | exon 12 |
| 28A33T38Q40R44E68R70A75N | 28K30G38G44A68N70N75N | exon 13 |
| 28K33R38A40Q44N68R70N75N | 28K33S38R40D42T44K70S75N77Y | exon 13 |
| 32T33C44Q68R70D75N | 28K33T38A40A44A68R70N75N | exon 14 |
| 32T33C44K68A70S75N | 30D33R38G44N68R70N75N | exon 14 |
| 28Q33Y38R40K44A68R70S75N | 30D33R38T42T44K70S75N77Y | exon 15 |
| 30D33R38T44K68G70T75N | 30D33R38G44A68D70K75N | exon 15 |

**Table VIII: Sequence of heterodimeric I-CreI variants having a DNA target site (SEQ ID NO: 24) situated in the third intron of the XPC gene**

| **First monomer** | **Second monomer** |
|---|---|
| 28K30N33S38R40S70S75N | 28E30N33Y38R40K44K68S70S75N |
| 28A30N33S38R40K70S75N | 28E30N33Y38R40K44K68R70E75N85R109T |
| 19A28A30N33S38R40K70S75N | 28E30N33Y38R40K44K68R70E75N85R109T161F |
| 19A28A30N33Y38R40K70S75N87L | 28E30N32R33Y38Q40K44K68R70E75N85R109T |
| 19A28A30N33S38R40K69G70S75N | 28S30N33Y38R40K44K68S70S75N |
| | 28S30N33Y38R40K44K68R70D75N |
| | 28S30N33Y38R40K44K68A70S75N |
| | 28K30G33Y38H40S44K68R70E75N |
| | 28K30G33Y38H40S44K68A70G75N |
| | 28K30G33Y38R40S44K68R70E75N |
| | 28K30G33Y38R40S44K68T70H75N |
| | 28K30G33Y38R40S44K68S70S75N |
| | 28K30G33Y38R40S44K68T70S75N |

In addition, the variants of the invention may include one or more residues inserted at the NH₂ terminus and/or COOH terminus of the sequence. For example, a tag (epitope or polyhistidine sequence) is introduced at the NH₂ terminus and/or COOH terminus; said tag is useful for the detection and/or the purification of said variant.

The subject-matter of the present invention is also a single-chain chimeric meganuclease derived from an I-*Cre*I variant as defined above. The single-chain chimeric meganuclease is a fusion protein comprising two I-*Cre*I monomers, two I-*Cre*I core domains (positions 6 to 94 of I-*Cre*I) or a combination of both. Preferably, the two monomers/core domains or the combination of both are connectd by a peptidic linker.

The subject-matter of the present invention is also a polynucleotide fragment encoding a variant or a single-chain chimeric meganuclease as defined above; said polynucleotide may encode one monomer of an heterodimeric variant, or two domains/monomers of a single-chain chimeric meganuclease.

The subject-matter of the present invention is also a recombinant vector for the expression of a variant or a single-chain meganuclease according to the invention. The recombinant vector comprises at least one polynucleotide fragment encoding a variant or a single-chain meganuclease, as defined above. In a preferred embodiment, said vector comprises two different polynucleotide fragments, each encoding one of the monomers of an heterodimeric variant.

A vector which can be used in the present invention includes, but is not limited to, a viral vector, a plasmid, a RNA vector or a linear or circular DNA or RNA molecule which may consists of a chromosomal, non chromosomal, semisynthetic or synthetic DNA. Preferred vectors are those capable of autonomous replication (episomal vector) and/or expression of nucleic acids to which they are linked (expression vectors). Large numbers of suitable vectors are known to those of skill in the art and commercially available.

Viral vectors include retrovirus, adenovirus, parvovirus (e. g. adeno-associated viruses), coronavirus, negative strand RNA viruses such as orthomyxovirus (e. g., influenza virus), rhabdovirus (e. g., rabies and vesicular stomatitis virus), para-myxovirus (e. g. measles and Sendai), positive strand RNA viruses such as picor-navirus and alphavirus, and double-stranded DNA viruses including adenovirus, herpesvirus (e. g., Herpes Simplex virus types 1 and 2, Epstein-Barr virus, cytomegalovirus), and poxvirus (e. g., vaccinia, fowlpox and canarypox). Other viruses include Norwalk virus, togavirus, flavivirus, reoviruses, papovavirus, hepadnavirus, and hepatitis virus, for example. Examples of retroviruses include: avian leukosis-sarcoma, mammalian C-type, B-type viruses, D type viruses, HTLV-BLV group, lentivirus, spumavirus (Coffin, J. M., Retroviridae: The viruses and their replication, In Fundamental Virology, Third Edition, B. N. Fields, et al., Eds., Lippincott-Raven Publishers, Philadelphia, 1996).

Vectors can comprise selectable markers, for example: neomycin phosphotransferase, histidinol dehydrogenase, dihydrofolate reductase, hygromycin phosphotransferase, herpes simplex virus thymidine kinase, adenosine deaminase, glutamine synthetase, and hypoxanthine-guanine phosphoribosyl transferase for eukaryotic cell culture ; TRP1 for *S. cerevisiae*; tetracycline, rifampicin or ampicillin resistance in *E. coli*.

Preferably said vectors are expression vectors, wherein the sequence(s) encoding the variant/single-chain meganuclease of the invention is placed under control of appropriate transcriptional and translational control elements to permit production or synthesis of said variant. Therefore, said polynucleotide is comprised in an expression cassette. More particularly, the vector comprises a replication origin, a promoter operatively linked to said encoding polynucleotide, a ribosome-binding site, an RNA-splicing site (when genomic DNA is used), a polyadenylation site and a transcription termination site. It also can comprise an enhancer. Selection of the promoter will depend upon the cell in which the polypeptide is expressed. Preferably, when said variant is an heterodimer, the two polynucleotides encoding each of the monomers are included in one vector which is able to drive the expression of both polynucleotides, simultaneously. Suitable promoters include tissue specific and/or inducible promoters. Examples of inducible promoters are: eukaryotic metallothionine promoter which is induced by increased levels of heavy metals, prokaryotic lacZ promoter which is induced in response to isopropyl-β-D-thiogalacto-pyranoside (IPTG) and eukaryotic heat shock promoter which is induced by increased temperature. Examples of tissue specific promoters are skeletal muscle creatine kinase, prostate-specific antigen (PSA), α-antitrypsin protease, human surfactant (SP) A and B proteins, β-casein and acidic whey protein genes.

According to another advantageous embodiment of said vector, it includes a targeting construct comprising a DNA sequence to be introduced in a human xeroderma pigmentosum gene flanked by at least 50 bp of DNA sequences having at least 95 % identity with the region of the human xeroderma pigmentosum gene flanking one of the target sequences SEQ ID NO: 1 to 24 and 45 to 216.

Homologous sequences of more than 100 bp and more preferably more than 200 bp may be used. Indeed, shared DNA homologies are located in regions flanking upstream and downstream the site of the break and the DNA sequence to be introduced should be located between the two arms. The sequence to be introduced is preferably a sequence which repairs a mutation in the xeroderma pigmentosum gene (gene correction or recovery of a functional gene), for the purpose of genome therapy. Alternatively, it can be any other sequence used to alter the chromosomal DNA in some specific way including a sequence used to modify a specific sequence, to attenuate or activate the endogenous gene of interest, to inactivate or delete the endogenous gene of interest or part thereof, to introduce a mutation into a site of interest or to introduce an exogenous gene or part thereof. Such chromosomal DNA alterations are used for genome engineering (animal models).

For correcting the *XP* gene, cleavage of the gene occurs in the vicinity of the mutation, preferably, within 500 bp of the mutation (figure 1B). The targeting construct comprises a *XP* gene fragment which has at least 200 bp of homologous sequence flanking the target site (minimal repair matrix) for repairing the cleavage, and includes the correct sequence of the *XP* gene for repairing the mutation (figure 1B). Consequently, the targeting construct for gene correction comprises or consists of the minimal repair matrix; it is preferably from 200 bp to 6000 bp, preferably from 1000 bp to 2000 bp.

For example, the target which is cleaved by each of the variant (Tables I to VIII) and the minimal matrix for repairing the cleavage with each variant are indicated in Tables IX to XV and in figures 16 to 22.

**Table IX: XPA gene targets cleaved by I-CreI variants**

| | | | | | **minimal repair matrix** | |
|---|---|---|---|---|---|---|
| **Exon closest to the target sequence** | **Exon position** | **Target SEQ ID NO:** | **target sequence** | **target position** | **start** | **end** |
| Exon 1 | 1-237 | 45 | ctgcctgcctcggtgcgggcga | 123 | 34 | 233 |
| Exon 1 | 1-237 | 46 | cagactggctcgtgcaagccga | 290 | 201 | 400 |
| Exon 2 | 3599-3709 | 47 | tttacttacttttgtaggcatg | 3582 | 3493 | 3692 |
| Exon 3 | 7719-7824 | 48 | taggacctgttatggaatttga | 7716 | 7627 | 7826 |
| Exon 4 | 10097-10262 | 49 | taatctgttttcagagatgctg | 10083 | 9994 | 10193 |
| Exon 4 | 10097-10262 | 50 | tgaaactctacttaaagttaca | 10240 | 10151 | 10350 |
| Exon 5 | 12318-12435 | 51 | taagctcaaacctcacagtacg | 12602 | 12513 | 12712 |
| Exon 5 | 12318-12435 | 52 | tacgacatttctgaaaagcatg | 12620 | 12531 | 12730 |
| Exon 6 | 21771-22448 | 53 | cagaattgcggcgagcagtaag | 21768 | 21679 | 21878 |
| Exon 6 | 21771-22448 | 54 | tgacctgttttatagaatttta | 21933 | 21844 | 22043 |
| Exon 6 | 21771-22448 | 55 | taatacttcagtaataattatg | 22044 | 21955 | 22154 |
| Exon 6 | 21771-22448 | 56 | caccactgtaccccaggttcta | 22317 | 22228 | 22427 |
| Exon 6 | 21771-22448 | 57 | tgtaccccaggttctagtcatg | 22323 | 22234 | 22433 |

**Table X : XPB gene targets cleaved by I-CreI variants**

| | | | | | **minimal repair matrix** | |
|---|---|---|---|---|---|---|
| **Exon closest to the target sequence** | **Exon Position** | **Target SEQ ID NO** | **target sequence** | **target position** | **start** | **end** |
| Exon 1 | 1-123 | 58 | cgggcctgtgggagcggggtca | 49 | -40 | 159 |
| Exon 2 | 459-664 | 59 | tggtatcttgcagacaagaaga | 446 | 357 | 556 |
| Exon 3 | 1331-1567 | 60 | ccaacccatgtgcatgagtaca | 1424 | 1335 | 1534 |
| Exon 3 | 1331-1567 | 61 | caacccatgtgcatgagtacaa | 1425 | 1336 | 1535 |
| Exon 3 | 1331-1567 | 62 | tggaattatgcagtttattaag | 1546 | 1457 | 1656 |
| Exon 4 | 3904-3953 | 63 | tgttctcagaaagcagaggcca | 3713 | 3624 | 3823 |
| Exon 5 | 4353-4488 | 64 | ttaaatcatggccggcagctca | 4197 | 4108 | 4307 |
| Exon 6 | 4676-4840 | 65 | tttgcctgctgattagatttca | 5104 | 5015 | 5214 |
| Exon 7 | 5313 5517 | 66 | ttggacctcttagaagatgtaa | 5237 | 5148 | 5347 |
| Exon 7 | 5313-5517 | 67 | tatgacttccggaatgattctg | 5373 | 5284 | 5483 |
| Exon 7 | 5313-5517 | 68 | ttccctgcggtaagtggtacca | 5509 | 5420 | 5619 |
| Exon 8 | 7160-7474 | 69 | ccataccaggtaagcaggctgg | 7466 | 7377 | 7576 |
| Exon 9 | 13546-13730 | 70 | cgaccctcgtccgcgaagatga | 13606 | 13517 | 13716 |
| Exon 9 | 13546-13730 | 71 | ttaaattttctgattgggccta | 13641 | 13552 | 13751 |
| Exon 9 | 13546-13730 | 72 | tgtgctgaggtagctgggcctg | 13722 | 13633 | 13832 |
| Exon 10 | 14802-15004 | 73 | tcttactgtattacaggtctgg | 14786 | 14697 | 14896 |
| Exon 10 | 14802-15004 | 74 | caaaaccaagaaacgaatcttg | 14849 | 14760 | 14959 |
| Exon 10 | 14802-15004 | 75 | tttgccctaaaggaatatgcca | 14970 | 14881 | 15080 |
| Exon 11 | 21216-21312 | 76 | cggacctacgtctcagggggaa | 21228 | 21139 | 21338 |
| Exon 11 | 21216-21312 | 77 | ccatcttcatatccaaggtttg | 21296 | 21207 | 21406 |
| Exon 12 | 22724-22841 | 78 | taaaccactttagttaggaaga | 22885 | 22796 | 22995 |
| Exon 13 | 32831-32849 | 79 | cttactggctatttttatattg | 32467 | 32378 | 32577 |
| Exon 13 | 32831-32849 | 80 | ctgcatgaatctctgagggcag | 33092 | 33003 | 33202 |
| Exon 14 | 34729-34881 | 81 | tggtctctggaatgctagggag | 34570 | 34481 | 34680 |
| Exon 14 | 34729-34881 | 82 | tagtcctgcctggatgggccca | 34958 | 34869 | 35068 |
| Exon 14 | 34729-34881 | 83 | tgggatttttttggaaatgttg | 34980 | 34891 | 35090 |
| Exon 15 | 36450-36887 | 84 | ccttccctccagcgttggccaa | 36673 | 36584 | 36783 |
| Exon 15 | 36450-36887 | 85 | ttggctgtgccttcataggtca | 36723 | 36634 | 36833 |
| Exon 15 | 36450-36887 | 86 | tgtgccttcataggtcatctag | 36728 | 36639 | 36838 |

**Table XI : XPC gene targets cleaved by I-CreI variants**

| | | | | | **minimal repair matrix** | |
|---|---|---|---|---|---|---|
| **Exon closest to the target sequence** | **Exon position** | **Target SEQ ID NO** | **target sequence** | **target position** | **start** | **end** |
| Exon 1 | 1-118 | 1 | ccagacgcggaggcctggtgga | 194 | 105 | 304 |
| Exon 2 | 5522-5717 | 2 | tcttacttgtcactggggtcca | 5793 | 5704 | 5903 |
| Exon 3 | 8034-8146 | 3 | caccatctgaagagaggggcta | 8062 | 7973 | 8172 |
| Exon 4 | 10204-10327 | 4 | ctgtctgggttgtgtgggttaa | 9976 | 9887 | 10086 |
| Exon 4 | 10204-10327 | 5 | tctgcctgtgaagccagtggag | 10262 | 10173 | 10372 |
| Exon 5 | 11331-11415 | 6 | tgagacatatcttcggagggcg | 11352 | 11263 | 11462 |
| Exon 6 | 12999-13156 | 7 | tcaaacctggtgaagtggtaag | 13140 | 13051 | 13250 |
| Exon 7 | 13651-13771 | 8 | cgggctggggaaagtaggacag | 13521 | 13432 | 13631 |
| Exon 8 | 18754-18843 | 9 | tttaattactttcttaggataa | 18708 | 18619 | 18818 |
| Exon 9 | 19692-20575 | 10 | caaaatttcttattctgtttaa | 19669 | 19580 | 19779 |
| Exon 9 | 19692-20575 | 11 | caagcccatgacctatgtggtg | 20392 | 20303 | 20502 |
| Exon 9 | 19692-20575 | 12 | cgagatgtcacacagaggtacg | 20438 | 20349 | 20548 |
| Exon 9 | 19692-20575 | 13 | tgacccgcaagtgccgggttga | 20478 | 20389 | 20588 |
| Exon 10 | 22091-22252 | 14 | ctgtctggcctttgcagtttca | 22074 | 21985 | 22184 |
| Exon 10 | 22091-22252 | 15 | tggcctttgcagtttcaggcta | 22079 | 21990 | 22189 |
| Exon 10 | 22091-22252 | 16 | tttgcccactgccattggctta | 22117 | 22028 | 22227 |
| Exon 10 | 22091-22252 | 17 | ccatctatcccgagacagctcg | 22191 | 22102 | 22301 |
| Exon 11 | 26171-26252 | 18 | tgtcccgcattcccgcagtggg | 26106 | 26017 | 26216 |
| Exon 12 | 29639-29773 | 19 | ctaaccgtgctcggaaagcccg | 29655 | 29566 | 29765 |
| Exon 13 | 29856-30025 | 20 | ttcccctgctggccaaatgctg | 29815 | 29726 | 29925 |
| Exon 14 | 30586-30679 | 21 | ctgtcttccacaaactggggag | 30505 | 30416 | 30615 |
| Exon 15 | 31208-31297 | 22 | tctgctcatcagggagaggctg | 31255 | 31166 | 31365 |
| Exon 16 | 32428-33437 | 23 | cccaccactgccacctgtccag | 32406 | 32317 | 32516 |

**Table XII : XPD gene targets cleaved by I-CreI variants**

| | | | | | **Minimal repair matrix** | |
|---|---|---|---|---|---|---|
| **Exon closest to the target sequence** | **Exon Position** | **Target SEQ ID NO** | **target sequence** | **target position** | **start** | **end** |
| Exon 1 | 1-36 | 87 | tcgaccccgctgcacagtccgg | 2 | -87 | 112 |
| Exon 2 | 340-439 | 88 | ctatatatcagctactgtgcca | 901 | 812 | 1011 |
| Exon 3 | 1425-1502 | 89 | tggtccccaacatgcagggtca | 1408 | 1319 | 1518 |
| Exon 3 | 1425-1502 | 90 | cccaacatgcagggtcatggag | 1413 | 1324 | 1523 |
| Exon 4 | 1580-1642 | 91 | ctgaacccgtaaaggcagacaa | 1515 | 1426 | 1625 |
| Exon 5 | 1829-1942 | 92 | cctgccccccaactttggagta | 1806 | 1717 | 1916 |
| Exon 5 | 1829-1942 | 93 | ccttctccttgcccttagccca | 1956 | 1867 | 2066 |
| Exon 6 | 5414-5530 | 94 | caggacaggtagcctggggcag | 5562 | 5473 | 5672 |
| Exon 7 | 5618-5734 | 95 | tgacctgaaggccctggggcgg | 5674 | 5585 | 5784 |
| Exon 7 | 5618-5734 | 96 | cgatactcagtgaggaggctgg | 5726 | 5637 | 5836 |
| Exon 8 | 6025-6148 | 97 | ctccccggcccccccagatcctg | 6009 | 5920 | 6119 |
| Exon 8 | 6025-6148 | 98 | cacaacattggtgaggggggcg | 6139 | 6050 | 6249 |
| Exon 9 | 6241-6337 | 99 | tgggaccctggcccctgtctga | 6379 | 6290 | 6489 |
| Exon 10 | 6453-6586 | 100 | cgggacgagtaccggcgtctgg | 6481 | 6392 | 6591 |
| Exon 10 | 6453-6586 | 101 | cgtgctgcccgacgaagtgctg | 6561 | 6472 | 6671 |
| Exon 11 | 6661-6829 | 102 | ctggctccatccgcacggccga | 6670 | 6581 | 6780 |
| Exon 12 | 8930-9048 | 103 | ctccccgccagattctgtgctg | 8919 | 8830 | 9029 |
| Exon 12 | 8930-9048 | 104 | cagcacctacgccaaaggtaag | 9032 | 8943 | 9142 |
| Exon 13 | 12873-12942 | 105 | tactcccaggcagtacggggtg | 12750 | 12661 | 12860 |
| Exon 14 | 13029-13098 | 106 | tgtcatcatcacatctggggta | 13080 | 12991 | 13190 |
| Exon 15 | 13201-13302 | 107 | tgagatccctcccactgtcccg | 13173 | 13084 | 13283 |
| Exon 16 | 14844-14907 | 108 | cagcctgggcaacatggtgaca | 14703 | 14614 | 14813 |
| Exon 16 | 14844-14907 | 109 | tggtatgctgccagtggggctg | 14906 | 14817 | 15016 |
| Exon 17 | 15721-15842 | 110 | cctgacaggcagcctcagtggg | 15617 | 15528 | 15727 |
| Exon 17 | 15721-15842 | 111 | cagcatgtaggaatggggtgta | 15967 | 15878 | 16077 |
| Exon 17 | 15721-15842 | 112 | caggctgagaatgcagatataa | 16025 | 15936 | 16135 |
| Exon 18 | 17238-17330 | 113 | caccacatctcagatgagccag | 17112 | 17023 | 17222 |
| Exon 19 | 17417-17489 | 114 | ccatcctgctgtcagtggcccg | 17439 | 17350 | 17549 |
| Exon 19 | 17417-17489 | 115 | tggccccggggcaaagtgtccga | 17454 | 17365 | 17564 |
| Exon 20 | 17756-17826 | 116 | ccaactcagacacagcatcctg | 17661 | 17572 | 17771 |
| Exon 22 | 18220-18363 | 117 | cccactccccaccctcagcctg | 18436 | 18347 | 18546 |
| Exon 22 | 18220-18363 | 118 | ctgtcctcctgccctcagcaaa | 18459 | 18370 | 18569 |
| Exon 23 | 18851-18984 | 119 | ccaaattcattcaaacatcctg | 18730 | 18641 | 18840 |

**Table XIII : XPE gene targets cleaved by I-CreI variants**

| | | | | | **minimal repair matrix** | |
|---|---|---|---|---|---|---|
| **Exon closest to the target sequence** | **Exon position** | **Target SEQ ID NO** | **target sequence** | **target position** | **start** | **end** |
| Exon 1 | 1-170 | 120 | caagcctcgacatgtcgtacaa | 99 | 10 | 209 |
| Exon 2 | 1387-1535 | 121 | caggacactttacttcggccga | 1384 | 1295 | 1494 |
| Exon 3 | 3004-3120 | 122 | taatattggtttccagggggag | 2988 | 2899 | 3098 |
| Exon 4 | 3494-3715 | 123 | tggccttttcaaggttattcca | 3577 | 3488 | 3687 |
| Exon 4 | 3494-3715 | 124 | ttgtctaccaggtactggatca | 3705 | 3616 | 3815 |
| Exon 5 | 6185-6299 | 125 | caggaccctcaggggcggcacg | 6182 | 6093 | 6292 |
| Exon 5 | 6185-6299 | 126 | ttccatggtgatcgcaggttgg | 6283 | 6194 | 6393 |
| Exon 6 | 7370-7467 | 127 | cccacccataggaatagtcgaa | 7123 | 7034 | 7233 |
| Exon 6 | 7370-7467 | 128 | tcttactctgtcgctcaggctcg | 7742 | 7653 | 7852 |
| Exon 7 | 8941-9099 | 129 | taaaatggctgggtttggtaaa | 8842 | 8753 | 8952 |
| Exon 8 | 9984-10067 | 130 | ctgaattatggctgcagttggg | 9870 | 9781 | 9980 |
| Exon 8 | 9984-10067 | 131 | ccagcttgtgaaggtgagaaga | 10055 | 9966 | 10165 |
| Exon 9 | 10666-10782 | 132 | ccatctcttttgggtgggtctg | 10600 | 10511 | 10710 |
| Exon 9 | 10666-10782 | 133 | tggcctggagaggcaggggca | 10754 | 10665 | 10864 |
| Exon 10 | 11381-11483 | 134 | ttaaacatatctgaaagtataa | 11623 | 11534 | 11733 |
| Exon 11 | 16511-16586 | 135 | tctgaccctaatcgtgagactg | 16528 | 16439 | 16638 |
| Exon 12 | 16685-16793 | 136 | tcttctgtggcaacgtggctca | 16756 | 16667 | 16866 |
| Exon 13 | 18592-18770 | 137 | ttccccaccagctgtaggtttg | 18357 | 18268 | 18467 |
| Exon 13 | 18592-18770 | 138 | tgccatattcttctttgttcag | 18846 | 18757 | 18956 |
| Exon 14 | 18868-19031 | 139 | tggcctctggacggacatctcg | 18952 | 18863 | 19062 |
| Exon 15 | 19109-19216 | 140 | ttacacagaattcttagtccca | 19268 | 19179 | 19378 |
| Exon 16 | 19372-19579 | 141 | tgtcattctctctgtaggtctg | 19355 | 19266 | 19465 |
| Exon 16 | 19372-19579 | 142 | tgagatgggaagtatagtgcag | 19685 | 19596 | 19795 |
| Exon 17 | 20994-21089 | 143 | ccagaccaatgaaataagagta | 20803 | 20714 | 20913 |
| Exon 17 | 20994-21089 | 144 | tggcaccatcgatgagatccag | 21027 | 20938 | 21137 |
| Exon 18 | 21183-21294 | 145 | tctgctaccaggaagtgtccca | 21188 | 21099 | 21298 |
| Exon 19 | 22660-22783 | 146 | caggctctgtccagcagtgtaa | 22657 | 22568 | 22767 |
| Exon 19 | 22660-22783 | 147 | ctccctaatccaggctgttctg | 22821 | 22732 | 22931 |
| Exon 20 | 23118-23282 | 148 | tggtctttcagtattcggatgg | 23262 | 23173 | 23372 |
| Exon 20 | 23118-23282 | 149 | cagtattcggatggtaagtggg | 23270 | 23181 | 23380 |
| Exon 21 | 24001-24095 | 150 | tgtactctatggtqqaatttaa | 24043 | 23954 | 24153 |
| Exon 21 | 24001-24095 | 151 | tagcacggtgagcctggacca | 24089 | 24000 | 24199 |
| Exon 22 | 29043-29213 | 152 | ccagcccaqtattaggggcaga | 29294 | 29205 | 29404 |
| Exon 23 | 29923-30032 | 153 | ccaaatgggctgcgttggcaga | 29740 | 29651 | 29850 |
| Exon 24 | 30327-30496 | 154 | tggtcttttccacctgggcgag | 30369 | 30280 | 30479 |
| Exon 24 | 30327-30496 | 155 | ttccacccccacacaaggctcg | 30444 | 30355 | 30554 |
| Exon 25 | 30719-30821 | 156 | caacctcctgctggacatgcag | 30750 | 30661 | 30860 |
| Exon 25 | 30719-30821 | 157 | tcgactcaataaagtcatcaaa | 30774 | 30685 | 30884 |
| Exon 26 | 32146-32269 | 158 | caagatgcaggaggtggtggca | 32239 | 32150 | 32349 |
| Exon 27 | 32859-33624 | 159 | ccatcttctcattgcagtatga | 32842 | 32753 | 32952 |
| Exon 27 | 32859-33624 | 160 | taggacgatggcagcggtatga | 32859 | 32770 | 32969 |
| Exon 27 | 32859-33624 | 161 | cgacctcatcaaggttgtgpag | 32900 | 32811 | 33010 |
| Exon 27 | 32859-33624 | 162 | ctaactcggatccattagccaa | 32925 | 32836 | 33035 |
| Exon 27 | 32859-33624 | 163 | tgggatccattagccaagggca | 32930 | 32841 | 33040 |
| Exon 27 | 32859-33624 | 164 | tgtcctctttttatttagattg | 33319 | 33230 | 33429 |
| Exon 27 | 32859-33624 | 165 | ctgactgccaagccatgggtag | 33458 | 33369 | 33568 |
| Exon 27 | 32859-33624 | 166 | ccaaataaagtagaatataaga | 33601 | 33512 | 33711 |

**Table XIV : XPF gene targets cleaved by I-CreI variants**

| | | | | | **minimal repair matrix** | |
|---|---|---|---|---|---|---|
| **Exon closest to the target sequence** | **Exon Position** | **Target SEQ ID NO** | **target sequence** | **target position** | **start** | **end** |
| Exon 1 | 1-207 | 167 | tgacacagagaaggatggcagg | 333 | 244 | 443 |
| Exon 2 | 1866-2046 | 168 | ctgccctgtattaaatagccta | 1820 | 1731 | 1930 |
| Exon 3 | 6396-6591 | 169 | tttgatactggtttttgtcatg | 6518 | 6429 | 6628 |
| Exon 3 | 6396-6591 | 170 | ctgtatctgtggccaaggtaaa | 6575 | 6486 | 6685 |
| Exon 4 | 7863-8070 | 171 | taacccatcgcttgaagtggaa | 8007 | 7918 | 8117 |
| Exon 5 | 10545-10725 | 172 | caagactaaatccttagttcag | 10589 | 10500 | 10699 |
| Exon 5 | 10545-10725 | 173 | ttgaataaagtgttaggtttta | 10765 | 10676 | 10875 |
| Exon 6 | 11992-12120 | 174 | tttaacttttcgtattaggttg | 11974 | 11885 | 12084 |
| Exon 6 | 11992-12120 | 175 | ttaacttttcgtattaggttgg | 11975 | 11886 | 12085 |
| Exon 6 | 11992-12120 | 176 | tgtaatgtatgttgaaagtata | 12265 | 12176 | 12375 |
| Exon 7 | 14027-14137 | 177 | tttgcttccaaaatctatcaaa | 14162 | 14073 | 14272 |
| Exon 7 | 14027-14137 | 178 | ttagctctttaaaagtagttca | 14199 | 14110 | 14309 |
| Exon 8 | 14981-15578 | 179 | tcatccatccgcttctgggttg | 15425 | 15336 | 15535 |
| Exon 8 | 14981-15578 | 180 | ctaacctttgttcggcagcttg | 15520 | 15431 | 15630 |
| Exon 8 | 14981-15578 | 181 | tttaatatccgttacgatgctg | 15663 | 15574 | 15773 |
| Exon 9 | 17601-17693 | 182 | tagtcctgctcaggaaggatag | 17762 | 17673 | 17872 |
| Exon 9 | 17601-17693 | 183 | cctgctcaggaaggatagggca | 17766 | 17677 | 17876 |
| Exon 10 | 24558-24670 | 184 | ttgtccctgaagaaagagaagg | 24575 | 24486 | 24685 |
| Exon 11 | 27449-28182 | 185 | cactccagaaatgtgcgtggag | 27585 | 27496 | 27695 |
| Exon 11 | 27449-28182 | 186 | cagcactggccattacagcaga | 27911 | 27822 | 28021 |
| Exon 11 | 27449-28182 | 187 | ctggccattacagcagattctg | 27916 | 27827 | 28026 |
| Exon 11 | 27449-28182 | 188 | cagaattagcagccctgtcaca | 28052 | 27963 | 28162 |

**Table XV : XPG gene targets cleaved by I-CreI variants**

| | | | | | **minimal repair matrix** | |
|---|---|---|---|---|---|---|
| **Exon closest to the target sequence** | **Exon Position** | **Target SEQ ID NO** | **target sequence** | **target position** | **start** | **end** |
| Exon 1 | 1-285 | 189 | cgggcctgtgggagcggggtca | 247 | 158 | 357 |
| Exon 2 | 6049-6224 | 190 | tggtatcttgcagacaagaaga | 6045 | 5956 | 6155 |
| Exon 3 | 7688-7803 | 191 | ccaacccatgtgcatgagtaca | 7780 | 7691 | 7890 |
| Exon 4 | 8219-8305 | 192 | caacccatgtgcatgagtacaa | 8133 | 8044 | 8243 |
| Exon 5 | 9983-10043 | 193 | tggaattatgcagtttattaag | 10066 | 9977 | 10176 |
| Exon 6 | 12206-12349 | 194 | tgttctcagaaagcagaggcca | 12290 | 12201 | 12400 |
| Exon 6 | 12206-12349 | 195 | ttaaatcatggccggcagctca | 12378 | 12289 | 12488 |
| Exon 7 | 15438-15645 | 196 | tttgcctgctgattagatttca | 15319 | 15230 | 15429 |
| Exon 7 | 15438-15645 | 197 | ttggacctcttagaagatgtaa | 15892 | 15803 | 16002 |
| Exon 8 | 15961-17034 | 198 | tatgacttccggaatgattctg | 16130 | 16041 | 16240 |
| Exon 8 | 15961-17034 | 199 | ttccctgcggtaagtggtacca | 16235 | 16146 | 16345 |
| Exon 8 | 15961-17034 | 200 | ccataccaggtaagcaggctgg | 16263 | 16174 | 16373 |
| Exon 8 | 15961-17034 | 201 | cgaccctcgtccgcgaagatga | 16483 | 16394 | 16593 |
| Exon 8 | 15961-17034 | 202 | ttaaattttctgattgggccta | 16622 | 16533 | 16732 |
| Exon 8 | 15961-17034 | 203 | tgtgctgaggtagctgggcctg | 16976 | 16887 | 17086 |
| Exon 9 | 19598-19842 | 204 | tcttactgtattacaggtctgg | 19576 | 19487 | 19686 |
| Exon 9 | 19598-19842 | 205 | caaaaccaagaaacgaatcttg | 19816 | 19727 | 19926 |
| Exon 9 | 19598-19842 | 206 | tttgccctaaaggaatatgcca | 19871 | 19782 | 19981 |
| Exon 10 | 20193-20312 | 207 | cggacctacgtctcagggggaa | 20296 | 20207 | 20406 |
| Exon 11 | 20563-20776 | 208 | ccatcttcatatccaaggtttg | 20589 | 20500 | 20699 |
| Exon 12 | 22044-22188 | 209 | taaaccactttagttaggaaga | 21979 | 21890 | 22089 |
| Exon 12 | 22044-22188 | 210 | cttactggctatttttatattg | 22206 | 22117 | 22316 |
| Exon 13 | 26129-26329 | 211 | ctgcatgaatctctgagggcag | 25965 | 25876 | 26075 |
| Exon 13 | 26129-26329 | 212 | tggtctctggaatgctagggag | 26539 | 26450 | 26649 |
| Exon 14 | 27190-27274 | 213 | tagtcctgcctggatgggccca | 26921 | 26832 | 27031 |
| Exon 14 | 27190-27274 | 214 | tgggatttttttggaaatgttg | 27347 | 27258 | 27457 |
| Exon 15 | 29238-29926 | 215 | ccttccctccagcgttggccaa | 29269 | 29180 | 29379 |
| Exon 15 | 29238-29926 | 216 | ttggctgtgccttcataggtca | 29545 | 29456 | 29655 |

For example, for correcting some of the mutations in the *XPC* gene found in Xeroderma pigmentosum (XP), as indicated in figure 1A, the following combinations of variants/targeting constructs may be used:
- ARG579TER (Exon 4; premature stop codon):
   * variant : 28Q,33S,38R,40K,44Q,68Y,70S;75N,77Y (first monomer)/ 28T,33T,38Q,40R,44T,68E,70S,75R,77R (second monomer), and a targeting construct comprising at least positions 9887 to 10086 of the *XPC* gene, for efficient repair of the DNA double-strand break, and all sequences between the meganuclease cleavage site and the mutation site, for efficient repair of the mutation.
   * variant 30D,33R,38T,44N,68R,70S,75Q,77R (first monomer)/ 28R,33A,38Y,40Q,44D,68Y,70S,75S,77R (second monomer) and a targeting construct comprising at least positions 10173 to 10372 of the *XPC* gene, for efficient repair of the DNA double-strand break, and all sequences between the meganuclease cleavage site and the mutation site, for efficient repair of the mutation.
- Exon 6: substitution PR0218HIS:
   * variant: 28K,33N,38Q,40Q,44R,68Y,70S,75E,77I (first monomer)/ 28K,33T,38A,40Q,44K,68Q,70S,75N,77R (second monomer), and a targeting construct comprising at least positions 13051 to 13250 of the *XPC* gene, for efficient repair of the DNA double-strand break, and all sequences between the meganuclease cleavage site and the mutation site, for efficient repair of the mutation.
- Exon 9: deletion DEL1132AA or insertion insVAL580:
   * variant : 28K,30N,38Q,44Q,68R,70S,75R,77T,80K (first monomer)/ 28T,33T,38Q,40R,44T,68Y,70S,75R,77V (second monomer) and a targeting construct comprising at least positions 19580 to 19779 of the *XPC* gene, for efficient repair of the DNA double-strand break, and all sequences between the meganuclease cleavage site and the mutation site, for efficient repair of the mutation.
   * variant : 28K,30N,38Q,44A,68Y,70S,75Y,77K (first monomer)/ 28K,33R,38E,40R,44T,68R,70S,75Y,77T,133V (second monomer) and a targeting construct comprising at least positions 20303 to 20502 of the *XPC* gene, for efficient repair of the DNA double-strand break, and all sequences between the meganuclease cleavage site and the mutation site, for efficient repair of the mutation.
   * variants : 28K,33R,38Q,40A,44Q,68R,70N,75N (first monomer)/ 28K,33R,38A,40Q,44Q,68R,70S,75N,77K (second monomer) or 33H,75N (first monomer) and 33R,38A,40Q,44K,70N,75N (second monomer), and a targeting construct comprising at least positions 20349 to 20548 of the *XPC* gene, for efficient repair of the DNA double-strand break, and all sequences between the meganuclease cleavage site and the mutation site, for efficient repair of the mutation.
   * variant : 30N,33H,38Q,44K,68A,70S,75N,77I (first monomer)/ 28K,33N,38Q,40Q,44R,68Y,70S,75E,77V (second monomer), and a targeting construct comprising at least positions 20389 to 20588 of the *XPC* gene, for efficient repair of the DNA double-strand break, and all sequences between the meganuclease cleavage site and the mutation site, for efficient repair of the mutation.
- Exon 14: substitution LYS822GLN:
   * variant : 28Q,33S,38R,40K, 42T,44K,70S,75N,77Y (first monomer)/ 28R,33A,38Y,40Q,44A,68R,70S,75E,77R (second monomer), and a targeting construct comprising at least positions 30416 to 30615 of the *XPC* gene, for efficient repair of the DNA double-strand break, and all sequences between the meganuclease cleavage site and the mutation site, for efficient repair of the mutation.

Alternatively, for restoring a functional gene (figure 1C), cleavage of the gene occurs upstream of a mutation, for example at position 9119 (target SEQ ID NO: 24). Preferably said mutation is the first known mutation in the sequence of the gene, so that all the downstream mutations of the gene can be corrected simultaneously. The targeting construct comprises the exons downstream of the cleavage site fused in frame (as in the cDNA) and with a polyadenylation site to stop transcription in 3'. The sequence to be introduced (exon knock-in construct) is flanked by introns or exons sequences surrounding the cleavage site, so as to allow the transcription of the engineered gene (exon knock-in gene) into a mRNA able to code for a functional protein (figure 1C). For example, when cleavage occurs in an exon, the exon knock-in construct is flanked by sequences upstream and downstream of the cleavage site, from a minimal repair matrix as defined above.

The subject-matter of the present invention is also a composition characterized in that it comprises at least one variant, one single-chain chimeric endonuclease and/or at least one expression vector encoding said variant/single-chain molecule, as defined above.

In a preferred embodiment of said composition, it comprises a targeting DNA construct comprising a sequence which repairs a mutation in the *XP* gene, flanked by sequences sharing homologies with the region of said gene surrounding the genomic DNA cleavage site of said variant, as defined above. The sequence which repairs the mutation is either fragment of the gene with the correct sequence or an exon knock-in construct, as defined above.

Preferably, said targeting DNA construct is either included in a recombinant vector or it is included in an expression vector comprising the polynucleotide(s) encoding the variant/single-chain chimeric endonuclease according to the invention.

In the case where two vectors may be used, the subject-matter of the present invention is also products containing an I-*Cre*I variant or single-chain chimeric meganuclease expression vector as defined above and a vector which includes a targeting construct as defined above, as a combined preparation for simultaneous, separate or sequential use in Xeroderma pigmentosum.

The subject-matter of the present invention is also the use of at least one meganuclease variant/single-chain chimeric meganuclease and/or one expression vector, as defined above, for the preparation of a medicament for preventing, improving or curing Xeroderma pigmentosum in an individual in need thereof, said medicament being administrated by any means to said individual.

In this case, the use of the meganuclease (variant/single-chain derivative) comprises at least the step of (a) inducing in somatic tissue(s) of the individual a double stranded cleavage at a site of interest comprising at least one recognition and cleavage site of said meganuclease by contacting said cleavage site with said meganuclease, and (b) introducing into the individual a targeting DNA, wherein said targeting DNA comprises (1) DNA sharing homologies to the region surrounding the cleavage site and (2) DNA which repairs the site of interest upon recombination between the targeting DNA and the chromosomal DNA. The targeting DNA is introduced into the individual under conditions appropriate for introduction of the targeting DNA into the site of interest.

According to the present invention, said double-stranded cleavage is induced, either *in toto* by administration of said meganuclease to an individual, or *ex vivo* by introduction of said meganuclease into somatic cells (skin cells) removed from an individual and returned into the individual after modification.

The meganuclease (variant/single-chain derivative) can be used either as a polypeptide or as a polynucleotide construct encoding said polypeptide. It is introduced into somatic cells of an individual, by any convenient mean well-known to those in the art, which is appropriate for the particular cell type, alone or in association with either at least an appropriate vehicle or carrier and/or with the targeting DNA.

The meganuclease (polypeptide) may be associated with:
- liposomes, polyethyleneimine (PEI); in such a case said association is administered and therefore introduced into somatic target cells.
- membrane translocating peptides (Bonetta, 2002, The Scientist, 16, 38; Ford et al, Gene Ther, 2001, 8, 1-4 ; Wadia & Dowdy, 2002, Curr Opin Biotechnol, 13, 52-56); in such a case, the sequence of the variant/single-chain derivative is fused with the sequence of a membrane translocating peptide (fusion protein).

The meganuclease (polynucleotide encoding said meganuclease) and/or the targeting DNA may be inserted in a vector. Vectors comprising targeting DNA and/or nucleic acid encoding a meganuclease can be introduced into a cell by a variety of methods (e.g., injection, direct uptake, projectile bombardment, liposomes). Meganucleases can be stably or transiently expressed into cells using expression vectors. Techniques of expression in eukaryotic cells are well known to those in the art. (See Current Protocols in Human Genetics: Chapter 12 "Vectors For Gene Therapy" & Chapter 13 "Delivery Systems for Gene Therapy"). Optionally, it may be preferable to incorporate a nuclear localization signal into the recombinant protein to be sure that it is expressed within the nucleus.

Once in a cell, the meganuclease and if present, the vector comprising targeting DNA and/or nucleic acid encoding a meganuclease are imported or translocated by the cell from the cytoplasm to the site of action in the nucleus.

For purposes of therapy, the meganucleases and a pharmaceutically acceptable excipient are administered in a therapeutically effective amount. Such a combination is said to be administered in a "therapeutically effective amount" if the amount administered is physiologically significant. An agent is physiologically significant if its presence results in a detectable change in the physiology of the recipient. In the present context, an agent is physiologically significant if its presence results in a decrease in the severity of one or more symptoms of the targeted disease and in a genome correction of the lesion or abnormality. The meganuclease may be substantially non-immunogenic, i.e., engender little or no adverse immunological response. A variety of methods for ameliorating or eliminating deleterious immunological reactions of this sort can be used in accordance with the invention. The meganuclease may be substantially free of N-formyl methionine. Another way to avoid unwanted immunological reactions is to conjugate meganucleases to polyethylene glycol ("PEG") or polypropylene glycol ("PPG") (preferably of 500 to 20,000 daltons average molecular weight (MW)). Conjugation with PEG or PPG, as described by Davis et al., (US 4,179,337) for example, can provide non-immunogenic, physiologically active, water soluble endonuclease conjugates with anti-viral activity. Similar methods also using a polyethylene--polypropylene glycol copolymer are described in Saifer et al. (US 5,006,333). The invention also concerns a prokaryotic or eukaryotic host cell comprising a polynucleotide or a vector as defined above, preferably an expression vector.

The invention also concerns a non-human transgenic animal or a transgenic plant, comprising one or two polynucleotide fragments as defined above. As used herein, a cell refers to a prokaryotic cell, such as a bacterial cell, or an eukaryotic cell, such as an animal, plant or yeast cell.

The subject-matter of the present invention is further the use of a meganuclease (variant or single-chain derivative) or an expression vector as defined above for genome engineering (animal models generation: knock-in or knock-out), for non-therapeutic purposes.

Said use may be for inducing a double-strand break in the gene of interest, thereby inducing a DNA recombination event, a DNA loss or cell death. Said double-strand break may be for: repairing a specific sequence, modifying a specific sequence, restoring a functional gene in place of a mutated one, attenuating or activating an endogenous gene of interest, introducing a mutation into a site of interest, introducing an exogenous gene or a part thereof, inactivating or deleting an endogenous gene or a part thereof, translocating a chromosomal arm, or leaving the DNA unrepaired and degraded.

According to another advantageous embodiment of said use, said variant, vector is associated with a targeting DNA construct as defined above.

The use of the meganuclease (variandsingle-chain derivative) according to the present invention may comprise at least the following steps: 1) introducing a double-strand break at the genomic locus comprising at least one recognition and cleavage site of said meganuclease by contacting said cleavage site with said meganuclease; 2) providing a targeting DNA construct comprising the sequence to be introduced flanked by sequences sharing homologies to the targeted locus. Said meganuclease variant can be provided directly to the cell or through an expression vector comprising the polynucleotide sequence encoding said meganuclease and suitable for its expression in the used cell. This strategy is used to introduce a DNA sequence at the target site, for example to generate knock-in or knock-out animal models or cell lines that can be used for drug testing.

The present application discloses also the use of at least one meganuclease variant, as defined above, as a scaffold for making other meganucleases. For example a third round of mutagenesis and selection/screening can be performed on said variants, for the purpose of making novel, third generation homing endonucleases.

The different uses of the I-CreI variant and the methods of using said I-*Cre*I variant according to the present invention include also the use of the single-chain chimeric meganuclease derived from said variant, the polynucleotide(s), vector, cell, transgenic plant or non-human transgenic mammal encoding said variant or single-chain chimeric endonuclease, as defined above.

The I-*Cre*I variant according to the invention may be obtained by a method for engineering I-*Cre*I variants able to cleave a genomic DNA target sequence selected from the group consisting of the sequences SEQ ID NO: 1 to 24 and 45 to 216 from a human XP gene, comprising at least the steps of:
(a) constructing a first series of I-*Cre*I variants having at least one substitution in positions 28, 30, 32, 33, 38 and/or 40 of a first functional subdomain of the LAGLIDADG core domain situated from positions 28 to 40 of I-*Cre*I, ,
(b) constructing a second series of I-*Cre*I variants having at least one substitution in positions 44, 68 and/or 70 of a a second functional subdomain of the LAGLIDADG core domain situated from positions 44 to 70 of *I-CreI,*
(c) selecting the variants from the first series of step (a) which are able to cleave a mutant I-*Cre*I site wherein (i) the nucleotide triplet in positions -10 to -8 of the I-*Cre*I site has been replaced with the nucleotide triplet which is present in positions -10 to -8 of said genomic target and (ii) the nucleotide triplet in positions +8 to +10 has been replaced with the reverse complementary sequence of the nucleotide triplet which is present in positions -10 to -8 of said genomic target
(d) selecting the variants from the second series of step (b) which are able to cleave a mutant I-*Cre*I site wherein (i) the nucleotide triplet in positions -5 to -3 of the I-*Cre*I site has been replaced with the nucleotide triplet which is present in positions -5 to -3 of said genomic target and (ii) the nucleotide triplet in positions +3 to +5 has been replaced with the reverse complementary sequence of the nucleotide triplet which is present in positions -5 to -3 of said genomic target,
(e) selecting the variants from the first series of step (a) which are able to cleave a mutant I-*Cre*I site wherein (i) the nucleotide triplet in positions +8 to +10 of the I-*Cre*I site has been replaced with the nucleotide triplet which is present in positions +8 to +10 of said genomic target and (ii) the nucleotide triplet in positions -10 to -8 has been replaced with the reverse complementary sequence of the nucleotide triplet which is present in positions +8 to +10 of said genomic target
(f) selecting the variants from the second series of step (b) which are able to cleave a mutant I-*Cre*I site wherein (i) the nucleotide triplet in positions +3 to +5 of the I-*Cre*I site has been replaced with the nucleotide triplet which is present in positions +3 to +5 of said genomic target and (ii) the nucleotide triplet in positions -5 to -3 has been replaced with the reverse complementary sequence of the nucleotide triplet which is present in positions +3 to +5 of said genomic target
(g) combining in a single variant, the mutation(s) in positions 28 to 40 and 44 to 70 of two variants from step (c) and step (d), to obtain a novel homodimeric I-*Cre*I variant which cleaves a sequence wherein (i) the nucleotide triplet in positions -10 to -8 is identical to the nucleotide triplet which is present in positions -10 to -8 of said genomic target, (ii) the nucleotide triplet in positions +8 to +10 is identical to the reverse complementary sequence of the nucleotide triplet which is present in positions -10 to -8 of said genomic target, (iii) the nucleotide triplet in positions -5 to -3 is identical to the nucleotide triplet which is present in positions -5 to -3 of said genomic target and (iv) the nucleotide triplet in positions +3 to +5 is identical to the reverse complementary sequence of the nucleotide triplet which is present in positions -5 to -3 of said genomic target
(h) combining in a single variant, the mutation(s) in positions 28 to 40 and 44 to 70 of two variants from step (e) and step (f), to obtain a novel homodimeric I-*Cre*I variant which cleaves a sequence wherein (i) the nucleotide triplet in positions +3 to +5 is identical to the nucleotide triplet which is present in positions +3 to +5 of said genomic target, (ii) the nucleotide triplet in positions -5 to - 3 is identical to the reverse complementary sequence of the nucleotide triplet which is present in positions +3 to +5 of said genomic target, (iii) the nucleotide triplet in positions +8 to +10 of the *I-CreI* site has been replaced with the nucleotide triplet which is present in positions +8 to +10 of said genomic target and (iv) the nucleotide triplet in positions -10 to -8 is identical to the reverse complementary sequence of the nucleotide triplet in positions +8 to +10 of said genomic target.
(i) combining the variants obtained in steps (g) and (h) to form heterodimers.
(j) selecting the heterodimers from step (i) which are able to cleave said genomic DNA target from a human XP gene.

Steps (a), (b), (g), (h) and (i) may further comprise the introduction of additional mutations in order to improve the binding and/or cleavage properties of the mutants. Additional mutations may be introduced at other positions contacting the DNA target sequence or interacting directly or indirectly with said DNA target. This additional step may be performed by generating a library of variants as described in the International PCT Application WO 2004/067736.

The method for engineering I-*Cre*I variants of the invention advantageously comprise the introduction of random mutations on the whole variant or in a part of the variant, in particular the C-terminal half of the variant (positions 80 to 163) to improve the binding and/or cleavage properties of the mutants towards the DNA target from the gene of interest. The mutagenesis may be performed by generating random mutagenesis libraries on a pool of variants, according to standard mutagenesis methods which are well-known in the art and commercially available. Preferably, the mutagenesis is performed on the entire sequence of one monomer of the heterodimer formed in step (i) or obtained in step (j), advantageously on a pool of monomers, preferably on both monomers of the heterodimer of step (i) or (j).

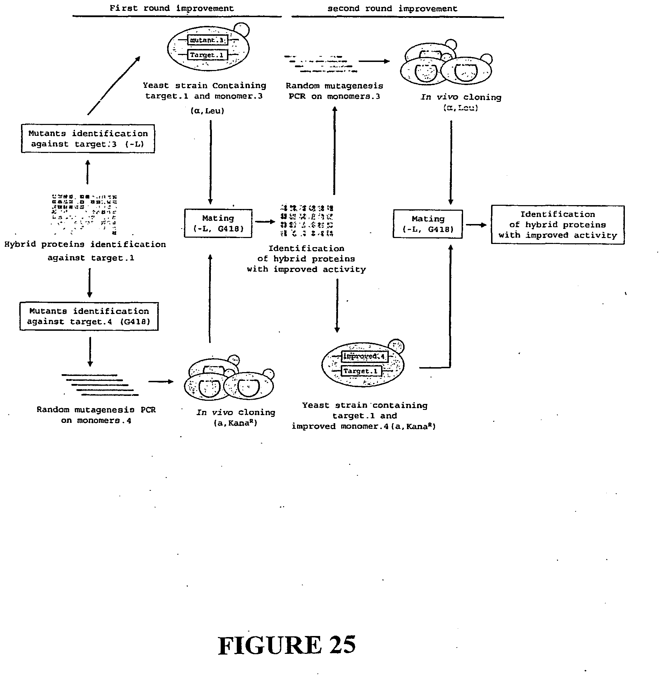

Preferably, two rounds of selection/screening are performed according to the process illustrated by figure 25. In the first round, one of the monomers of the heterodimer is mutagenised (monomer.4 in figure 25), co-expressed with the other monomer (monomer.3 in figure 25) to form heterodimers, and the improved monomers.4 are selected against said DNA target from a human XP gene. In the second round, the other monomer (monomer.3) is mutagenised, co-expressed with the improved monomers.4 to form heterodimers, and selected against said DNA target to obtain meganucleases havingimproved activity towards said DNA target from a XP gene in comparison to said initial heterodimer formed in step (i) or obtained in step (j).

The combination of mutations in steps (g) and (h) may be performed by amplifying overlapping fragments comprising each of the two subdomains, according to well-known overlapping PCR techniques.

The combination of the variants in step (i) is performed by coexpressing one variant from step (g) with one variant from step (h), so as to allow the formation of heterodimers. For example, host cells may be modified by one or two recombinant expression vector(s) encoding said variant(s). The cells are then cultured under conditions allowing the expression of the variant(s), so that heterodimers are formed in the host cells.

The selection in steps (c), (d), (e), (f) and/or (j) may be performed by using a cleavage assay *in vitro* or *in vivo*, as described in the International PCT Application WO 2004/067736 or in Arnould et al., J. Mol. Biol., 2006, 355(3): 443-58.

Steps (c), (d), (e), (f) and/or (j) may be performed *in vivo*, under conditions where the double-strand break in the mutated DNA target sequence which is generated by said variant leads to the activation of a positive selection marker or a reporter gene, or the inactivation of a negative selection marker or a reporter gene, by recombination-mediated repair of said DNA double-strand break.

The polynucleotide sequence(s) encoding the variant as defined in the present invention may be prepared by any method known by the man skilled in the art. For example, they are amplified from a cDNA template, by polymerase chain reaction with specific primers. Preferably the codons of said cDNA are chosen to favour the expression of said protein in the desired expression system.

The recombinant vector comprising said polynucleotides may be obtained and introduced in a host cell by the well-known recombinant DNA and genetic engineering techniques.

The variant of the invention is produced by expressing the polypeptide(s) as defined above; preferably said polypeptide(s) are expressed or co-expressed in a host cell modified by one or two expression vector(s), under conditions suitable for the expression or co-expression of the polypeptides, and the variant is recovered from the host cell culture.

Single-chain chimeric meganucleases able to cleave a DNA target from the gene of interest are derived from the variants according to the invention by methods well-known in the art (Epinat et al., Nucleic Acids Res., 2003, 31, 2952-62; Chevalier et al., Mol. Cell., 2002, 10, 895-905; Steuer et al., Chembiochem., 2004, 5, 206-13; International PCT Applications WO 03/078619 and WO 2004/031346). Any of such methods, may be applied for constructing single-chain chimeric meganucleases derived from the variants as defined in the present invention.

In addition to the preceding features, the invention further comprises other features which will emerge from the description which follows, which refers to examples illustrating the I-*Cre*I meganuclease variants and their uses according to the invention, as well as to the appended drawings in which:
- figure 1 represents the human *XPC* gene, and two different strategies for restoring a functional gene by meganuclease-induced recombination. **A.** The *XPC* gene CDS junctions are indicated; the mutations found in the XP-C complementation group are featured by an arrow. The Xa.1 sequence (position 9119, SEQ ID NO: 24) is found in an intronic sequence. The Xc.1 sequence (position 20438, SEQ ID NO: 12) is found in Exon 9. **B.** Gene correction. A mutation occurs within a known gene. Upon cleavage by a meganuclease and recombination with a repair matrix the deleterious mutation is corrected. **C.** Exonic sequences knock-in. A mutation occurs within a known gene. The mutated mRNA transcript is featured below the gene. In the repair matrix, exons located downstream of the cleavage site are fused in frame (as in a cDNA), with a polyadenylation site to stop transcription in 3'. Introns and exons sequences can be used as homologous regions. Exonic sequences knock-in results into an engineered gene, transcribed into a mRNA able to code for a functional protein.
- figure 2 illustrates the principle of the invention. **A:** Structure of I-*Cre*I bound to its target. Experimental data have shown that two independent subdomains (squares) could be identified in the DNA binding domain; each subdomain of the core domain binds a different half of the DNA target. **B.** One would like to identify smaller independent subdomains (squares), each binding a distinct part of a half DNA target. However, there is no structural or experimental data in favour of this hypothesis.
- figure 3 represents the map of the base specific interactions of I-*Cre*I with its DNA target C1221 (SEQ ID NO: 25) (Chevalier and Stoddard, Nucleic Acids Res., 2001, 29, 3757-74 ; Chevalier et al. J. Mol. Biol., 2003, 329, 253-69). The inventors have identified novel I-*Cre*I derived endonucleases able to bind DNA targets modified in regions -10 to -8 and +8 to +10, or -5 to -3 and +3 to +5. These DNA regions are indicated in grey boxes.
- figure 4 illustrates the rationale of the combinatorial approach. **A.** Given the separability of the two DNA binding subdomain (top left), one can combine different I-*Cre*I monomers binding different sequences derived from the *I-CreI* target sequence (top right and bottom left) to obtain heterodimers or single chain fusion molecules cleaving non-palindromic chimeric targets (bottom right). **B.** The identification of smaller independent subunit, *i.e.*, subunit within a single monomer or αββαββα fold (top right and bottom left) would allow for the design of novel chimeric molecules (bottom right), by combination of mutations within the same monomer. Such molecules would cleave palindromic chimeric targets (bottom right). C. The combination of the two former steps would allow a larger combinatorial approach, involving four different subdomains (top right, middle left and right, bottom left) that could be combined in new molecules (bottom right). Thus, the identification of a small number of new cleavers for each subdomain would allow for the design of a very large number of novel endonucleases.

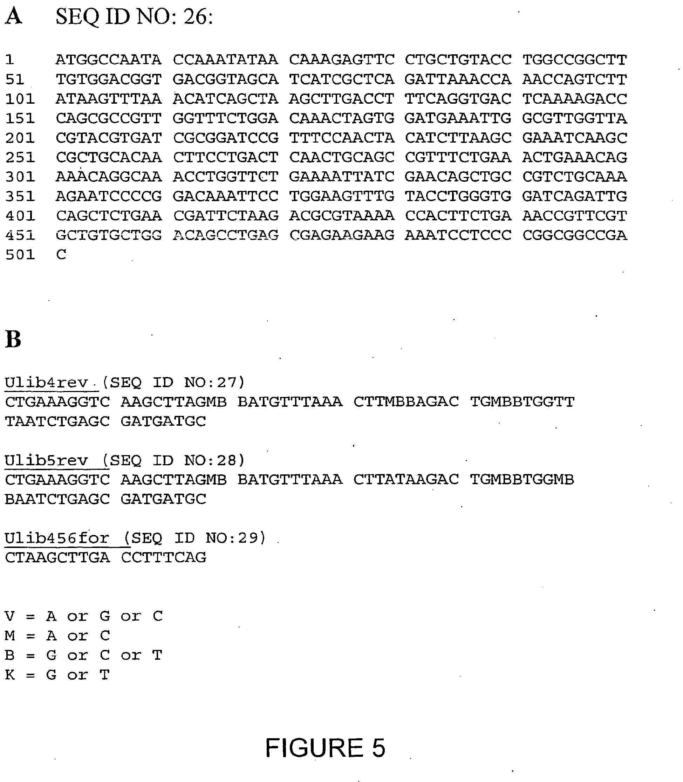

- figure 5 represents the sequences of the cDNA encoding I-*Cre*I N75 scaffold protein and degenerated primers used for the Ulib4 and Ulib5 libraries construction. A. The scaffold (SEQ ID NO: 26) encodes an I-*Cre*I ORF (SEQ ID NO: 218) which differs from the I-*Cre*I sequence SWISSPROT P05725 by the insertion of an alanine codon in position 2, the A42T, D75N, W110E and R111Q codons substitutions and three additional codons (AAD) at the 3' end. B. Primers (SEQ ID NO: 27, 28, 29),

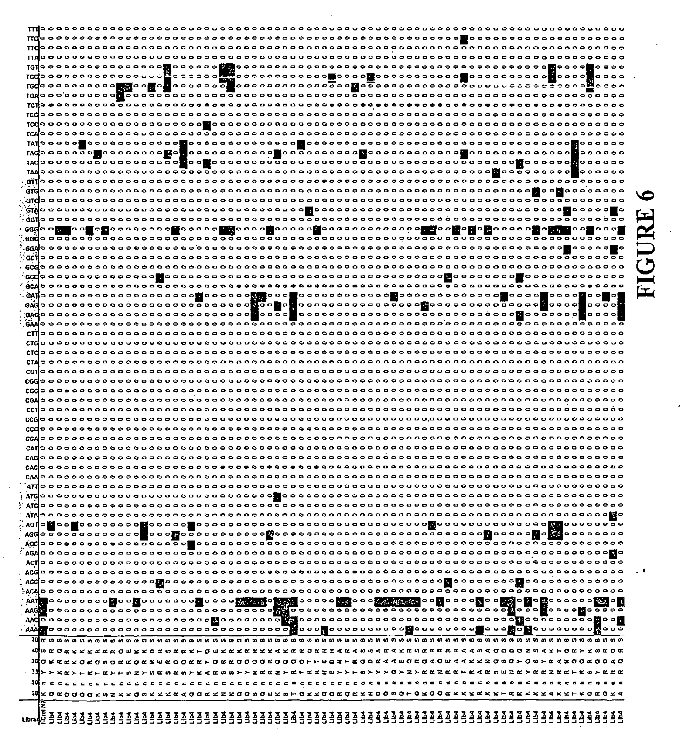

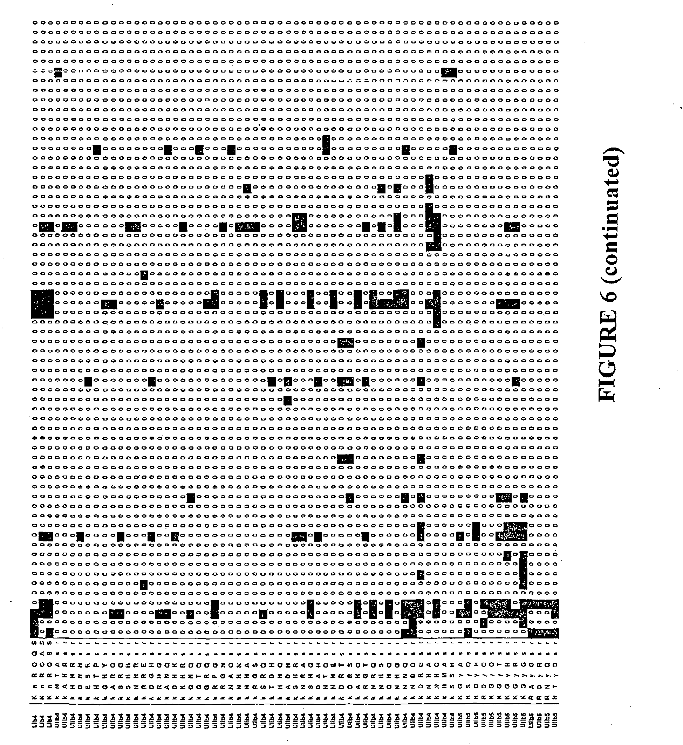

- figure 6 represents the cleavage patterns of the I-*Cre*I variants in positions 28, 30, 33, 38 and/or 40. For each of the 141 I-*Cre*I variants obtained after screening, and defined by residues in position 28, 30, 33, 38, 40, 70 and 75, cleavage was monitored in yeast with the 64 targets derived from the C 1221 palindromic target cleaved by I-*Cre*I, by substitution of the nucleotides in positions ± 8 to 10.Targets are designated by three letters, corresponding to the nucleotides in position -10, -9 and -8. For example GGG corresponds to the tcgggacgtcgtacgacgtcccga target (SEQ ID NO: 30). Values correspond to the intensity of the cleavage, evaluated by an appropriate software after scanning of the filter. For each protein, observed cleavage (black box) or non observed cleavage (0) is shown for each one of the 64 targets. All the variants are mutated in position 75: D75N.

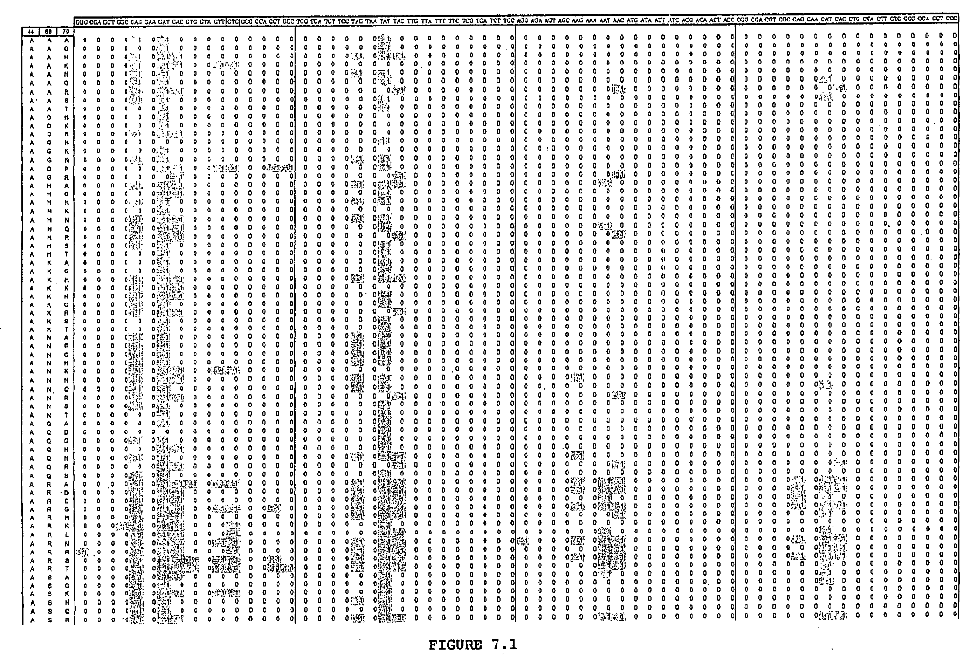

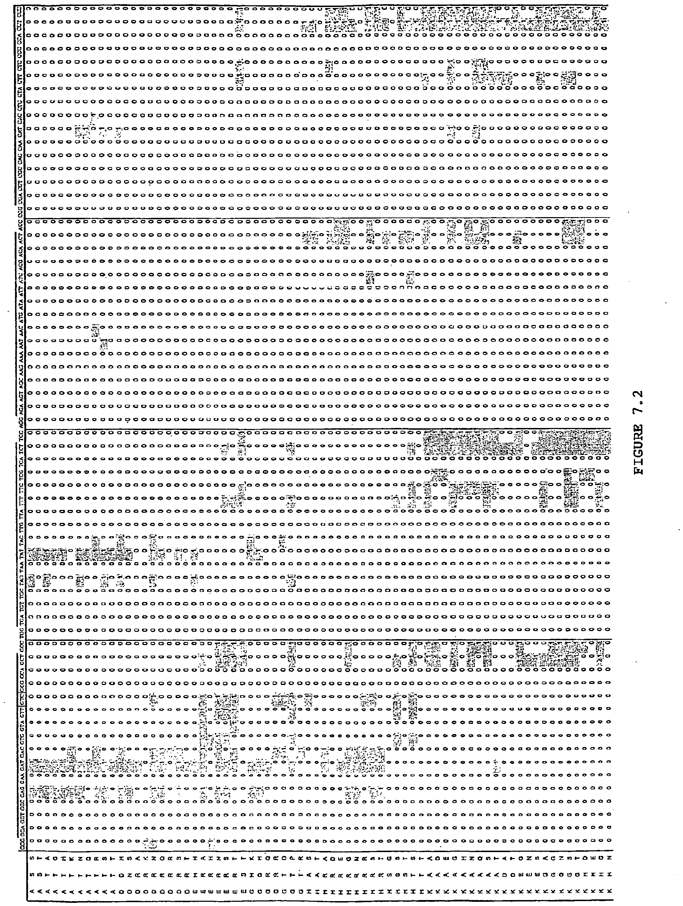

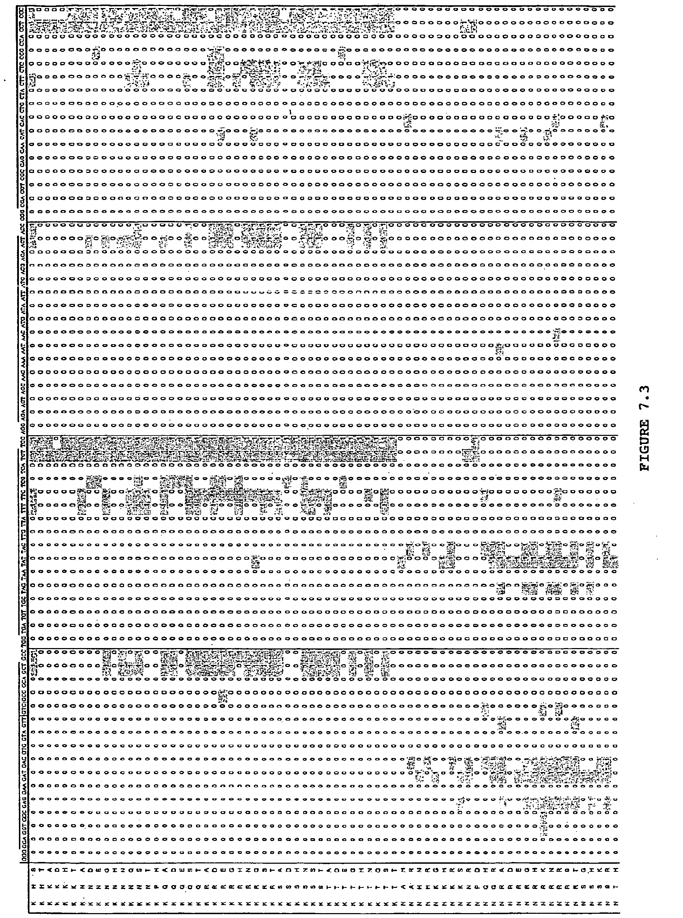

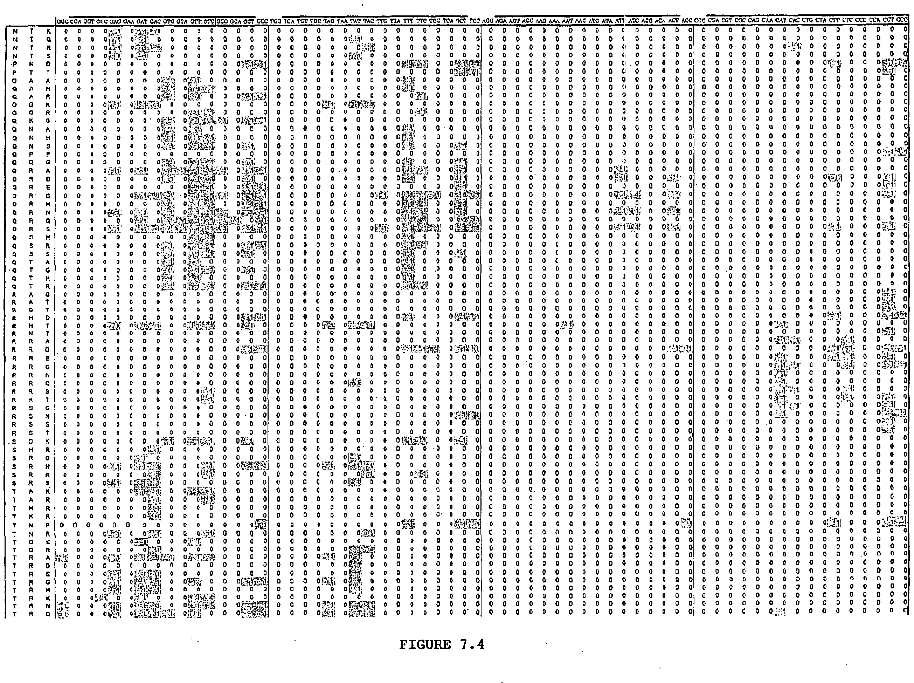

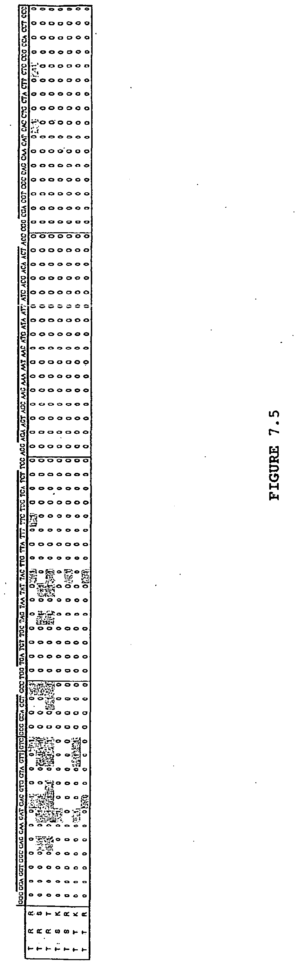

- figure 7 represents the cleavage patterns of the I-*Cre*I variants in position 44, 68 and/or 70. For each of the 292 I-*Cre*I variants obtained after screening, and defined by residues in position 44, 68 and 70 (first three columns) cleavage was monitored in yeast with the 64 targets derived from the C1221 palindromic target cleaved by I-*Cre*I, by substitution of the nucleotides in positions ± 3 to 5. Targets are designated by three letters, corresponding to the nucleotides in position -5, -4 and -3. For each protein, observed cleavage (1) or non observed cleavage (0) is shown for each one of the 64 targets. All the variants are mutated in position 75: D75N.

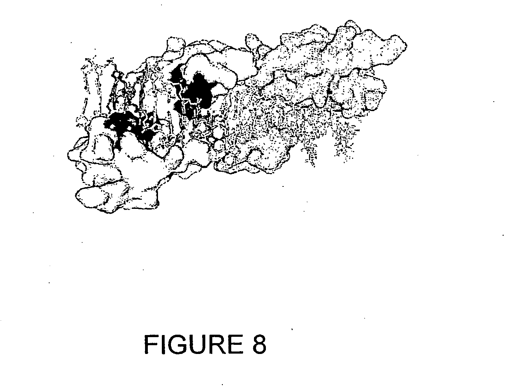

- figure 8 represents the localisation of the mutations in the protein and DNA target, on a I-*Cre*I homodimer bound to its target. The two set of mutations (residues 44, 68 and 70; residues 28, 30, 33, 38 and 40 are shown in black on the monomer on the left. The two sets of mutations are clearly distinct spatially. However, there is no structural evidence for distinct subdomains. Cognate regions in the DNA target site (region -5 to -3; region -10 to -8) are shown in grey on one half site.
- figure 9 represents the Xa series of targets and close derivatives. C1221 (SEQ ID NO: 25) is one of the I-*Cre*I palindromic target sequences. 10TGC_P, 10AGG_P, 5CCT_P and 5TTT (SEQ ID NO: 31, 32, 33, 34) are close derivatives found to be cleaved by I-*Cre*I mutants. They differ from C1221 by the boxed motives. C1221, 10TGC_P, 10AGG_P, 5CCT_P and 5TTT were first described as 24 bp sequences, but structural data suggest that only the 22 bp are relevant for protein/DNA interaction. However, positions ±12 are indicated in parenthesis.Xa.1 (SEQ ID NO:24 ) is the DNA sequence located in the human *XPC* gene at position 9119. In the Xa.2 target (SEQ ID NO:35), the TTGA sequence in the middle of the target is replaced with GTAC, the bases found in C1221. Xa.3 (SEQ ID NO:36) is the palindromic sequence derived from the left part of Xa.2, and Xa.4 (SEQ ID NO:37) is the palindromic sequence derived from the right part of Xa.2. The boxed motives from 10TGC_P, 10AGG_P, 5CCT_P and 5TTT are found in the Xa series of targets.

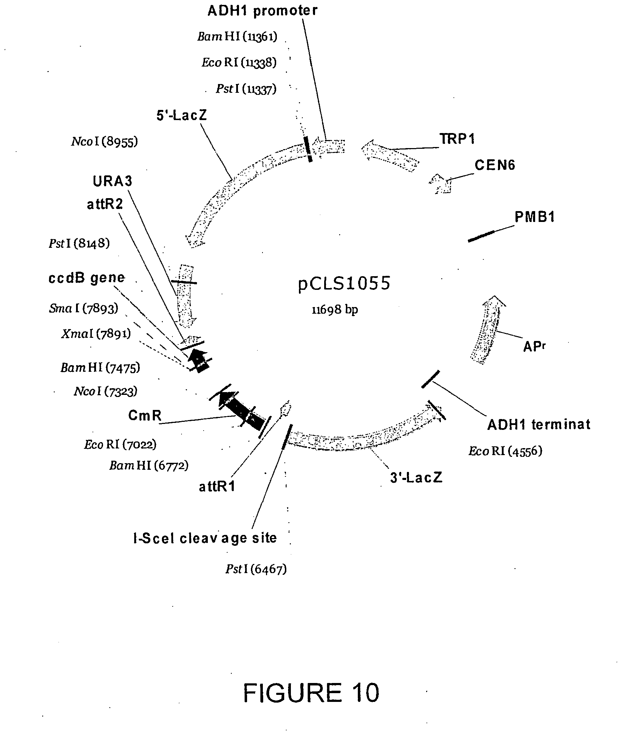

- figure 10 represents the pCLS1055 plasmid vector map.

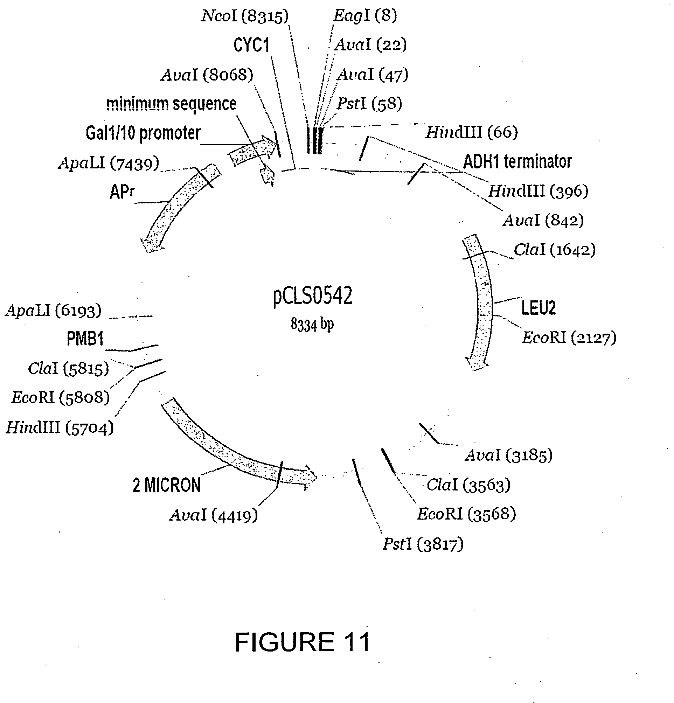

- figure 11 represents the pCLS 10542 plasmid vector map.

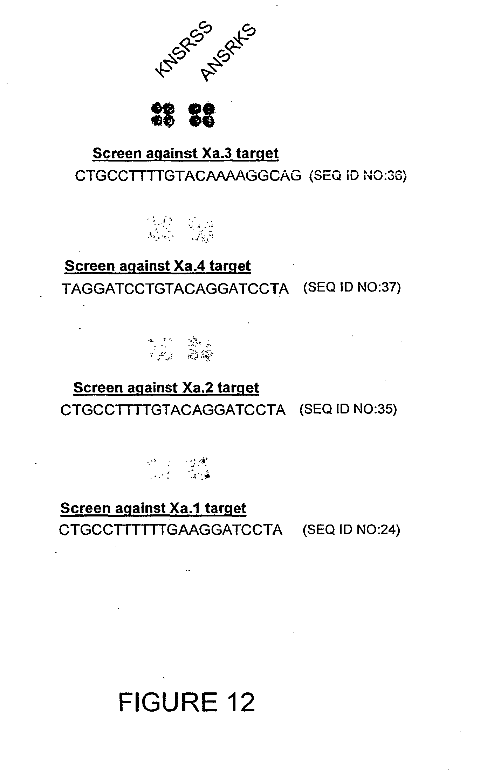

- figure 12 illustrates the cleavage of the Xa.3 target. The figure displays secondary screening of the I-*Cre*I K28, N30, S33, R38, S40, S70 N75 (KNSRSSN) and I-*Cre*I A28, N30, S33, R38, K40, S70 N75 (ANSRKSN) mutants with Xa.1, Xa.2, Xa.3 and Xa.4 targets (SEQ ID NO:24,35, 36, 37).

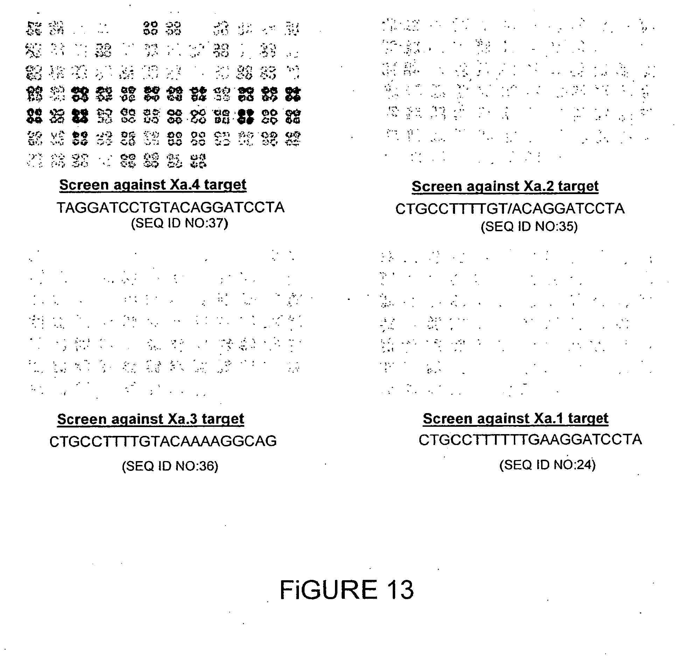

- figure 13 illustrates the cleavage of the Xa.4 target. The figure displays secondary screening of a series of combinatorial mutants among those described in Table XVI with Xa.1, Xa.2, Xa.3 and Xa.4 targets (SEQ ID NO:24,35, 36,37).

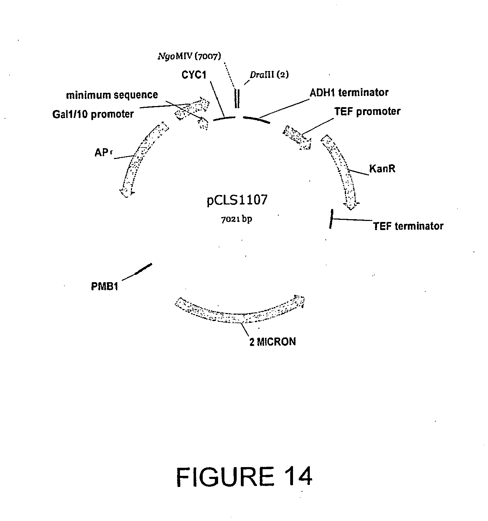

- figure 14 represents the pCLS 1107 vector map.

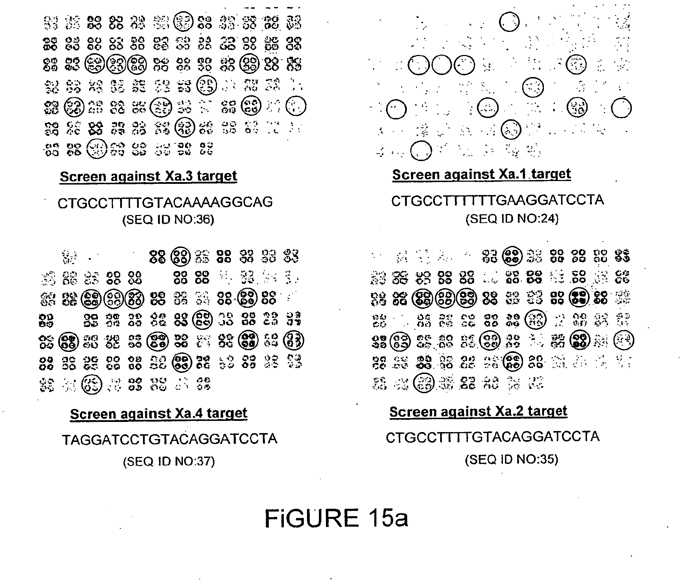

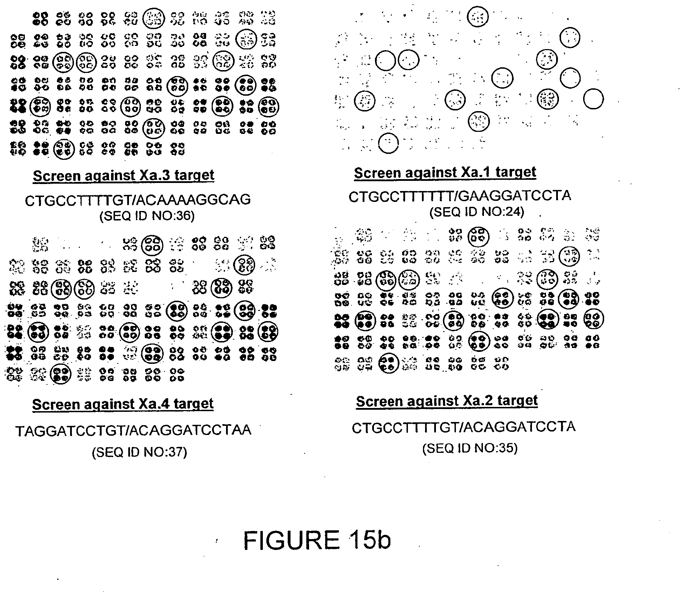

- figure 15 illustrates the cleavage of the Xa.1 and Xa.2 targets. A series of I-*Cre*I N75 mutants cutting Xa.4 are co-expressed with either KNSRSS (a) or ANSRKS (b). Cleavage is tested with the Xa.1, Xa.2, Xa.3 and Xa.4 targets (SEQ ID NO:24, 35, 36, 37). Mutants cleaving Xa.1 are circled.
- figures 16 to 22 illustrate the DNA target sequences found in each exon of the human XP genes (XPA to XPG gene) and the corresponding I-*Cre*I variant which is able to cleave said DNA target. The exons closest to the target sequences, and the exons junctions are indicated (columns 1 and 2), the sequence of the DNA target is presented (column 3), with its position (column 4). The minimum repair matrix for repairing the cleavage at the target site is indicated by its first nucleotide (start, column 7) and last nucleotide (end, column 8). The sequence of each variant is defined by its amino acid residues at the indicated positions. For example, the first heterodimeric variant of figure 16 consists of a first monomer having K, S, R, D, K, R, G and N in positions 28, 33, 38, 40, 44, 68, 70 and 75, respectively and a second monomer having R, D, R, K, A, S, N and I in positions 28, 30, 38, 44, 68, 70, 75 and 77, respectively. The positions are indicated by reference to I-*Cre*I sequence SWISSPROT P05725 or pdb accession code 1g9y; I-*Cre*I has I, Q, K, N, S, Y, Q, S, A, Q, R, R, D, I, E and A, in positions 24, 26, 28, 30, 32, 33, 38, 40, 42, 44, 68, 70, 75, 77, 80 and 133, respectively. The positions which are not indicated may be mutated or not mutated. In the latter case, the positions which are not indicated correspond to the wild-type I-*Cre*I sequence.
- figure 23 represents three targets, derivated from C1221: Xa.1, Xb.1 and Xc.1 (SEQ ID NO: 24, 8, 12), identified in the human *XPC* gene. Each initial target was transformed in a Xx.2 target (SEQ ID NO: 35, 219, 222) more favourable for I-*Cre*I cleavage, then in two palindromics targets Xx.3 (SEQ ID NO: 36, 220, 223) and Xx.4 (SEQ ID NO: 37, 221, 224). Stars represent potential targets found in the gene. Black squares or vertical lines represent the XPC exons.

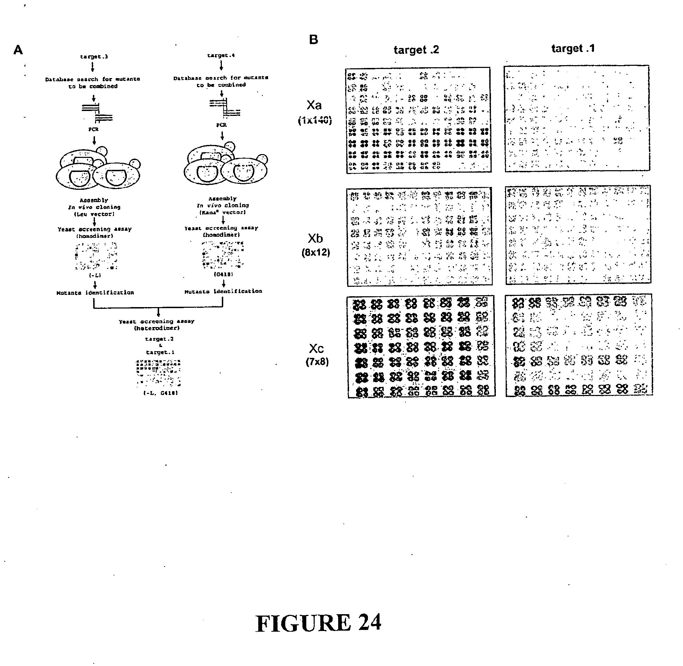

- figure 24 illustrates the strategy for the making and screening of custom-designed Homing Endonucleases. A. General strategy of primary screen. Appropriate I-*Cre*I derivatives with locally altered specificities are identified in the database. Then, a combinatorial approach is used to assemble these mutants by *in vivo* cloning. Active combinatorial mutants are identified as homodimers using a yeast screening assay, on either Xx.3 either Xx.4 targets. Heterodimers were screened by co-expression against both non palindromic targets: Xx.2 and Xx.1. B. Heterodimer Screening Examples. Each new endonuclease is screened on both non palindromic targets: Xx.2 and Xx.1, differing at positions ±2 and ±1. The screening is performed as described previously (International PCT Application WO 2004/067736; Arnould et al., J. Mol. Biol., 2006, 355, 443-458); blue staining indicates cleavage.
- figure 25 illustrates the strategy of activity improvement of custom-designed Homing Endonucleases. A pool of 4 monomers active against Xx.4 is mutagenized by error-prone PCR while its counterpart (monomers active against Xx.3) is used to generate yeast expression strain containing the target Xx.1. The mutagenized library is transformed in a second yeast strain and cloned by *in vivo* cloning. Screening of heterodimer activity is performed by mating of the two yeast strains. The same procedure is then repeated on the I-*Cre*I variants active against the Xx.3 target.

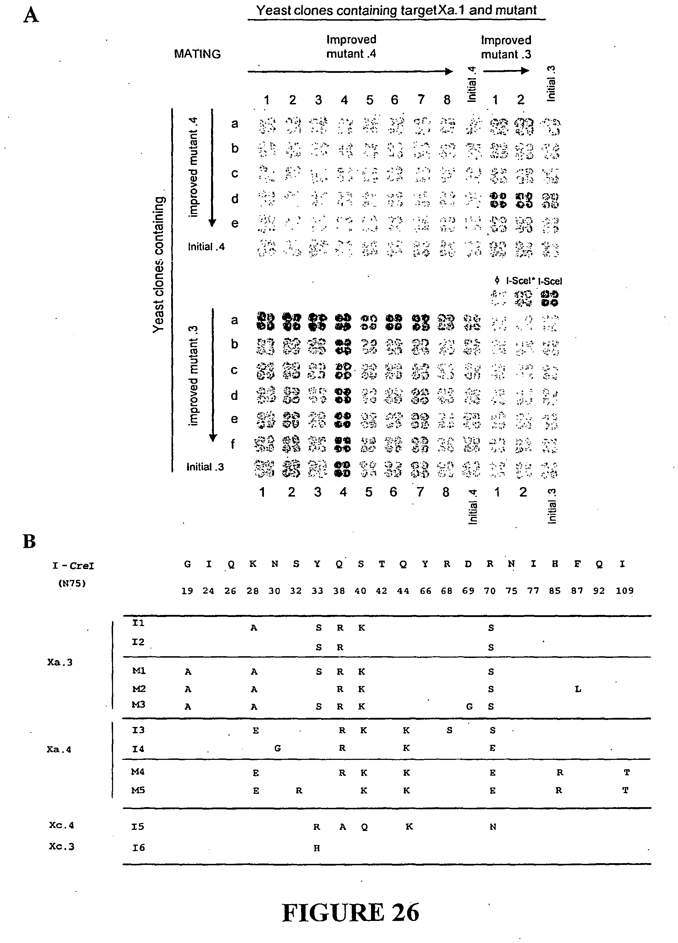

- figure 26 illustrates the screening and characterisation of improved heterodimers cleaving Xb.1 target. A. Final screen of heterodimers from initials to improve versions. Two forms of I-SceI homing endonuclease are used as a control: I-*Sce*I*, I-*Sce*I variant with poor activity; I-*Sce*I, original I-*Sce*I ORF with strong activity. 0: yeast strain transformed with empty vector. Initial: representative mutant activity before improvement. Yeast strains containing the improved mutant active either against Xa.3, or against Xa.4 were mated with yeast strain containing the improved mutants and the Xa.1 target. B. Protein sequences of the most active I-*Cre*I variants. I: Protein sequence before activity improvement. M: Protein sequence after random mutagenesis by error-prone PCR.

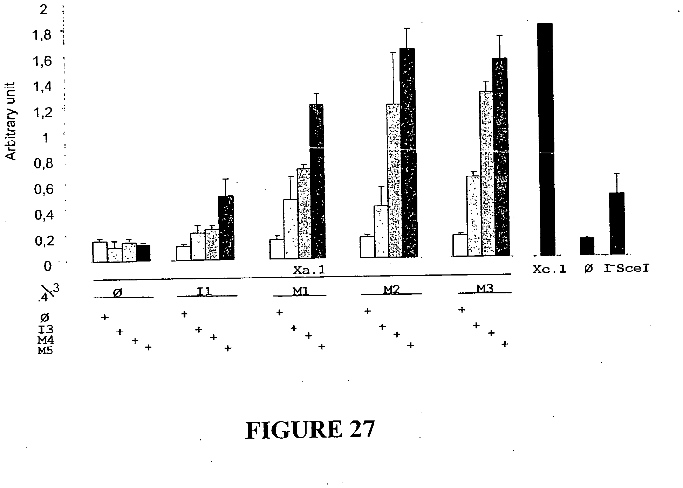

- figure 27 illustrates the heterodimer activity in mammalian cells. Heterodimer activity was quantified by co-expression assay as described in materials and methods section. Ø: CHO transfected with empty vector. I-*Sce*I: CHO transfected with I-*Sce*I expressing vector. I: mutants before activity improvement. M: mutants after activity improvement. All the target vectors carry an extra sub-optimal 18bp I-*Sce*I site with poor cleavage efficiency. It is used as control experiment. The I-*Sce*I activity represents the mean obtained with all target vectors.

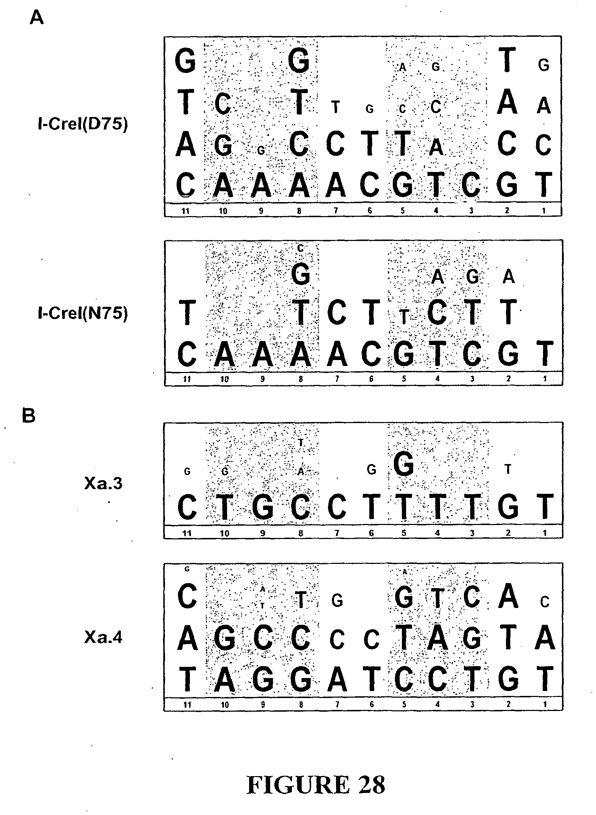

- figure 28 represents the profiling of combinatorial homodimeric mutants. A. The half site of I-*Cre*I C1221 palindromic target is indicated on the bottom line of each box. The individual nucleotide changes tolerated at each position are indicated. The size of the letters is proportional to the activity of the enyme I-*Cre*I D75N and wild-type. B. The half site of Xa.3 and Xa.4 palindromic targets are indicated on the bottom line. The individual nucleotide changes tolerated at each position are indicated for a mutant.3 and a mutant.4, respectively. The size of the letter is proportional to the activity of the mutants.

### Example 1: Engineering of I-CreI variants with modified specificity in positions ±8 to ±10

The method for producing meganuclease variants and the assays based on cleavage-induced recombination in mammal or yeast cells, which are used for screening variants with altered specificity, are described in the International PCT Application WO 2004/067736, Arnould et al., J. Mol. Biol., 2006, 355, 443-458, Epinat et al., N.A.R., 2003, 31, 2952-2962 and Chames et al., Nucleic Acids Res., 2005, 33, e178). These assays result in a functional LacZ reporter gene which can be monitored by standard methods.

### A) Material and methods

### a) Construction of the Ulib4, Ulib5 and Lib4 libraries

I-*Cre*I wt and I-*Cre*I D75N open reading frames were synthesized, as described previously (Epinat et al., N.A.R., 2003, 31, 2952-2962). Mutation D75N was introduced by replacing codon 75 with aac. Three combinatorial libraries (Ulib4, Ulib5 and Lib4) were derived from the I-*Cre*I D75N protein by replacing three different combinations of residues, potentially involved in the interactions with the bases in positions ± 8 to 10 of one DNA target half-site. The diversity of the meganuclease libraries was generated by PCR using degenerated primers harboring a unique degenerated codon (coding for 10 or 12 different amino acids), at each of the selected positions.

The three codons at positions N30, Y33 and Q38 (Ulib4 library) or K28, N30 and Q38 (Ulib5 library) were replaced by a degenerated codon VVK (18 codons) coding for 12 different amino acids: A,D,E,G,H,K,N,P,Q,R,S,T). In consequence, the maximal (theoretical) diversity of these protein libraries was 12³ or 1728. However, is terms of nucleic acids, the diversity was 18³ or 5832. Fragments carrying combinations of the desired mutations were obtained by PCR, using a pair of degenerated primers (Ulib456for and Ulib4rev; Ulib456for and Ulib5rev, figure 5B) and as DNA template, the D75N open reading frame (ORF), (figure 5A). The corresponding PCR products were cloned back into the I-*Cre*I N75 ORF in the yeast replicative expression vector pCLS0542 (Epinat *et al.*, precited), carrying a *LEU2* auxotrophic marker gene. In this 2 micron-based replicative vector, I-*Cre*I variants are under the control of a galactose inducible promoter.

In Lib4, ordered from BIOMETHODES, an arginine in position 70 was first replaced with a serine (R70S). Then positions 28, 33, 38 and 40 were randomized. The regular amino acids (K28, Y33, Q38 and S40) were replaced with one out of 10 amino acids (A,D,E,K,N,Q,R,S,T,Y). The resulting library has a theoretical complexity of 10000 in terms of proteins.

### b) Construction of target clones

The C1221 twenty-four bp palindrome (tcaaaacgtcgtacgacgttttga, (SEQ ID NO: 25) is a repeat of the half-site of the nearly palindromic natural I-*Cre*I target (tcaaaacgtcgtgagacagtttgg, SEQ ID NO: 38). C1221 is cleaved as efficiently as the I-*Cre*I natural target *in vitro* and *ex vivo* in both yeast and mammalian cells. The 64 palindromic targets were derived from C1221 as follows: 64 pairs of oligonucleotides ((ggcatacaagtttcnnnacgtcgtacgacgtnnngacaatcgtctgtca (SEQ ID NO: 39) and reverse complementary sequences) were ordered form Sigma, annealed and cloned into pGEM-T Easy (PROMEGA) in the same orientation. Next, a 400 bp *Pvu*II fragment was excised and cloned into the yeast vector pFL39-ADH-LACURAZ, also called pCLS0042, and the mammalian vector pcDNA3 derivative (pcDNA3.1-LAACZ), both described previously (Epinat *et al.*, 2003, precited), resulting in 64 yeast reporter vectors (target plasmids).

Alternatively, double-stranded target DNA, generated by PCR amplification of the single stranded oligonucleotides, was cloned using the Gateway protocol (INVITROGEN) into yeast and mammalian reporter vectors.

### c) Yeast strains

The library of meganuclease expression variants was transformed into the *leu2* mutant haploid yeast strain FYC2-6A: alpha, trp1Δ63, leu2Δ1, his3Δ200. A classical chemical/heat choc protocol that routinely gives us 10⁶ independent transformants per µg of DNA derived from (Gietz and Woods, Methods Enzymol., 2002, 350, 87-96), was used for transformation. Individual transformant (Leu⁺) clones were individually picked in 96 wells microplates.13824 colonies were picked using a colony picker (QpixII, GENETIX), and grown in 144 microtiter plates.

The 64 target plasmids were transformed using the same protocol, into the haploid yeast strain FYBL2-7B: a, ura3Δ851, trp1Δ63, leu2Δ1, lys2Δ202, resulting in 64 tester strains.

### d) Mating of meganuclease expressing clones and screening in yeast

Meganuclease expressing clones were mated with each of the 64 target strains, and diploids were tested for beta-galactosidase activity, by using the screening assay illustrated on figure 2 of Arnould *et al.*, 2006, precited. I-*Cre*I variant clones as well as yeast reporter strains were stocked in glycerol (20 %) and replicated in novel microplates. Mating was performed using a colony gridder (QpixII, GENETIX). Mutants were gridded on nylon filters covering YPD plates, using a high gridding density (about 20 spots/cm²). A second gridding process was performed on the same filters to spot a second layer consisting of 64 different reporter-harboring yeast strains for each variant. Membranes were placed on solid agar YPD rich medium, and incubated at 30°C for one night, to allow mating. Next, filters were transferred to synthetic medium, lacking leucine and tryptophan, with galactose (2 %) as a carbon source (and with G418 for coexpression experiments), and incubated for five days at 37°C, to select for diploids carrying the expression and target vectors. After 5 days, filters were placed on solid agarose medium with 0.02% X-Gal in 0.5 M sodium phosphate buffer, pH 7.0, 0.1 % SDS, 6 % dimethyl formamide (DMF), 7 mM β-mercaptoethanol, 1 % agarose, and incubated at 37°C, to monitor β-galactosidase activity. After two days of incubation, positive clones were identified by scanning. The β-galactosidase activity of the clones was quantified using appropriate software. The clones showing an activity against at least one target were isolated (first screening). The spotting density was then reduced to 4 spots/cm² and each positive clone was tested against the 64 reporter strains in quadruplicate, thereby creating complete profiles (secondary screening).

### e) Sequence

The open reading frame (ORF) of positive clones identified during the first and/or secondary screening in yeast was amplified by PCR on yeast colonies using primers: PCR-Ga110-F (gcaactttagtgctgacacatacagg, SEQ ID NO:40) and PCR-Ga110-R (acaaccttgattgcagacttgacc, SEQ ID NO:41) or 5'ggggacaagtttgtacaaaaaagcaggcttcgaaggagatagaaccatggccaataccaaatataacaaagagttcc3' (SEQ ID NO: 225) and 5'ggggaccactttgtacaagaaagctgggtttagtcggccgccggggaggatttcttcttctcgc 3' (SEQ ID NO: 226), from PROLIGO. Briefly, yeast colony is picked and resuspended in 100 µl of LGlu liquid medium and cultures overnight. After centrifugation, yeast pellet is resuspended in 10 µl of sterile water and used to perform PCR reaction in a final volume of 50 µl containing 1.5 µl of each specific primers (100 pmol/µl). The PCR conditions were one cycle of denaturation for 10 minutes at 94°C, 35 cycles of denaturation for 30s at 94°C, annealing for 1 min at 55 °C, extension for 1.5 min at 72°C, and a final extension for 5 min. The resulting PCR products were then sequenced.

### f) Re-Cloning of primary hits

The open reading frames (ORFs) of positive clones identified during the primary screening were recloned using the Gateway protocol (Invitrogen). ORFs were amplified by PCR on yeast colonies, as described in e). PCR products were then cloned in : (i) yeast gateway expression vector harboring a galactose inducible promoter, *LEU2* or KanR as selectable marker and a 2 micron origin of replication, (ii) a pET 24d(+) vector from NOVAGEN, and (iii) a CHO gateway expression vector pcDNA6.2 from INVITROGEN. Resulting clones were verified by sequencing (MILLEGEN).

### B) Results

I-*Cre*I is a dimeric homing endonuclease that cleaves a 22 bp pseudo-palindromic target. Analysis of I-*Cre*I structure bound to its natural target has shown that in each monomer, eight residues establish direct interactions with seven bases (Jurica et al., 1998, precited). According to these structural data, the bases of the nucleotides in positions ± 8 to 10 establish specific contacts with I-*Cre*I amino-acids N30, Y33 and Q38 (figure 3). Thus, novel proteins with mutations in positions 30, 33 and 38 could display novel cleavage profiles with the 64 targets resulting from substitutions in positions ± 8, ± 9 and ± 10 of a palindromic target cleaved by I-*Cre*I. In addition, mutations might alter the number and positions of the residues involved in direct contact with the DNA bases. More specifically, positions other than 30, 33, 38, but located in the close vicinity on the folded protein, could be involved in the interaction with the same base pairs.

An exhaustive protein library vs. target library approach was undertaken to engineer locally this part of the DNA binding interface. First, the I-*Cre*I scaffold was mutated from D75 to N. The D75N mutation did not affect the protein structure, but decreased the toxicity of I-*Cre*I in overexpression experiments.

Next the Ulib4 library was constructed: residues 30, 33 and 38, were randomized, and the regular amino acids (N30, Y33, and Q38) replaced with one out of 12 amino acids (A,D,E,G,H,K,N,P,Q,R,S,T). The resulting library has a complexity of 1728 in terms of protein (5832 in terms of nucleic acids).

Then, two other libraries were constructed: Ulib5 and Lib4. In Ulib5, residues 28, 30 and 38 were randomized, and the regular amino acids (K28, N30, and Q38) replaced with one out of 12 amino acids (ADEGHKNPQRST). The resulting library has a complexity of 1728 in terms of protein (5832 in terms of nucleic acids). In Lib4, an Arginine in position 70 was first replaced with a Serine. Then, positions 28, 33, 38 and 40 were randomized, and the regular amino acids (K28, Y33, Q38 and S40) replaced with one out of 10 amino acids (A,D,E,K,N,Q,R,S,T,Y). The resulting library has a complexity of 10000 in terms of proteins.

In a primary screening experiment, 20000 clones from Ulib4, 10000 clones from Ulib5 and 20000 clones from Lib4 were mated with each one of the 64 tester strains, and diploids were tested for beta-galactosidase activity. All clones displaying cleavage activity with at least one out of the 64 targets were tested in a second round of screening against the 64 targets, in quadriplate, and each cleavage profile was established. Then, meganuclease ORF were amplified from each strain by PCR, and sequenced, and 141 different meganuclease variants were identified.

The 141 validated clones showed very diverse patterns. Some of these new profiles shared some similarity with the wild type scaffold whereas many others were totally different. Results are summarized in figure 6. Homing endonucleases can usually accommodate some degeneracy in their target sequences, and the I-*Cre*I N75 scaffold protein itself cleaves a series of 4 targets, corresponding to the aaa, aac, aag, an aat triplets in positions ±10 to ±8. A strong cleavage activity is observed with aaa, aag and aat, whereas AAC is only faintly cut (and sometimes not observed). Similar pattern is found with other proteins, such as I-*Cre*I K28 N30 D33 Q38 S40 R70 N75, I-*Cre*I K28 N30 Y33 Q38 S40 R70 N75. With several proteins, such as I-CreI R28 N30 N33 Q38 D40 S70 N75 and I-*Cre*I K28 N30 N33 Q38 S40 R70 N75, aac is not cut anymore.

However, a lot of proteins display very different patterns. With a few variants, cleavage of a unique sequence is observed. For example, protein I-*Cre*I K28 R30 G33 T38 S40 R70 N75 is active on the "ggg" target, which was not cleaved by wild type protein, while I-*Cre*I Q28 N30 Y33 Q38 R40 S70 N75 cleaves AAT, one of the targets cleaved by I-*Cre*I N75. Other proteins cleave efficiently a series of different targets: for example, I-*Cre*I N28 N30 S33 R38 K40 S70 N75 cleaves ggg, tgg and tgt, *Cre*I K28 N30 H33 Q38 S40 R70 N75 cleaves aag, aat, gac, gag, gat, gga, ggc, ggg, and ggt. The number of cleaved sequences ranges from 1 to 10. Altogether, 37 novel targets were cleaved by the mutants, including 34 targets which are not cleaved by I-*Cre*I and 3 targets which are cleaved by I-*Cre*I (aag, aat and aac, figure 6).

### Example 2: Strategy for engineering novel meganucleases cleaving a target from the XPC gene

A first series of I-*Cre*I variants having at least one substitution in positions 44, 68 and/or 70 of I-*Cre*I and being able to cleave mutant I-*Cre*I sites having variation in positions ± 3 to 5 was identified previously (Arnould et al., J. Mol. Biol., 2006, 355, 443-458). The cleavage pattern of the variants is presented in figure 7.

A second series of I-*Cre*I variants having at least one substitution in positions 28, 30, 33 or 28, 33, 38 and 40 of I-*CreI* and being able to cleave mutant I-*Cre*I sites having variation in positions ± 8 to 10 was identified as described in example 1. The cleavage pattern of the variants is presented in figure 6.

Positions 28, 30, 33, 38 and 40 on one hand, and 44, 68 and 70, on another hand are on a same DNA-binding fold, and there is no structural evidence that they should behave independently. However, the two sets of mutations are clearly on two spatially distinct regions of this fold (figure 8) located around different regions of the DNA target. These data suggest that I-*Cre*I comprises two independent functional subunits which could be combined to cleave novel chimeric targets. The chimeric target comprises the nucleotides in positions ± 3 to 5 and ± 8 to 10 which are bound by each subdomain.

This hypothesis was verified by using targets situated in a gene of interest, the *XPC* gene. The targets cleaved by the I-*Cre*I variants are 24 bp derivatives of C1221, a palindromic sequence cleaved by I-*Cre*I. However, the structure of I-*Cre*I bound to its DNA target suggests that the two external base pairs of these targets (positions -12 and 12) have no impact on binding and cleavage (Chevalier et al., Nat. Struct. Biol., 2001, 8, 312-316; Chevalier, B.S. and B.L. Stoddard, Nucleic Acids Res., 2001, 29, 3757-3774; Chevalier et al., 2003, J. Mol. Biol., 329, 253-269) and in this study, only positions -11 to 11 were considered. Consequently, the series of targets identified in the *XPC* gene were defined as 22 bp sequences instead of 24 bp.

Xa.1, Xb.1 and Xc.1 are 22 bp (non-palindromic) targets located at position 9119, 13521 and 20438, respectively of the human *XPC* gene (figures 1A, 9 and 23). The meganucleases cleaving Xa.1, Xb.1 or Xc.1 could be used to correct mutations in the vicinity of the cleavage site (figure 1B). Since the efficiency of gene correction decreases when the distance to the DSB increases (Elliott et al., Mol Cell Biol, 1998, 18, 93-101), this strategy would be most efficient with mutations located within 500 bp of the cleavage site. For example, meganucleases cleaving Xc.1 could be used to correct mutations in Exon 9 (deletion DEL1132AA or insertion insVAL580, Figure 1A). Alternatively, the same meganucleases could be used to knock-in exonic sequences that would restore a functional *XPC* gene at the XPC locus (figure 1C). This strategy could be used for any mutation downstream of the cleavage site.

Xa.1 is partly a patchwork of the 10TGC_P, 10AGG_P, 5TTT_P and 5CCT_P targets (figures 9 and 23) which are cleaved by previously identified meganucleases (figures 6 and 7). Thus, Xa.1 could be cleaved by combinatorial mutants resulting from these previously identified meganucleases.

Xb.1 is partly a patchwork of the 10GGG_P, 10TGT_P, 5GGG_P and 5TAC_P targets (figure 23) which are cleaved by previously identified meganucleases (figures 6 and 7). Thus, Xb-1 could be cleaved by combinatorial mutants resulting from these previously identified meganucleases.

Xc.1 is partly a patchwork of the 10GAG_P, 10GTA_P and 5TCT_P targets (figure 23) which are cleaved by previously identified meganucleases (figures 6 and 7), and 5GTC_P which is the sequence of C1221 cleaved by I-*Cre*I. Thus, Xc.1 could be cleaved by combinatorial mutants resulting from these previously identified meganucleases.

Therefore, to verify this hypothesis, two palindromic targets, corresponding to the left (Xx.3) and right half (Xx.4) sequences of the identified targets (Xx.1) were produced (figures 9 and 23). These two derived palindromic targets keep the GTAC sequence from the C1221 palindromic I-*Cre*I target at positions -2 to +2 (Figures 9 and 23). Since Xx.3 and Xx.4 are palindromic, they should be cleaved by homodimeric proteins. In a first step, proteins able to cleave the Xx.3 and Xx.4 sequences as homodimers were designed (examples 3 and 4), as illustrated in figure 24A. In a second step, the proteins obtained in examples 3 and 4 were co-expressed to obtain heterodimers cleaving Xx.2 and Xx.1, for some heterodimers (example 5), as illustrated in figure 24A.

### Example 3: Making of meganucleases cleaving Xx.3

This example shows that I-*Cre*I mutants can cut the Xx.3 DNA target sequences derived from the left part of the Xx.2 targets in a palindromic form (figures 9 and 23). Target sequences described in this example are 22 bp palindromic sequences. Therefore, they will be described only by the first 11 nucleotides, followed by the suffix _P; for example, target Xa.3 will be noted also c**tgc**ct**t**t**t**gt_P,

Xa.3 is similar to 5TTT_P in positions ±1, ±2, ±3, ±4, ±5 and ±11 and to 10TGC_Pin positions ±1, ±2, ±8, ±9, ±10 and ±11.

Xb.3 is similar to 5GGG_P in positions ±1, ±2, ±3, ±4, ±5 and ±11 and to 10GGG_P in positions ±1, ±2, ±8, ±9, ±10 and ±11

Xc.3 is similar to C1221 in positions ±1, ±2, ±3, ±4, ±5, ±7 and ±11 and to 10GAG_P in positions ±1, ±2, ±7, ±8, ±9, ±10 and ±11

The wild-type I-*Cre*I is known to be tolerant for nucleotide substitutions at positions ±11, ±7 and ±6 (Chevalier et al., J. Mol. Biol., 2003, 329, 253-269; Jurica et al., Mol. Cell., 1998, 2, 469-476). Thus, it was hypothesized that positions ±6 and ±7 would have little effect on the binding and cleavage activity.

Mutants able to cleave the 5TTT_P and 5GGG_P targets were previously obtained by mutagenesis on I-*Cre*I N75 at positions 44, 68 and 70 or I-*Cre*I S70 at positions 44, 68, 75 and 77, as described in Arnould et al., J. Mol. Biol., 2006, 355, 443-458 (figure 7). Mutants able to cleave the 10TGC_P, 10GGG_P, and 10TGA_P targets were obtained by mutagenesis on I-*Cre*I N75 at positions 28, 30, 38, or 30, 33, 38, on I-*Cre*I S70 N75 at positions 28, 33, 38 and 40 and 70, or on I-*Cre*I D75 or N75 at two positions chosen from 28, 30, 32, 33, 38 and 40 (example 1 and figure 6). Thus, combining such pairs of mutants would allow for the cleavage of the Xx.3 target.

Some sets of proteins are both mutated at position 70. However, it was hypothesized that two separable functional subdomains exist in I-*Cre*I. That implies that this position has little impact on the specificity in bases 10 to 8 of the target.

Therefore, to check whether combined mutants could cleave the Xa.3 and Xb.3 targets, mutations at positions 44, 68, 70 and/or 75 from proteins cleaving the 5NNN region of the target (5TTT_P and 5GGG_P targets) were combined with the 28, 30, 32, 33, 38 and/or 40 mutations from proteins cleaving the 10NNN region of the targets (10TGC_P and 10GGG_P targets), as illustrated in figure 24A.

Xc.3 which is identical to C1221 in positions ± 3 to 5 should be cleaved by previously identified mutants cleaving the 10GAG_P target (no combination of mutations).

### A) Material and Methods

### a) Construction of target vector:

The C1221 derived target was cloned as follows: oligonucleotide corresponding to the target sequence flanked by gateway cloning sequence was ordered from Proligo (as example: 5' tggcatacaagtttctgccttttgtacaaaaggcagacaatcgtctgtca 3' (SEQ ID NO: 42, for the Xa.3 target). Double-stranded target DNA, generated by PCR amplification of the single stranded oligonucleotide, was cloned using the *Gateway^{R}* protocol (INVITROGEN) into yeast reporter vector (pCLS1055, figure 10). Yeast reporter vector was transformed into *S. cerevisiae* strain FYBL2-7B (MAT alpha, ura3Δ851, trp1Δ63, leu2Δ1, lys2Δ202).

### b) Construction of combinatorial mutants:

I-*Cre*I mutants cleaving 10TGC_P, 10GGG_P, 5TTT_P or 5GGG_P were identified as described in example 1 and figure 6, and Arnould et al., J. Mol. Biol., 2006, 355, 443-458 and figure 7, respectively for the 10TGC_P, 10GGG_P and the 5TTT_P, 5GGG_P targets. In order to generate I-*Cre*I derived coding sequence containing mutations from both series, separate overlapping PCR reactions were carried out that amplify the 5' end (amino acid positions 1-43) or the 3' end (positions 39-167) of the I-*Cre*I coding sequence. For both the 5' and 3' end, PCR amplification is carried out using primers specific to the vector (pCLS0542, figure 11): Gal10F or Gal10R and primers specific to the I-*Cre*I coding sequence for amino acids 39-43 (assF 5'-ctannnttgaccttt-3' (SEQ ID NO: 43) or assR 5'-aaaggtcaannntag-3' (SEQ ID NO: 44)) where nnn codes for residue 40. The resulting PCR products contain 15bp of homology with each other and approximately 100-200 bp of homology with the 2 micron-based replicative vectors, pCLS0542, marked with the *LEU2* gene and pCLS1107, containing a kanamycin resistant gene.

Thus, to generate an intact coding sequence by *in vivo* homologous recombination in yeast, approximately 25 ng of each of the two overlapping PCR fragments and 25 ng of the pCLS0542 vector DNA linearized by digestion with *Nco*I and *Eag*I or 25ng of the pCLS 1107 vector DNA linearized by digestion with *Dra*III and *NgoM*IV were used to transform the yeast *Saccharomyces cerevisiae* strain FYC2-6A (MATα, trp1Δ63, leu2Δ1, his3Δ200) using a high efficiency LiAc transformation protocol (Gietz, R.D. and R.A. Woods, Methods Enzymol, 2002, 350, 87-96). Combinatorial mutants can advantageously be generated as libraries: PCR reactions were pooled in equimolar amounts and transformed into yeast together with the linearized plasmid. Transformants were selected on either synthetic medium lacking leucine (pCLS0542) or rich medium containing G418 (pCLS1107). Colonies were picked using a colony picker (QpixII, Genetix), and grown in 96 well microtiter plates.

### c) Mating of meganuclease expressing clones and screening in yeast:

The experimental procedure is as described in example 1, except that a low gridding density (about 4 spots/cm²) was used.

### c) Sequencing of mutants

To recover the mutant expressing plasmids, yeast DNA was extracted using standard protocols and used to transform *E. coli*. Sequencing of mutant ORF was then performed on the plasmids by MILLEGEN SA. Alternatively, the ORFs of positive clones identified during the primary screening in yeast were amplified by PCR on yeast DNA extract from colonies (Akada et al., Biotechniques, 2000, 28(4): 668-70, 672, 674) using primers 5'ggggacaagtttgtacaaaaaagcaggcttcgaaggagatagaaccatggccaataccaaatataacaaagagttcc3' (SEQ ID NO: 225) and 5'ggggaccactttgtacaagaaagctgggtttagtcggccgccggggaggatttcttcttctcgc 3' (SEQ ID NO: 226) from PROLIGO and sequencing was performed directly on PCR product by MILLEGEN. PCR products were cloned either in (i) yeast gateway expression vectors harboring a galactose inducible promoter, *LEU2* or KanR as selectable marker and a 2 micron origin of replication, (ii) CHO gateway expression vector pCDNA6.2 from INVITROGEN. Resulting clones were verified by sequencing (MILLEGEN).

### B) Results

I-*Cre*I N75 mutants cutting the 10TGC_P (**ctgc**acgtcgt_P) target and I-*Cre*I N75 (Q44, R68, R70) mutants cutting the 5TTT_P (caaaac**t**t**t**gt_P) target were combined, resulting in combinatorial mutants that were screened against the Xa.3 target (**ctgc**ct**t**t**t**gt_P).

I-*Cre*I N75 mutants cutting the 10GGG_P target and I-*Cre*I N75 mutants cutting the 5GGG_P target were combined, resulting in combinatorial mutants that were screened against the Xb.3 target.

At least twice the diversity of each library was screened. No correlation was observed between the number of active mutants identified and the number of combinations tested.

The Xc.3 target sequence contained the wild type sequence GTC at positions ±5, ±4 and ±3; therefore the combinatorial approach was not necessary to generate specific variants towards this target. I-*Cre*I variants previously identified with altered substrate specificity towards bases ±10, ±9 and ±8 were directly screened against the Xc.3 DNA target.

**Table XVI: Mutants used for combinatorial construction for the palindromic target Xx.3**

| **Targets** | **Number of I-CreI mutants used for the combinatorial reaction** | | **diversity** | **Tested clones (X diversity)** | **Number of combined unique positive clones** |
|---|---|---|---|---|---|
| | **10NNN_P X** | **5NNN_P** | | | |
| **Xa.3** | 510 31 | na* 19 | 1099 | 2 | 8 (0.7 %) |
| **Xb.3** | 45 | 32 | 1440 | 2 | 30 (2 %) |
| **Xc.3** | 811 | na* | 811 | na* | 21 (2 %) |

| | | | | | |
|---|---|---|---|---|---|
| na: non applicable *: only I-*Cre*I variants with altered specificity towards nucleotides ±10, ±9 and ±8 were screened. | | | | | |

Eight combinatorial mutants were found cleave the Xa.3 target (Table XVI). Two of the mutants cleaving the Xa.3 target have the following sequence:
- I-*Cre*I K28, N30, S33, R38, S40, S70 and N75 (called KNSRSSN), obtained by combination of I-*Cre*I K28, N30, S33, R38, S40 and I-*Cre*I S70,N75, and
- I-*Cre*I A28, N30, S33, R38, K40, S70 and N75 (called ANSRKSN), obtained by combination of I-*Cre*I K28, N30, S33, R38, K40 and I-*Cre*I S70, N75.

Thirty combinatorial mutants were found to cleave the Xb.3 target (Table XVI).

Among the mutants cutting the 10GAG_P target which were tested, twenty one were found to cleave the Xc.3 target (Table XVI).

Results were confirmed in a secondary screen (figure 12) and the predicted amino acid primary structure was confirmed by sequencing.

### Example 4: Making of meganucleases cleaving Xx.4

This example shows that I-*Cre*I variants can cleave the Xx.4 DNA target sequence derived from the right part of the Xx.2 target in a palindromic form (figures 9 and 23). All target sequences described in this example are 22 bp palindromic sequences. Therefore, they will be described only by the first 11 nucleotides, followed by the suffix _P; for example, Xa.4 will be called taggatcctgt_P (SEQ ID NO: 37).

Xa.4 is similar to 5CCT_P in positions ±1, ±2, ±3, ±4, ±5 and ±7 and to 10AGG_P in positions ±1, ±2, ±7, ±8, ±9 and ±10. It was hypothesized that positions ±6 and ±11 would have little effect on the binding and cleavage activity.

Xb.4 is similar to 5TAC_P in positions ±1, ±2, ±3, ±4, ±5, ±6 and ±11 and to 10TGT_P in positions ±1, ±2, ±6, ±8, ±9, ±10, and ±11. It was hypothesized that positions ±7 would have little effect on the binding and cleavage activity.

Xc.4 is similar to 5TCT_P in positions ±1, ±2, ±3, ±4, ±5, ±7 and ±11 and to 10GTA_P in positions ±1, ±2, ±7, ±8, ±9, ±10, and ±11. It was hypothesized that positions ±6 would have little effect on the binding and cleavage activity.

Mutants able to cleave the 5CCT_P, 5TAC_P and 5TCT_P targets were previously obtained by mutagenesis on I-*Cre*I N75 at positions 44, 68 and 70 or I-*Cre*I S70 at positions 44, 68, 75 and 77, as described in Arnould et al., J. Mol. Biol., 2006, 355, 443-458 (figure 7). Mutants able to cleave the 10AGG_P, 10TGT_P, and 10GTA_P targets were obtained by mutagenesis on I-*Cre*I N75 at positions 28, 30, 38, or 30, 33, 38, on I-*Cre*I S70 N75 at positions 28, 33, 38 and 40 and 70, or on I-*Cre*I D75 or N75 at two positions chosen from 28, 30, 32, 33, 38 and 40 (example 1 and figure 6). Thus, combining such pairs of mutants would allow for the cleavage of the Xx.4 target.

Some sets of proteins are both mutated at position 70. However, it was hypothesized that I-*Cre*I comprises two separable functional subdomains. That implies that this position has little impact on the specificity in base 10 to 8 of the target.

Therefore, to check whether combined mutants could cleave the Xx.4 target, mutations at positions 44, 68, 70 and/or 75 from proteins cleaving 5CCT_P, 5TAC_P and 5TCT_P targets were combined with the 28, 30, 32, 33, 38, and/or 40 mutations from proteins cleaving 10AGG_P, 10TGT_P, and 10GTA_P targets, as illustrated in figure 24A.

### A) Material and Methods

See example 3.

### B) Results

I-*Cre*I combined mutants were constructed by associating mutations at positions 44, 68 and 70 with the 28, 30, 33, 38 and 40 mutations on the I-*Cre*I N75 scaffold. Combined mutants were screened against the Lax.4 DNA targets At least twice the diversity of each library was screened. No correlation was observed between the number of active mutants identified and the number of combinations tested. Two percent of the hybrid mutants appear to be functional for the Xb.4 DNA targets while as many as 55% were active against Xa.4. After secondary screening and sequencing, 104, 8 and 4 different cleavers were identified, for the Xa.4, Xb.4 and Xc.4 target, respectively (Table XVII).

**Table XVII: Mutants used for combinatorial construction for the palindromic target Xx.4**

| **Targets** | **Number of I-Crel mutants used for the combinatorial reaction** | | **diversity** | **Tested clones (X diversity)** | **Number of combined unique positive clones** |
|---|---|---|---|---|---|
| | **10NNN_P X** | **5NNN_P** | | | |
| **Xa.4** | 9 | 21 | 189 | 4 | 104(55%) |
| **Xb.4** | 7 | 46 | 322 | 5 | 8(2%) |
| **Xc.4** | 3 | 47 | 141 | 3 | 24 (17 %) |

| | | | | | |
|---|---|---|---|---|---|
| na: non applicable *: only I-*Cre*I variants with altered specificity towards nucleotides 10, 9 and 8 were screened. | | | | | |

I-*Cre*I N75 mutants cutting the 10AGG_P (c**agg**acgtcgt_P; SEQ ID NO: 32) target (amino acids at positions 28, 30, 33, 38 and 40 are indicated) and I-*CreI* N75 mutants cutting the 5CCT_P (caaaac**cct**gt_P; SEQ ID NO: 34) target (amino acids at positions 44, 68 and 70 are indicated) are listed in Table XVIII. 39 of the 104 positives are presented in figure 13 and Table XVIII.

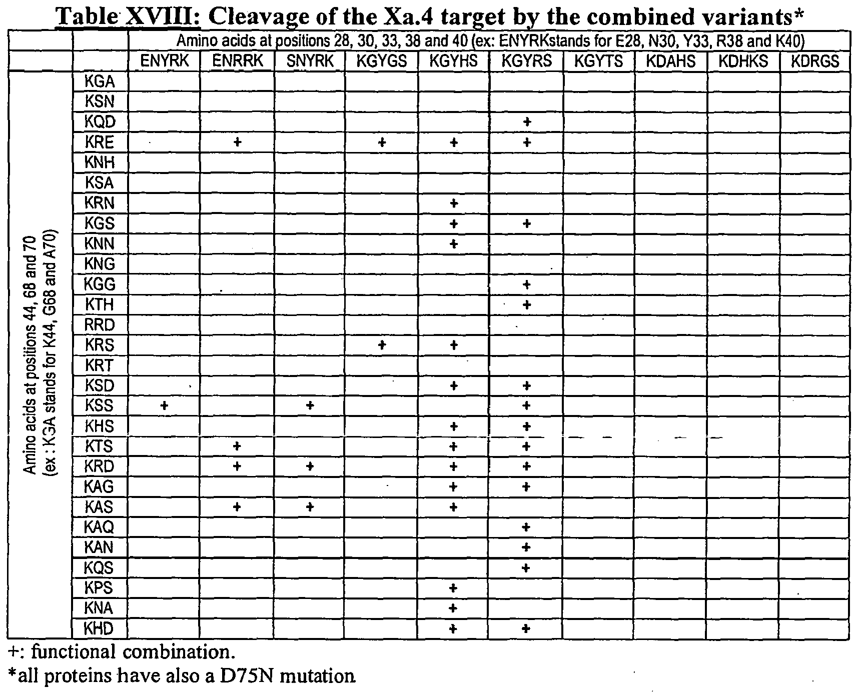

### Example 5: Malting of meganucleases cleaving Xx.2 and Xx.1.1

I-*Cre*I mutants able to cleave each of the palindromic Xx.2 derived targets Xx.3 and Xx.4, were identified in examples 3 and 4. A subset of pairs of such mutants (one cutting Xx.3 and one cutting Xx.4), were co-expressed in yeast. Upon co-expression, there should be three active molecular species, two homodimers, and one heterodimer. It was assayed whether the heterodimers that should be formed could cleave the Xx.1 and Xx.2 targets as depicted in figure 24A.

### A) Material and Methods

### a) Cloning of mutants in kanamycin resistant vector:

In order to co-express two I-*Cre*I mutants in yeast, mutants cutting the Xx.3 sequence were subcloned in a kanamycin resistant yeast expression vector (pCLS 1107, figure 14).

Mutants were amplified by PCR reaction using primers common for leucine vector (pCLS0542) and kanamycin vector (pCLS1107) (Gal10F and Gal10R). Approximately 25ng of PCR fragment and 25ng of vector DNA (pCLS1107) linearized by digestion with *DraIII* and *NgoMIV* are used to transform the yeast *Saccharomyces cerevisiae* strain FYC2-6A (MATα, trp1Δ63, leu2Δ1, his3Δ200) using a high efficiency LiAc transformation protocol. An intact coding sequence for the I-*Cre*I mutant is generated by *in vivo* homologous recombination in yeast.

### b) Mutants co-expression:

Yeast strain expressing a mutant cutting the Xx.4 target was transformed with DNA coding for a mutant cutting the Xx.3 target in pCLS1107 expression vector. Transformants were selected on -L Glu + G418 medium.

### c) Mating of meganucleases coexpressing clones and screening in yeast

The experimental procedure is as described in example 1, except that a low gridding density (about 4 spots/cm²) was used.

### B) Results:

The Table XIX summarizes the number of total and active heterodimers tested by co-expression in yeast against the targets Xx.2 and Xx.1.

**Table XIX: Mutants used for the heterodimeric assay for each XPC target**

| **Targets** | **Number of XPC mutants used for heterodimeric assay I** | | **Number of positives heterodimer combinations** | |
|---|---|---|---|---|
| | **target .3 X** | **target .4** | **target.2** | **target .1** |
| **Xa** | 2 | 104 | 156 | 15 |
| **Xb** | 12 | 8 | 43 | 0 |
| **Xc** | 7 | 8 | 56 | 41 |

In all cases, heterodimers with cleavage activity for the target.2 were identified (figures 15a, 15b and 24B). All of the heterodimers tested were active against Xc.2 while 75 % and 45 % were able to cleave the Xa.2 and Xb.2 targets respectively. On the other hand, while the 4 central nucleotides sequences TTGA, GAAA, and ACAC found respectively in Xa.1, Xb.1 and Xc.1 targets did not impact the I-*Cre*I cleavage activity when inserted in the I-*Cre*I C1221 palindromic target, only a sub-fraction of the active heterodimers against Xa.2 and Xc.2 were able to cleave the Xa.1 and Xc.1 targets, respectively, while no cutters were found for the Xb.1 target (figures 15a, 15b and 24B). The influence of the central sequence has been explained by its particular topology. Structure analysis revealed that this DNA region of the target is a region of maximal DNA bending with a curvature of around 50° resulting in base twisting and unstacking near the scissile phosphate groups (Chevalier et al., J. Mol. Biol., 2003329, 253-269). However, a precise mechanism explaining why certain sequences at the four central positions are compatible with cleavage activity and some others are not has still to be described.

Examples of functional combinations for the Xa.1 target are presented in Tables XX and XXI. As a general rule, functional heterodimers cutting Xx.1 sequence were always obtained when the two expressed proteins gave a strong signal as homodimer.Moreover, while many mutants are still very active against Xc.1, the mutants capable of cleaving the Xa.1 target displayed a weak activity

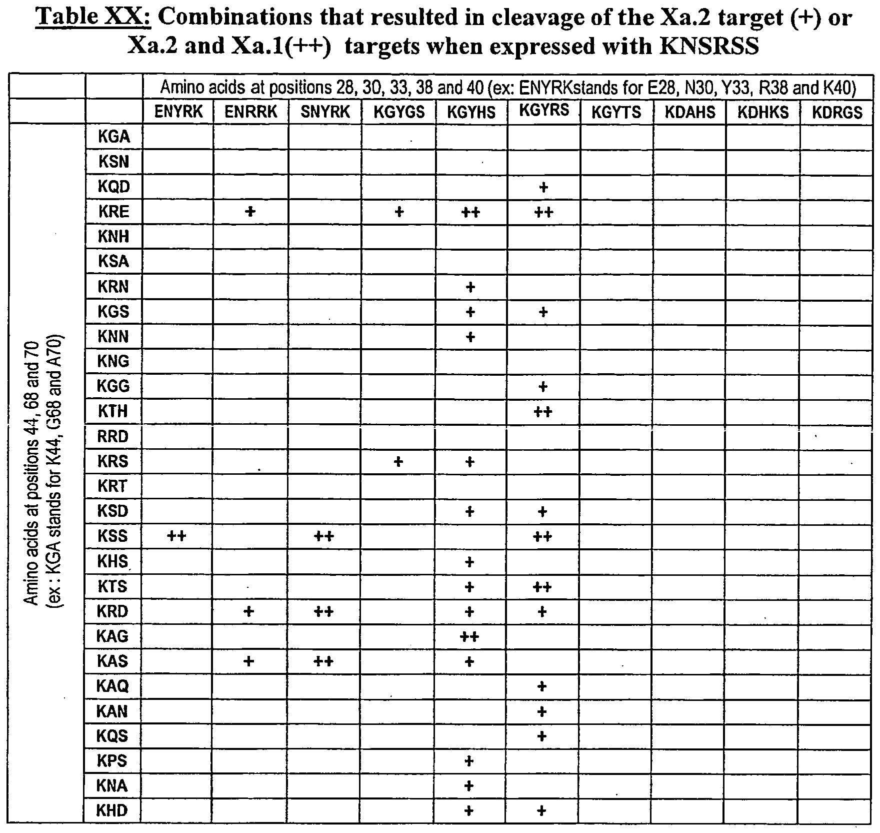

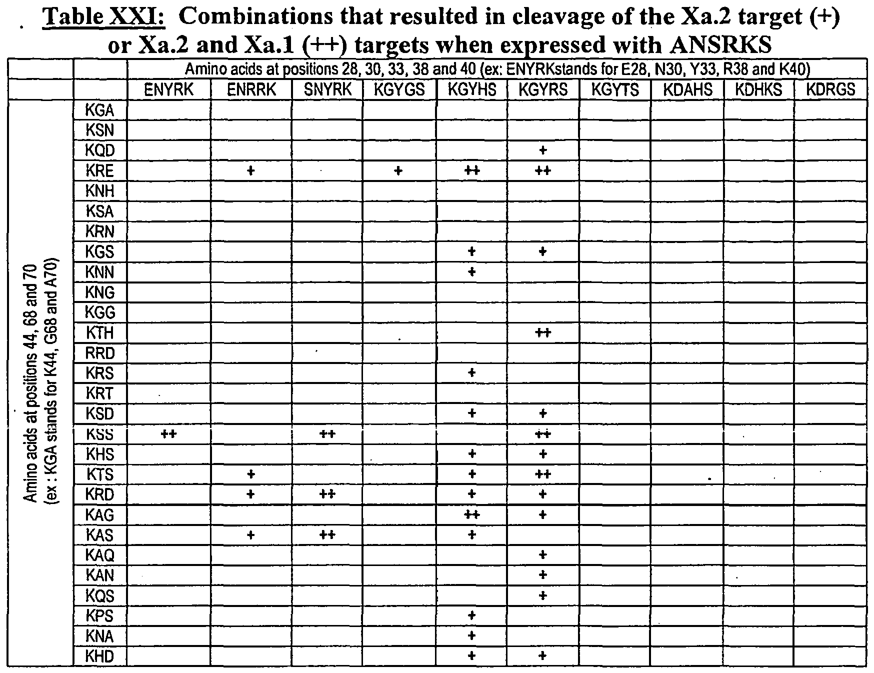

### Example 6: Optimization of the cleavage activity of the meganucleases

### A) Material and methods

### Activity improvment

Error-prone PCR was used to introduce random mutations in a pool of 4 chosen mutants. Libraries were generated by PCR using either Mn²⁺, or by two-steps process using dNTPs derivatives 8-oxo-dGTP and dPTP as described in the protocol from JENA BIOSCIENCE GmbH for the JBS dNTP-Mutagenesis kit. Primers used are: preATGCreFor (5'-gcataaattactatacttctatagacacgcaaacacaaatacacagcggccttgccacc-3', SEQ ID NO: 227) and ICreIpostRev (5'-ggctcgaggagctcgtctagaggatcgctcgagttatcagtcggccgo-3', SEQ ID NO: 228). For the first round of activity improvement, the new libraries were cloned *in vivo* in yeast in the linearized kanamycin vector (pCLS 1107, figure 14) harboring a galactose inducible promoter, a KanR as selectable marker and a 2 micron origin of replication. For the second round of activity improvement, a 2 micron-based replicative vector marked with the *LEU2* gene is used. Positives resulting clones were verified by sequencing (MILLEGEN).

### B) Results

Since, the decrease in the cleavage activity between the target.2 and the target .1 (figure 24B) could be a consequence of a bias towards the 4 central nucleotides gtac introduced during the process of making meganucleases, it was hypothesized that a weak activity could be subsequently improved by compensatory mutations. The figure 25 depicts the general workflow of the strategy, which was used to refine the proteins activity. The process of optimization of the cleavage activity is performed in two steps. First, after identification of the functional heterodimers for the target sequence Xa.1, a pool of 2 to 4 monomers initially identified by screening against Xa.4 target (mutant .4) is mutagenized by error-prone PCR, while its counterpart (e.g. the mutants identified by screening against the Xa.3 target) remain unmodified. The mutagenized library is then transformed in yeast and the screening is performed by mating with a yeast strain containing the target Xa.1 and the non-mutagenized mutant (active on Xa.3 in this case). About 2300 clones are usually tested, since this number of clones was sufficient to find mutants with improved activity. Once the combinations giving enhanced activity are identified, the same procedure is repeated to optimize the mutants interacting with the Xa.3 target (mutant .3). After error-prone PCR, the library is screened against Xa.1 target by mating with yeast containing the target and the improved mutants previously identified. At the end of this procedure, protein heterodimers are obtained, wherein both monomers have been optimized for a defined protein combination versus one target sequence. Finally, the improved variants are validated in a crossed experiment as shown in figure 26A (example of the cleavage activity in yeast of a subset of heterodimers against the target Xa.1 obtained after monomers improvements). Five improved mutants cleaving Xa.4 and 6 improved mutants cleaving Xa.3 were tested in combination with 2 and 8 improved mutants cleaving Xa.3 and Xa.4 respectively, against the Xa.1 target. The original mutants used for the error-prone PCR were incorporated in the experiment as controls. No activity could be detected against the target Xa.1 with homodimers alone as shown by the white colonies obtained after mating of yeast clones containing mutants cleaving only Xa.3 or Xa.4. In contrast, a strong cleavage activity can be visualized when mating occurred between yeast clones containing both mutant species indicating that the cleavage activity resulted from heterodimer formation. Furthermore, the strongest activity is achieved when both monomers have been optimized as compared to the activity achieved with heterodimers for which only one monomer activity has been improved.

The 6 combinations giving the strongest cleavage activity were selected and the ORFs were sequenced. Interestingly, the 6 most active heterodimers resulted from 6 different combinations of 3 independent mutants cleaving Xa.3 with 2 different mutants cleaving Xa.4. The figure 26B shows the sequence of the original proteins used for the improvement process (I1-I4)) and their respective optimized proteins (M1 - M5). The I5 and I6 sequences in figure 26B are the protein sequences of the 2 monomers giving the best activity on Xc.1 target by co-expression assay. As the cleavage of this target was highly efficient, no further activity refinement was needed.

The protein sequence analysis of the best cutters does not reveal any particular protein domains affected by the mutation process. As compared with the original sequence, the error-prone PCR introduced mutations at positions 19, 69 and 87 in the ORF of active mutants cleaving Xa.3 target and mutations at positions 32, 85 and 109 in the coding sequence of the mutants cleaving Xa.4 target. The positions 33 and 38 were also reverted in the protein sequence of the M2 and M5 proteins. Interestingly, the amino acid at position 19 was mutated in all proteins with activity towards the Xa.3 target. This position is part of the catalytic site (Chevalier et al., Biochemistry, 2004, 43, 14015-14026) and with the adjacent Asp20, is involved in the metal cation binding. This is the only mutation which can be directly linked to improvement of the catalytic mechanism. The mutations in positions 32 and 69 affect the protein-DNA interface and the mutations in positions 85 and 87 affect the hydrophobic core. The other mutations affect mainly the core protein indicating that a mechanism of propagated conformational change is responsible for the improved activity. This long range effect could, for example, improve the binding affinity of the mutant and therefore increase its cleavage activity.

These results demonstrate that the combinatorial approach associated to random mutagenesis allows the rapid and efficient production of custom-designed endonucleases for specific DNA substrates.

### Example 7: The heterodimeric meganucleases are functional in mammalian cells

### A) Material and methods

### Mammalian cells assays

CHO cells were transfected with Polyfect® transfection reagent according to the supplier (QIAGEN) protocol. 72 hours after transfection, culture medium was removed and 150 µl of lysis/revelation buffer added for β-galactosidase liquid assay (1 liter of buffer contains 100 ml of lysis buffer (Tris-HCl 10 mM pH7.5, NaCl 150 mM, Triton X100 0.1%, BSA 0.1 mg/ml, protease inhibitors), 10 ml of Mg 100X buffer (MgCl₂ 100 mM, β-mercaptoethanol 35 %), 110 ml ONPG (8 mg/ml) and 780 ml of sodium phosphate 0.1 M pH7.5). After incubation at 37°C, OD was measured at 42 0nm. The entire process is performed on an automated Velocity11 BioCel platform.

### B) Results

The hybrid meganucleases active in yeast were tested in mammalian cells by transient co-transfection of CHO cells with a target vector and meganuclease expression vectors. For this purpose, subsets of mutants and their corresponding targets (Xa.1 and Xc.1) were cloned into appropriate vectors as described in example 1, and the meganuclease-induced recombination efficiency was measured by a standard, quantitative ONPG assay that monitors the restoration of a functional β-galactosidase gene, as described previously (International PCT Application WO 2006/097853; Arnould et al., J. Mol. Biol., 2006, 355, 443-458; Smith et al., Nucleic Acids Res., Epub 27 november 2006). Figure 27 shows the cleavage activity in CHO-K1 cells for the most active hybrid proteins identified in yeast. All target plasmids carry a minimal I-*Sce*I site of 18 bp as an internal control; therefore all mutants can be compared to the I-*Sce*I activity in the same experimental conditions. This 18 bp I-*Sce*I site is weakly cleaved by the I-*Sce*I protein and was chosen in order to avoid saturation of the signal in CHO cells. No cleavage activity could be detected when a single protein is expressed as judged by their background level activity (Figure 27; Ø/ Ø, Ø/I1 to Ø/M3, and Ø/I3 to Ø/M5) indicating that activity is dependent on heterodimer formation. Furthermore, the co-expression of the initial combinatorial mutants (I1, I3) has a very weak activity which is virtually not distinguishable from the background level. The cleavage activity increases as soon as one of the protein partners has been improved. Finally, the most efficient cleavage activities for the Xa.1 target is achieved by co-expression of 2 improved proteins and reaches an activity level similar to the activity of the mutants identified for the Xc.1 target. These results confirm the data obtained in yeast in which co-expression of the initial combinatorial mutants lead to efficient cleavage of the Xc.1 target without the need of improvement activity while the mutants directed towards Xa.1 had poor activity. However a high activity for the Xa.1 target could be generated after introduction of compensatory mutations. When monomers are expressed on their own, neither toxicity nor cleavage, as shown in figure 27, could be detected, showing that the mutants designed towards Xa and Xc keep the original specificity of the I-*Cre*I homing endonuclease or at least a specificity compatible with cell survival.

### Example 8: Analysis of the individually mutated target cleavage pattern

The degeneracy at individual positions of the I-*Cre*I target has been previously assayed using *in vitro* site selection in which variant DNA targets cleavable by wtI-CreI could be recovered (Argast et al., J. Mol. Biol., 1998, 280, 345-353). It indicates that most nucleotide positions in the site can be mutated without loss of binding or cleavage. However no exhaustive study was done. In order to compare the improved mutants towards Xa.1 target with I-*Cre*I homing endonuclease, we have generated all possible targets carrying individual mutation were generated for Xa.3, Xa.4 and the palindromic I-*Cre*I target C1221. The protein scaffold used to generate all the mutants carries a D to N mutation at position 75. This mutation was introduced in order to decrease the energetic strains caused by the replacement of the basic residues at positions 68 and 7.0 in the libraries. It was shown previously that the D75N mutation decreased the toxicity of over-expressed I-*Cre*I protein without affecting the protein basic folding properties and activity (Arnould et al., J.Mol. Biol., 2006, 355, 443-458). The extent of degeneracy of base-pair recognition of wild type I-*Cre*I (wt I-CreI), the initial protein scaffold I-*Cre*I(N75) and the combinatorial mutants was assayed by measuring their cleavage efficiency in yeast on their respective palindromic targets (C1221, Xa.3 and Xa.4) carrying individual site mutations.

### I-CreI D75 (wt) and I-CreI N75

As shown in figure 28A, the wild type protein (I-*Cre*I-D75) can accommodate many individual mutations on its palindromic target (the font size of each nucleotide is proportional to the cleavage efficiency). The positions ± 11, ±8, ±2, and ±1 are the most tolerant positions since none of the 4 nucleotides at these positions affect the cleavage efficiency. In contrast, positions ±9 and ±3 accept very few changes. In comparison, the I-*Cre*I scaffold used to generate the mutants (I-*Cre*I-N75) reveals a different pattern and seems to be less tolerant to point mutations in its target. The most stringent differences are seen at positions ±1, ±3, ±10, and ±11. The only bases allowing target cleavage are ±1T, ±9A and ±10A while G and A at position ±11 inhibit the cleavage of the target by I-*Cre*I(N75) protein. For both wt I-*Cre*I and I-*Cre*I(N75), C or A and T or C at positions ±7 and ±6 respectively, allow maximum cleavage efficiency. Altogether wt I-*Cre*I and I-*Cre*I(N75) cleave respectively 26 and 14 targets carrying single mutation. Based on this data, and if it is assumed that each half target can have an independent impact on the global cleavage efficiency, wt I-*Cre*I should be able to cleave a maximum of 676 (26x26) out of 1156 (34x34) non palindromic targets with individual mutations while I-*Cre*N75 should cut only 196 (14x14). Probably, the restricted pattern of the I-*Cre*(N75) observed in this study could explain the absence of toxicity of this I-*Cre*I variant. As suggested in early studies (Chevalier et al., J. Mol. Biol., 2003, 329, 253-269; Seligman et al., Genetics, 1997, 147, 1353-1664), the tolerance of individual nucleotide polymorphisms allows I-*Cre*I(D75) to recognize a defined population of targets and facilitates the repeated horizontal transmission of the intron during evolution.

### Combinatorial mutants cleaving the Xa.3 and Xa. 4 targets

The figure 28B shows the results obtained with the best combinatorial mutants cleaving the Xa.3 and Xa.4 targets. The mutant cleaving the Xa.4 target is much more permissive than the mutant cleaving the Xa.3 target for individual mutation on their respective targets. The combinatorial Xa.4 mutant is able to cleave 24 palindromic targets of which 20 are strongly cleaved. The pattern of single-base substitutions tolerated by the Xa.4 mutants is very similar to that of wt I-*Cre*I towards its own target. In contrast, custom-designed variant towards Xa.3 targets revealed to have the highest stringency regarding individual mutations as only 3 palindromic targets are highly cleavable. This mutant is capable of cleaving only 8 (of which 4 are efficiently cleaved) out of the 34 palindromic targets. Except at the position ±5 which can tolerate G and T, efficient cleavage is achieved only for the target on which it has been selected. As none of the mutants tested displayed degeneracy greater than the wt I-*Cre*I natural protein, these data provide evidence that the combinatorial approach used to generate the mutants results in a change of substrate specificity instead of a simple relaxation of the protein specificity towards its target. Furthermore, the combinatorial mutants have been checked against the parental sequences and the original I-*Cre*I target sequence and none of the proteins tested cleaves these targets. Altogether, the heterodimer designed to cleave the Xa.1 target should be able to cut a maximum of 192 (8x24) out of the 1156 non palindromic targets with individual mutations.

## Claims

1. An I-*Cre*I variant, **characterized in that** it has at least two substitutions, one in each of the two functional subdomains of the LAGLIDADG core domain situated from positions 28 to 40 and 44 to 70 of 1-*Cre*I amino acid sequence SEQ ID NO: 217, said variant being able to cleave a DNA target sequence selected from the group consisting of the sequences SEQ ID NO: 1 to 24 and 45 to 216 from a human xeroderma pigmentosum gene, and being obtainable by a method comprising at least the steps of:
(a) constructing a first series of I-*Cre*I variants having at least one substitution in positions 28, 30, 32, 33, 38 and/or 40 of a first functional subdomain of the LAGLIDADG core domain situated from positions 28 to 40 of I-*Cre*I,
(b) constructing a second series of I-*Cre*I variants having at least one substitution in positions 44, 68 and/or 70 of a second functional subdomain of the LAGLIDADG core domain situated from positions 44 to 70 of I-*Cre*I,
(c) selecting the variants from the first series of step (a) which are able to cleave a mutant I-*Cre*I site wherein (i) the nucleotide triplet in positions -10 to -8 of the I-*Cre*I site has been replaced with the nucleotide triplet which is present in positions -10 to -8 of a genomic DNA target selected from the group consisting of the sequences SEQ ID NO: 1 to 24 and 45 to 216 from a human xeroderma pigmentosum gene and (ii) the nucleotide triplet in positions +8 to +10 has been replaced with the reverse complementary sequence of the nucleotide triplet which is present in positions -10 to -8 of said genomic target,
(d) selecting the variants from the second series of step (b) which are able to cleave a mutant I-*Cre*I site wherein (i) the nucleotide triplet in positions -5 to -3 of the I-*Cre*I site has been replaced with the nucleotide triplet which is present in positions -5 to -3 of said genomic target in (c) and (ii) the nucleotide triplet in positions +3 to +5 has been replaced with the reverse complementary sequence of the nucleotide triplet which is present in positions -5 to - 3 of said genomic target,
(e) selecting the variants from the first series of step (a) which are able to cleave a mutant I-*Cre*I site wherein (i) the nucleotide triplet in positions +8 to +10 of the I-*Cre*I site has been replaced with the nucleotide triplet which is present in positions +8 to +10 of said genomic target in (c) and (ii) the nucleotide triplet in positions -10 to -8 has been replaced with the reverse complementary sequence of the nucleotide triplet which is present in position +8 to +10 of said genomic target,
(f) selecting the variants from the second series of step (b) which are able to cleave a mutant I-*Cre*I site wherein (i) the nucleotide triplet in positions +3 to +5 of the I-*Cre*I site has been replaced with the nucleotide triplet which is present in positions +3 to +5 of said genomic target in (c) and (ii) the nucleotide triplet in positions -5 to -3 has been replaced with the reverse complementary sequence of the nucleotide triplet which is present in positions +3 to +5 of said genomic target,
(g) combining in a single variant, the mutation(s) in positions 28 to 40 and 44 to 70 of two variants from step (c) and step (d), to obtain a novel homodimeric I-*Cre*I variant which cleaves a sequence wherein (i) the nucleotide triplet in positions -10 to -8 is identical to the nucleotide triplet which is present in positions -10 to -8 of said genomic target in (c), (ii) the nucleotide triplet in positions +8 to +10 is identical to the reverse complementary sequence of the nucleotide triplet which is present in positions -10 to -8 of said genomic target, (iii) the nucleotide triplet in positions -5 to -3 is identical to the nucleotide triplet which is present in positions -5 to -3 of said genomic target and (iv) the nucleotide triplet at positions +3 to +5 is identical to the reverse complementary sequence of the nucleotide triplet which is present at positions -5 to -3 of said genomic target,
(h) combining in a single variant, the mutation(s) in positions 28 to 40 and 44 to 70 of two variants from step (e) and step (f), to obtain a novel homodimeric I-*Cre*I variant which cleaves a sequence wherein (i) the nucleotide triplet in positions +3 to +5 is identical to the nucleotide triplet which is present in positions +3 to +5 of said genomic target in (c), (ii) the nucleotide triplet in positions - 5 to -3 is identical to the reverse complementary sequence of the nucleotide triplet which is present in positions +3 to +5 of said genomic target, (iii) the nucleotide triplet in positions +8 to +10 of the I-*Cre*I site has been replaced with the nucleotide triplet which is present in positions +8 to +10 of said genomic target and (iv) the nucleotide triplet in positions -10 to -8 is identical to the reverse complementary sequence of the nucleotide triplet in positions +8 to +10 of said genomic target,
(i) combining the variants obtained in steps (g) and (h) to form heterodimers, and
(j) selecting the heterodimers from step (i) which are able to cleave said DNA target sequence selected from the group consisting of the sequences SEQ ID NO: 1 to 24 and 45 to 216 from a human xeroderma pigmentosum gene.

2. The variant according to claim 1, wherein said substitutions are replacement of the initial amino acids with amino acids selected in the group consisting of A, D, E, G, H, K, N, P, Q, R, S, T, Y, L and V.

3. The variant according to claim 1 or claim 2, which comprises one or more substitutions in positions: 19, 24, 26, 42, 69, 75, 77, 80, 85, 87, 109, 133 and 161 of I-*Cre*I.

4. The variant according to anyone of claims 1 to 3, which comprises the substitution of the aspartic acid in position 75 in an uncharged amino acid.

5. The variant according to claim 4, wherein said uncharged amino acid is an asparagine or a valine residue.

6. The variant according to anyone of claims 1 to 5, which is obtainable by a method comprising the steps a) to j) as defined in claim 1, and the additional steps of: random mutagenesis on at least one monomer of the heterodimer formed in step (i) or obtained in step (j) and selection and/or screening of the heterodimers having improved activity towards said DNA target from a xeroderma pigmentosum gene in comparison to said initial heterodimer formed in step (i) or obtained in step (j)

7. The variant according to anyone of claims 1 to 6, which is an heterodimer able to cleave a DNA target selected from the group consisting of the sequences SEQ ID NO: 45 to 57 from the human *XPA* gene, wherein the first and the second monomers have amino acids in positions 24, 26, 28, 30, 33, 38, 40, 42, 44, 68, 70, 75, 77 and/or 80 of I-*Cre*I, which are as indicated in Table XXII:
| **First monomer** | **Second monomer** 28R30D38R44K68A70575N771 |
|---|---|
| 28K33S38R40D44K68R70G75N | 28R30D38R44K68A70S75N77I |
| 28K30G38H44Q68R70Q75N | 28K30G38K44Q68R70S75R77T80K |
| 28K33T38A40Q44N68K70S75R77N | 30D33R38G44N68K70S75R77N |
| 28K30G38H44R68Y70S75E77Y | 28K33N38Q40Q44K68R70E75N |
| 28K30N38Q44G68A70N75N | 28Q33Y38R40K42R44Q70S75N77N |
| 30N33H38Q44K68R70E75N | 28K33R3SA40Q44068R70S75N |
| 28K30N38Q44K68H70E75N | 28K33R38A40Q44Q68R70S75N |
| 28K30G38G44Q68R70G75N | 30D33R38G44Q68R70S75N |
| 28K30G38H44Q68R70S75R77T80K | 28K33T38A40Q44Q68R70G75N |
| 30N33H38Q44Q68A70N75N | 24I26Q28K30N33Y38Q40S44K68R70E75N |
| 28K30N38Q44K68A70N75N | 28K33R38A40Q44A68R70G75N |
| 28K33R38E40R44K68S70N75N | 28K30G38H44R68Y70S75E77Y |
| 28K33R38A40Q44N68R70N75N | 30D33R38G44A68N70N75N |

8. The variant according to anyone of claims 1 to 6, which is an heterodimer able to cleave a DNA target selected from the group consisting of the sequences SEQ ID NO: 58 to 86 from the human *XPB* gene, wherein the first and the second monomers have amino acids in positions 24, 26, 28, 30, 33, 38, 40, 42, 44, 68, 70, 75, 77 and/or 80 of I-*Cre*I which are as indicated in Table XXIII:
| **First monomer** | **Second monomer** |
|---|---|
| 30R33G38S44K68S70N75N | 30N33H38Q44Q68R70S75R77T80K |
| 30N33H38A44K68R70E75N | 30D33R38T44K68S70N75N |
| 28K33N38Q40Q44R68R70R75N | 28K33R38A40Q44R68R70R75N |
| 28R30D38Q44E68R70A75N | 28Q33S38R40K44K68T70T75N |
| 30N33H38A44A68R70G75N | 28K33T38A40Q44Q68Y70S75R77Q |
| 30N33H38A44A68N70N75N | 30N33H38Q44R68Y70S75E77Y |
| 28K33S38040Q44R68R70R75N | 28K33R38Q40S44K68R70G75N |
| 28K33T38A40A44Q68R70S75R77T80K | 30N33H38Q44K68Y70S75Q77N |
| 30N33T38A42R44Q70S75N77N | 28R33A38Y40Q44K68R70E75N |
| 30D33R38G44K68A70N75N | 28K30G38H44R68R70R75N |
| 28R33A38Y40Q44068R70G75N | 30N33H38A44A68S70R75N |
| 28Q33Y38Q40K44A68N70N75N | 30D33R38G44Q68R70S75R77T80K |
| 30N33H38Q44K68Y70S75D77T | 20Q33Y38Q40K4468Y70S75D77T |
| 28K33S38Q40Q44Q68R70S75N | 28K30G38H44K68H70E75N |
| 30N33H38A44A68R70S75N | 28K30G38H44A68N70N75N |
| 30D33R38T44K68S70N75N | 30D33R38G44R68Y70S75E77Y |
| 24I26Q28K30N33Y38Q40S44K68H70E75N | 28K30N38Q44K68Y70S75D77T |
| 28K33T38A40A44K68Y70S75Q77N | 30N33H38Q44Q68R70G75N |
| 30N33H38A44N68K70S75R77N | 28R33A38Y40Q44R68Y70S75E77Y |
| 28Q33Y38Q40K44K68T70T75N | 24I26Q28K30N33Y38Q40S44E68R70A75N |
| 24I26Q28K30N33Y38Q40S44Q68R70N75N | 30D33R38T44A68R70S75Y77Y |
| 28K33T38A40Q44Q68R70Q75N | 28K30N38Q44Q68Y70S75R77Q |
| 28K33S38R40D44Y68D70S75R77T | 28K33S38R40D44K68T70T75N |
| 30N33H38A44R68Y70S75E77Y | 28R33A38Y40Q44Q68R70S75R77T80K |
| 28Q33Y38R40K44Q68R70S75R77T80K | 30R33G38S44R68R70R75N |
| 30R33G38S44Q68R70S75N | 28R30D38Q44K68A70N75N |
| 30D33R38T42R44Q70S77N | 30N33T38A44A68R70S75N |
| 30N33T38A44Q68R70S75N | 30N33H38Q44E68R70A75N |
| 30N33H38A44K68A70N75N | 30N33T38Q44A68S70R75N |

9. The variant according to anyone of claims 1 to 6, which is an heterodimer, able to cleave a DNA target selected from the group consisting of the sequences SEQ ID NO: 1 to 24 from the human *XPC* gene, wherein the first and the second monomers have amino acids in positions 28, 30, 33, 38, 40, 42, 44, 68, 70, 75, 77, 80 and/or 133 of I-*Cre*I which are as indicated in Table XXIV:
| **First monomer** | **Second monomer** |
|---|---|
| 30D33R38G44R68Y70S75Y77T | 28K33R38E40R44R68Y70S75E77V |
| 30D33R38T44T68Y70S75R77T | 28K33R38N40Q44N68R70N75N |
| 28K33R38E40R44R68Y70S75E77I | 28Q33Y38R40K42R44070S77N |
| 28Q33S38R40K44Q68Y70S75N77Y | 28T33T38Q40R44T68E70S75R77R |
| 30D33R38T44N68R70S75Q77R | 28R33A38Y40Q44D68Y70S75S77R |
| 28K33R38Q40A44T68R70S75Y77T133V | 28K33R38E40R44K68Y70S75D77T |
| 28K33N38Q40Q44R68Y7OS75E77I | 28K33T38A40Q44K6S070S75N77R |
| 28E33R38R40K44068Y70S75N77Y | 28Q33S38R40K44A68R70S75R77L |
| 28K33T38A40Q44A68R70S75R77L | 28A33T38Q40R44R68S70S75E77R |
| 28K30N38Q44Q68R70S75R77T80K | 28T33T38Q40R44T6SY70S75R77V |
| 28K30N38Q44A68Y70S75Y77K | 28K33R38E40R44T68R70S75Y77T133V |
| 28K33R38Q40A44Q68R70N75N | 28K33R38A40Q44Q68R70S75N77K |
| 30N33H38Q44K68A70S75N77I | 28K33N38Q40Q44R68Y70S75E77V |
| 28Q33S38R40K44N68R70S75R77D | 28T33R38Q40R44Q68R70S75R77T80K |
| 28Q33R38R40K44T68Y70S75R77T | 28Q33Y38R40K44Y68D70S75R77V |
| 28K33T38A40A44T68E70S75R77R | 28K30N38Q44A68N70S75Y77R |
| 28Q33Y38Q40K44Q68R70S75R77T80K | 28K33R3SQ40A44Q68R70N75N |
| 28K33R38A40Q44K68A70S75N77I | 28K33R38E40R44R6SY70S75Y77T |
| 28T33T38Q40R44Q68R70S75D77K | 28E33R38R40K44Q68R70S75R77T80K |
| 28R33A38Y40Q44Q68R70S75R77T80K | 2SQ33Y3SR40K44T68Y70S75R77T |
| 28Q33S38R40K42T44K70S75N77Y | 28R33A38Y40Q44A68R70S75E77R |
| 30D33R38T44A68Y70S75Y77K | 28Q33Y38R40K42R44Q70S75N77N |
| 28K33R38E40R44K68Q70S75N77R | 28Q33S38R40K44R68Y70S75E77V |
| 28K30N33H38Q40S44Q68R70R75N | 28K30N33R38A40Q44K68R70N75N |

10. The variant according to anyone of claims 1 to 6, which is an heterodimer able to cleave a DNA target selected from the group consisting of the sequences SEQ ID NO: 87 to 119 from the human *XPD* gene, wherein the first and the second monomers have amino acids in positions 28, 30, 33, 38, 40, 44, 68, 70, 75, 77 and/or 80 of I-*Cre*I which are as indicated in Table XXV:
| **First monomer** | **Second monomer** |
|---|---|
| 30A33D38H44K68R70E75N | 28K30G38K44K68S70N75N |
| 30N33T38Q44Q68R70G75N | 30N33H38Q44A68R70S75E77R |
| 30N33H38A44N68R70N75N | 30N33H38Q44Q68R70S75R77T80K |
| 28K33R38E40R44E68R70A75N | 28R33A38Y40Q44A68S70R75N |
| 28K33T38R40Q44K68R70E75N | 28Q33S38R40K44K68R70G75N |
| 30D33R38T44K68R70E75N | 28K30G38G44068Y70S75R77Q |
| 30D33R38T44K68R70E75N | 30R33G38S44A68R70S75N |
| 28K30G38H44A68R70N75N | 28K33S38R40D44A68N70N75N |
| 30N33H38Q44Y68D70S75R77T | 28R30D38R44A68N70N75N |
| 28K33R38Q40S44K68T70T75N | 30D33R38G44K68R70E75N |
| 28R33A38Y40Q44Q68R70Q75N | 28K30G38H44R68Y70S75E77Y |
| 33R38E44Q68R70G75N | 28K33R38E40R44K68R70E75N |
| 30R33G38S44K68R70E75N | 30D33R38G44A68R70N75N |
| 30R33G38S44A68R70S75N | 30D33R38G44K68R70G75N |
| 30N33H38A44K68R70G75N | 28Q33Y38R40K44K68A70N75N |
| 30N33T38A44N68R70N75N | 28K30G38K44Q68R70S75N |
| 28R33A38Y40Q44K68R70G75N | 28Q33Y38R40K44T68Y70S75R77V |
| 28Q33Y38R40K44K68Y70S75Q77N | 33T44Q68R70S75N |
| 28K30G38G44A68N70N75N | 28K33R38E40R44E68R70A75N |
| 28K33R38A40Q44R68R70R75N | 28K33R38E40R44T68Y70S75R77V |
| 28K33R38Q40S44K68R70E75N | 30R33G38S44A68R70S75E77R |
| 28Q33Y38R40K44D68R70N75N | 28K33R38A40Q44R68R70R75N |
| 30N33H38A44Q68R70G75N | 28Q33Y38R40K44A68S70R75N |
| 30D33R38T44A68R70N75N | 28K33R38E40R44A68R70S75N |
| 28Q33Y38R40K44E68R70A75N | 33R38E44N68R70N75N |
| 28K30G38H44A68R70S75N | 30N33T38Q44R68Y70S75E77Y |
| 28K33R38E40R44Q68R70G75N | 30N33T38A44R68R70R75N |
| 28Q33Y38Q40K44Q68R70S75R77T80K | 30R33G38S44G68Q70T75N |
| 30N33H38Q44D68R70N75N | 28K30G38K44G68Q70T75N |
| 28K33N38Q40Q44A68N70N75N | 28K30G38H44Q68R70G75N |
| 28K33R38E40R44K68R70E75N | 28K30G38H44A68R70S75N |
| 28Q33S38R40K44K68R70E75N | 28K33T38A40A44A68R70S75N |
| 28K33N38Q40Q44K68T70T75N | 28K30G38H44Q68A70N75N |

11. The variant according to anyone of claims 1 to 6, which is an heterodimer able to cleave a DNA target selected from the group consisting of the sequences SEQ ID NO: 120 to 166 from the human *XPE* gene, wherein the first and the second monomers have amino acids in positions 28, 30, 33, 38, 40, 44, 68, 70, 75, 77, 80 and/or 133 of I-*Cre*I which are as indicated in Table XXVI:
| **First monomer** | **Second monomer** |
|---|---|
| 28K30N38Q44K68R70E75N | 28Q33S38R40K44A68S70R75N |
| 28K30G38H44Q68R70N75N | 28K30G38K44Y68D70S75R77T |
| 28K30N38Q44D68Y70S75S77R | 28R33A38Y40Q44R68Y70S75E77Y |
| 30N33H38Q44Q68R70S75N | 30N33H38A44A68R70S75N |
| 28Q33S38R40K44K68T70G75N | 28K33R38Q40S44A68R70S75E77R |
| 28K30G38H44K68R70E75N | 30N33H38A44K68R70G75N |
| 28R33A38Y40Q44D68R70R75N | 28K33N38Q40Q44Q68R70S75R77T80K |
| 28K33R38E40R44R68R70R75N | 30Q33G38H44A68R70G75N |
| 30D33R38T44K68R70E75N | 28Q33Y38R40K44A68R70S75N |
| 44Q68R70Q75N | 33T44Q68Y70S75R77Q |
| 28K33T38R40Q44A68R70G75N | 28K33R38E40R44Q68R70S75R77T80K |
| 30D33R38G44K68S70N75N | 30D33R38T44A68S70R75N |
| 28Q33Y38Q40K44K68R70E75N | 28K30G38H44A68S70R75N |
| 30N33H38A44D68Y70S75S77R | 28K33R38E40R44Q68R70S75R77T80K |
| 28K33S38Q40Q44T68R70S75Y77T133V | 30N33T38Q44Q68R70S75N |
| 30D33R38T44K68R70E75N | 28Q33Y38R40K44A68S70R75N |
| 30D33R38T44Q68R70S75N | 28K33R38Q40S44K68Y70S75D77T |
| 28R33A38Y40Q44K68T70G75N | 44N68R70R75N |
| 28K33R38E40R44Q68R70G75N | 28K33T38R40Q44Q68Y70S75R77Q |
| 30N33H38Q44R68Y70S75E77Y | 28K33R38Q40S70S75N |
| 28R33A38Y40Q44T68Y70S75R77V | 30R33G38S44A68R70S75Y77Y |
| 28K33R38A40Q44K68R70E75N | 28K30G38H44N68R70R75N |
| 28K33R38Q40S44D68R70N75N | 28K33S38R40D44A68R70G75N |
| 30D33R38G44K68H70E75N | 28K30G38G44A68R70G75N |
| 30N33H38Q44R68R70R75N | 30N33T38A44K68R70E75N |
| 30D33R38T44K68T70G75N | 30R33G38S44Q68R70N75N |
| 28K30G38H44R68Y70S75E77Y | 28R33A38Y40Q44Q68R70S75R77T80K |
| 28R33A38Y40Q44A68R70S75N | 28K30G38H44Q68R70S75R77T80K |
| 30N33H38A44K68A70N75N | 28Q33Y38Q40K44Y68D70S75R77T |
| 28Q33Y38R40K44K68R70E75N | 28K33R38E40R44N68R70R75N |
| 28K33R38A40Q44K68Y70S75Q77N | 28K33S38Q40Q44K68R70E75N |
| 28Q33Y38R40K44D68R70R75N | 30N33H38A44A68R70N75N |
| 30D33R38G44A68N70N75N | 30D33R38T44K68R70E75N |
| 28K33N38Q40Q44D68R70N75N | 30D33R38T44A68R70S75N |
| 30N33H38A44Q68R70S75N | 30Q33G38H44A68N70N75N |
| 28R33A38Y40Q44K68R70E75N | 28K33R38Q40S44E68R70A75N |
| 28R30D38Q44K68R70E75N | 28K33S38R40D70S75N |
| 30A33D38H44K68H70E75N | 28K33T38A40A44N68R70R75N |
| 28K30N38Q44K68A70S75N77I | 28K33R38E40R44A68S70R75N |
| 28Q33Y38Q40K44K68R70E75N | 28Q33Y38Q40K44Q68R70S75R77T80K |
| 30D33R38G44N68R70A75N | 28Q33Y38Q40K44Q68R70S75R77T80K |
| 30N33H38Q44R68R70R75N | 28R33A38Y40Q44A68R70S75N |
| 28K33S38Q40Q44R68Y70S75E77I | 30N33T38A44A68R70S75N |
| 28K30G38K44N68R70N75N | 28K33R38E40R44D68Y70S75S77R |
| 28K33R38A40Q44K68R70E75N | 28K30N38Q44Q68Y70S75R77Q |
| 28K33T38R40Q44K68R70G75N | 28R33A38Y40Q44E68R70A75N |
| 28K33N38Q40Q44Q68Y70S75R77Q | 30D33R38T44Q68R70G75N |

12. The variant according to anyone of claims 1 to 6, which is an heterodimer, able to cleave a DNA target selected from the group consisting of the sequences SEQ ID NO: 167 to 188 from the human *XPF* gene, wherein the first and the second monomers have amino acids in positions 28, 30, 33, 38, 40, 44, 68, 70, 75, 77 and/or 80 of I-*Cre*I which are as indicated in Table XXVII:
| **First monomer** | **Second monomer** |
|---|---|
| 30N33H38Q44T68Y70S75R77V | 30D33R38T44Q68R70G75N |
| 28K33S38R40A44K68S70N75N | 28E33R38R40K44Q68R70G75N |
| 28K33T38A40A44Q68R70N75N | 30D33R38G44Q68R70S75N |
| 28Q33S38R40K44R68Y70S75E77Y | 28K33T38A40Q44T68Y70S75R77T |
| 28R30D38Q44Q68R70G75N | 28R33A38Y40Q44Q68R70S75R77T80K |
| 28K30N38Q44A68R70S75Y77Y | 28K33T38R40Q44A68R70S75Y77Y |
| 28K33T38R40Q44Q68Y70S75R77Q | 28K30N38Q44K68Y70S75Q77N |
| 28K33T38A40Q44Q68R70S75N | 28R30D38Q44A68N70S75Y77R |
| 28K33S38Q40Q44Q68R70S75N | 28K33N38Q40Q44A68R70S75Y77Y |
| 28K33R38A40Q44E68R70A75N | 28K33R38A40Q44Q68R70S75R77T80K |
| 28K33T38A40A42T44K70S75N77Y | 28K33T38A40A44T68Y70S75R77V |
| 30N33T38Q44K68R70E75N | 28T33R38Q40R44Q68R70N75N |
| 28Q33Y38Q40K44Q68R70G75N | 28R30D38Q44T68Y70S75R77V |
| 28K33S38Q40Q44Q68R70S75N | 28K30N38Q44K68R70G75N |
| 28K33T38A40Q44Q68R70G75N | 28Q33Y38R40K44Q68R70R75E77R |
| 28033Y38R40K44Q68R70S75R77T80K | 28A33T38Q40R44Q68R70S75R77T80K |
| 30D33R38T44A68R70G75N | 28K33R38E40R44Q68R70G75N |
| 28Q33S38R40K44R68Y70S75D77N | 30D33R38T44K68R70E75N |
| 28K30G38G44T68Y70S75R77V | 28R33A38Y40Q44K68A70S75N77I |
| 28Q33Y38R40K44D68Y70S75S77R | 30D33R38T44E68R70A75N |
| 30N33T38A44Q68R70G75N | 28K30G38H44R68Y70S75E77Y |
| 28K30G38H44A68N70N75N | 30N33H38A44R68Y70S75E77V |

13. The variant according to anyone of claims 1 to 6, which is an heterodimer able to cleave a DNA target selected from the group consisting of the sequences SEQ ID NO: 189 to 216 from the human *XPG* gene, wherein the first and the second monomers have amino acids in positions 28, 30, 33, 38, 40, 42, 44, 68, 70, 75, 77, 80 and/or 133 of I-*Cre*I which are as indicated in Table XXVIII:
| **First monomer** | **Second monomer** |
|---|---|
| 30D33R38T44K68R70E75N | 30D33R38T44Q68N70R75N |
| 30D33R38T44E68R70A75N | 28T33T38Q40R44Q68Y70S75R770 |
| 30N33Y38044Q68R70S75R77T80K | 28K33T38A40A44K68R70E75N |
| 28T33R38S40R44A68R70S75N | 30N33H38Q44N68R70S75R77D |
| 28K30G38H44Q68R70Q75N | 28T33R38Q40R44A68R70S75N |
| 28K33T38R40Q44N68R70A75N | 28T33R38Q40R44K68R70E75N |
| 28K30G38H44A68Q70N75N | 32T33C44Y68D70S75R77T |
| 28R30D38044T68R70S75Y77T133V | 30N33T38A44Q68R70S75N |
| 28Q33Y38Q40K44A68R70N75N | 28K30N38Q44068R70G75N |
| 28K33R38E40R44Q68R70S75N | 28K33R38Q40A44K68Q70S75N77R |
| 30A33D38H44K68G70T75N | 28K33T38A40Q44N68R70S75R77D |
| 32T33C44N68R70A75N | 28R33A38Y40Q44K68R70E75N |
| 28K30G38H44A68R70D75N | 30N33H38A44Q68R70S75N |
| 30R33G38S44K68T70S75N | 28R33A38Y40Q42T44K70S75N77Y |
| 28R33S38Y40Q44K68T70S75N | 28R33A38Y40Q44N68R70S75R77D |
| 30N33H38Q44Q68R70D75N | 28Q33S38R40K44K68H70E75N |
| 28R33S38Y40Q44Y68E70S75R77V | 28K30N38Q44K68R70E75N |
| 32T33C44A68R70S75N | 28T33T38Q40R44T68Y70S75R77T |
| 30N33H38A44D68Y70S75S77R | 28K33R38E40R44T68Y70S75R77T |
| 28R33A38Y40Q44N68R70N75N | 28R33A38Y40044Q68Y70S75N77Y |
| 30N33Y38Q44Q68S70K75N | 30D33R38Q44A68N70S75Y77R |
| 28K33T38A40Q44E68R70A75N | 28R33A38Y40Q44S68Y70S75Y77V |
| 28A33T38Q40R44E68R70A75N | 28K30G38G44A68N70N75N |
| 28K33R38A40Q44N68R70N75N | 28K33S38R40D42T44K70S75N77Y |
| 32T33C44Q68R70D75N | 28K33T38A40A44A68R70N75N |
| 32T33C44K68A70S75N | 30D33R38G44N68R70N75N |
| 28Q33Y38R40K44A68R70S75N | 30D33R38T42T44K70S75N77Y |
| 30D33R38T44K68G70T75N | 30D33R38G44A68D70K75N |

14. The variant according to anyone of claims 1 to 6, which is an heterodimer able to cleave a DNA target selected from the group consisting of the sequences SEQ ID NO: 1 to 24 from the human *XPC* gene, wherein the first monomer has amino acids in positions 19, 28, 30, 33, 38, 40, 69, 70, 75 and/or 87 of I-*Cre*I which are selected from the group consisting of:
28K30N33S38R40S70S75N, 28A30N33S38R40K70S75N, 19A28A30N33S38R40K70S75N, 19A28A30N33Y38R40K70S75N87L, and 19A28A30N33S38R40K69G70S75N, and the second monomer has amino acids in positions 28, 30, 33, 38, 40, 44, 68, 70, 75, 85, 109 and/or 161 of I-*Cre*I which are a selected from the group consisting of : 28E30N33Y38R40K44K68S70S75N, , 28E30N33Y38R40K44K68R70E75N85R109T, 28E30N33Y38R40K44K68R70E75N85R109T161F 28E30N32R33Y38Q40K44K68R70E75N85R109T, 28S30N33Y38R40K44K68S70S75N, 28S30N33Y38R40K44K68R70D75N, 28S30N33Y38R40K44K68A70S75N, 28K30G33Y38H40S44K68R70E75N, 28K30G33Y38H40S44K68A70G75N, 28K30G33Y38R40S44K68R70E75N, 28K30G33Y38R40S44K68T70H75N, 28K30G33Y38R40S44K68S70S75N, and 28K30G33Y38R40S44K68T70S75N.

15. A single-chain chimeric meganuclease comprising two monomers or core domains of one or two I-*Cre*I variants of anyone of claims 1 to 14, or a combination of both.

16. A polynucleotide fragment encoding a variant of anyone of claims 1 to 14 or a single-chain chimeric meganuclease of claim 15.

17. An expression vector comprising at least one polynucleotide fragment of claim 16.

18. The expression vector according to claim 17, which comprises two different polynucleotide fragments, each encoding one of the monomers of an heterodimeric variant as defined in anyone of claims 7 to 14.

19. The vector according to claim 18, which includes a targeting construct comprising a DNA sequence to be introduced in a human xeroderma pigmentosum gene flanked by at least 50 bp of DNA sequences having at least 95 % identity with the region of the human xeroderma pigmentosum gene flanking one of the target sequences SEQ ID NO: 1 to 24 and 45 to 216.

20. The vector according to claim 19, wherein said sequence to be introduced is a sequence which repairs a mutation in a xeroderma pigmentosum gene.

21. The vector according to claim 20, wherein the sequence which repairs said mutation is the correct sequence of said xeroderma pigmentosum gene.

22. The vector according to claim 20, wherein the sequence which repairs said mutation comprises the exons of said xeroderma pigmentosum gene downstream of the genomic cleavage site of the variant, fused in frame, and a polyadenylation site to stop transcription in 3'.

23. The vector according to anyone of claims 19 to 22, wherein said targeting construct comprises a *XPA* gene fragment which is able to repair a cleavage in the *XPA* gene and which comprises at least the nucleotides from positions 34 to 233, 201 to 400, 3493 to 3692, 7627 to 7826, 9994 to 10193, 10151 to 10350 12513 to 12712, 12531 to 12730, 21679 to 21878, 21844 to 22043, 21955 to 22154, 22228 to 22427, or 22234 to 22433 of said *XPA* gene.

24. The vector according to anyone of claims 19 to 22, wherein said targeting construct comprises a *XPB* gene fragment which is able to repair a cleavage in the *XPB* gene and which comprises at least the nucleotides from positions: - 40 to 159, 357 to 556, 1335 to 1534, 1336 to 1535, 1457 to 1656, 3624 to 3823, 4108 to 4307, 5015 to 5214, 5148 to 5347, 5284 to 5483, 5420 to 5619, 7377 to 7576, 13517 to 13716, 13552 to 13751, 13633 to 13832, 14697 to 14896, 14760 to 14959, 14881 to 15080, 21139 to 21338, 21207 to 21406, 22796 to 22995, 32378 to 32577, 33003 to 33202, 34481 to 34680, 34869 to 35068, 34891 to 35090, 36584 to 36783, 36634 to 36833, or 36639 to 36838 of said *XPB* gene.

25. The vector according to anyone of claims 19 to 22, wherein said targeting construct comprises a *XPC* gene fragment which is able to repair a cleavage in the *XPC* gene and which comprises at least the nucleotides from positions: 105 to 304, 5704 to 5903, 7973 to 8172, 9887 to 10086, 10173 to 10372, 11263 to 11462, 13051 to 13250, 13432 to 13631, 18619 to 18818, 19580 to 19779, 20303 to 20502, 20349 to 20548, 20389 to 20588, 21985 to 22184, 21990 to 22189, 22028 to 22227, 22102 to 22301, 26017 to 26216, 29566 to 29765, 29726 to 29925, 30416 to 30615, 31166 to 31365, or 32317 to 32516 of said *XPC* gene.

26. The vector according to anyone of claims 19 to 22, wherein said targeting construct comprises a *XPD* gene fragment which is able to repair a cleavage in the *XPD* gene and which comprises at least the nucleotides from positions: -87 to 112, 812 to 1011, 1319 to 1518, 1324 to 1523, 1426 to 1625, 1717 to 1916, 1867 to 2066, 5473 to 5672, 5585 to 5784, 5637 to 5836, 5920 to 6119, 6050 to 6249, 6290 to 6489, 6392 to 6591, 6472 to 6671, 6581 to 6780, 8830 to 9029, 8943 to 9142, 12661 to 12860, 12991 to 13190, 13084 to 13283, 14614 to 14813, 14817 to 15016, 15528 to 15727, 15878 to 16077, 15936 to 16135, 17023 to 17222, 17350 to 17549, 17365 to 17564, 17572 to 17771, 18347 to 18546, 18370 to 18569, or 18641 to 18840 of said *XPD* gene.

27. The vector according to anyone of claims 19 to 22, wherein said targeting construct comprises *XPE* gene fragment which is able to repair a cleavage in the *XPE* gene and which comprises at least the nucleotides from positions: 10-209, 1295-1494, 2899-3098, 3488-3687, 3616-3815, 6093-6292, 6194-6393, 7034-7233, 7653-7852, 8753-8952, 9781-9980, 9966-10165, 10511-10710, 10665-10864, 11534-11733, 16439-16638, 16667-16866, 18268-18647, 18757-18956, 18863-19062, 19179-19378, 19266-19465, 19596-19795, 20714-20913, 20938-21137, 21099-21298, 22568-22767, 22732-22931, 23173-23372, 23181-23380, 23954-24153, 24000-24199, 29205-29404, 29651-29850, 30280-30479, 30355-30554, 30661-30860, 30685-30884, 32150-32349, 32753-32592, 32770-32969, 32811-33010, 32836-33035, 32841-33040, 33230-33429, 33369-33568, or 33512-33711 of said *XPE* gene.

28. The vector according to anyone of claims 19 to 22, wherein said targeting construct comprises a *XPF* gene fragment which is able to repair a cleavage in the *XPF* gene and which comprises at least the nucleotides from positions: 244-443, 1731-1930, 6429-6628, 6486-6685, 7918-8117, 10500-10699, 10676-10875, 11885-12084, 11886-12085, 12176-12375, 14073-14272, 14110-14309, 15336-15535, 15431-15630, 15574-15773, 17673-17872, 17677-17876, 24486, 24685, 27496-27695, 27822-28021, 27827-28026, or 27963-28162 of said *XPF* gene.

29. The vector according to anyone of claims 19 to 22, wherein said targeting construct comprises a *XPG* gene fragment which is able to repair a cleavage in the *XPG* gene and which comprises at least the positions: 158-357, 5956-6155, 7961-7890, 8044-8243, 9977-10176, 12201-12400, 12289-12488, 15230-15429, 15803-16002, 16041-16240, 16146-16345, 16174-16373, 16174-16373, 16394-16593, 16553-16732, 16887-17086, 19487-19686, 19727-19926, 19782-19981, 20207-20406, 20500-20699, 21890-22089, 22117-22316, 25876-26075, 26450-26649, 26832-27031, 27258-27457, 29180-29379, or 29456-29655 of said *XPG* gene.

30. The vector according to claims 20 or 22, wherein the sequence which repairs said mutation is flanked by a xeroderma pigmentosum gene fragment as defined in anyone of claims 23 to 29.

31. The vector according to claim 20 or 21, wherein the sequence which repairs said mutation is included in said xeroderma pigmentosum gene fragment as defined in anyone of claims 23 to 29.

32. A composition comprising at least one variant according to anyone of claims 1 to 14, one single-chain chimeric meganuclease according to claim 15, and/or at least one expression vector according to anyone of claims 17 to 31.

33. The composition according to claim 32, which comprises a targeting DNA construct comprising a sequence which repairs a mutation in a human xeroderma pigmentosum gene, flanked by sequences homologous to the region of said gene surrounding one of the target sequences SEQ ID NO: 1 to 24 and 45 to 216.

34. The composition according to claim 33, wherein said targeting DNA construct is included in a recombinant vector.

35. Products containing an expression vector according to claim 17 or 18 and a vector which includes a targeting construct as defined in anyone of claims 19 to 31, as a combined preparation for simultaneous, separate or sequential use for preventing, improving or curing a disease associated with xeroderma pigmentosum in an individual in need thereof

36. The use of at least one variant according to anyone of claims 1 to 14, one single-chain chimeric meganuclease according to claim 15, and/or one expression vector according to anyone of claims 19 to 31, for the preparation of a medicament for preventing, improving or curing a disease associated with Xeroderma pigmentosum in an individual in need thereof.

37. A host cell comprising a polynucleotide according to claim 16 or a vector according to anyone of claims 19 to 31.

38. A non-human transgenic animal comprising one or two polynucleotide fragments as defined in claim 16 or claim 18.

39. A transgenic plant comprising one or two polynucleotide fragments as defined in claim 16 or claim 18.

40. Use of at least one variant according to anyone of claims 1 to 14, one single-chain chimeric meganuclease according to claim 15, one vector according to anyone of claims 17 to 31, for genome engineering, for non-therapeutic purposes.

41. The use according to claim 40, wherein said variant, single-chain chimeric meganuclease, vector is associated with a targeting DNA construct as defined in anyone of claims 19 to 31.

## Patentansprüche

1. I-*Cre*I-Variante, **dadurch gekennzeichnet, dass** sie zumindest zwei Substitutionen aufweist, wobei sich eine in jeder der beiden funktionellen Subdomänen der LAGLIDADG-Kemdomäne an den Positionen 28 bis 40 und 44 bis70 der I-*Cre*I-Aminosäuresequenz SEQ ID NO: 217 befindet, wobei die Variante eine DNA-Targetsequenz spalten kann, die aus der Gruppe bestehend aus den Sequenzen SEQ ID NO: 1 bis 24 und 45 bis 216 von dem menschlichen Xeroderma-pigmentosum-Gen ausgewählt ist, und durch ein Verfahren erhalten werden kann, das zumindest folgende Schritte beinhaltet:
(a) Konstruieren einer Reihe von I-*Cre*I-Varianten, die zumindest eine Substitution in den Positionen 28, 30, 32, 33, 38 und/oder 40 von einer ersten funktionellen Subdomäne der LAGLIDADG-Kerndomäne an den Positionen 28 bis 40 von *I-CreI* aufweist,
(b) Konstruieren einer zweiten Reihe von I-*Cre*I-Varianten, die zumindest eine Substitution in den Positionen 44, 68 und/oder 70 von einer zweiten funktionellen Subdomäne der LAGLIDADG-Kemdomäne an den Positionen 44 bis 70 des I-*Cre*I aufweist,
(c) Auswählen der Varianten aus der ersten Reihe von Schritt (a), die eine Mutanten-I-*Cre*I-Stelle spalten können, wobei (i) das Nukleotid-Triplett in den Positionen -10 bis -8 der I-*Cre*I-Stelle durch das Nukleotid-Triplett ersetzt worden ist, das in den Positionen -10 bis -8 eines genomischen DNA-Targets, das aus der Gruppe bestehend aus den Sequenzen SEQ ID NO: 1 bis 24 und 45 bis 216 von einem menschlichen Xeroderma-pigmentosum-Gen ausgewählt ist, vorhanden ist, und (ii) das Nukleotid-Triplett in den Positionen +8 bis +10 durch die umgekehrte Komplementärsequenz des Nukleotid-Tripletts, das in den Positionen -10 bis -8 des genomischen Targets vorhanden ist, ersetzt worden ist,
(d) Auswählen der Varianten aus der zweiten Reihe von Schritt (b), die eine Mutanten-I-*Cre*I-Stelle zu spalten, wobei (i) das Nukleotid-Triplett in den Positionen -5 bis -3 der I-*Cre*I-Stelle durch das Nukleotid-Triplett, das in den Positionen -5 bis -3 des genomischen Targets in (c) vorhanden ist, ersetzt worden ist, und (ii) das Nukleotid-Triplett in den Positionen +3 bis +5 durch die umgekehrte Komplementärsequenz des in den Positionen -5 bis -3 des genomischen Targets vorhandenen Nukleotid-Tripletts ersetzt worden ist,
(e) Auswählen der Varianten aus der ersten Reihe von Schritt (a), die eine Mutanten-I-*Cre*I-Stelle spalten können, wobei (i) das Nukleotid-Triplett in den Positionen +8 bis +10 der I-*Cre*I-stelle durch das Nukleotid-Triplett ersetzt worden ist, das in den Positionen +8 bis +10 des genomischen Targets in (c) vorhanden ist, und (ii) das Nukleotid-Triplett in den Positionen -10 bis -8 durch die umgekehrte Komplementärsequenz des in der Position +8 bis +10 des genomischen Targets vorhandenen Nukleotid-Tripletts ersetzt worden ist,
(f) Auswählen der Varianten aus der zweiten Reihe von Schritt (b), die eine Mutanten-I-*Cre*I-Stellle spalten können, wobei (i) das Nukleotid-Triplett in den Positionen +3 bis +5 der I-*Cre*I-Stelle durch das Nukleotid-Triplett ersetzt worden ist, das in den Positionen +3 bis +5 des genomischen Targets in (c) vorhanden ist, und das Nukleotid-Triplett in den Positionen -5 bis -3 durch die umgekehrte Komplementärsequenz des Nukleotid-Tripletts ersetzt worden ist, das in den Positionen +3 bis +5 des genomischen Targets vorhanden ist,
(g) Kombinieren, in einer einzigen Variante, der Mutation(en) in den Positionen 28 bis 40 und 44 bis 70 der beiden Varianten von Schritt (c) und Schritt (d), um eine neuartige homodimere I-*Cre*I-Variante zu erhalten, die eine Sequenz spaltet, wobei (i) das Nukleotid-Triplett in den Positionen -10 bis -8 mit dem Nukleotid-Triplett identisch ist, das in den Positionen -10 bis -8 des genomischen Target in (c) vorhanden ist, (ii) das Nukleotid-Triplett in den Positionen +8 bis +10 mit der umgekehrten Komplementärsequenz des Nukleotid-Tripletts identisch ist, das in den Positionen -10 bis -8 des genomischen Targets vorhanden ist, (iii) das Nukleotid-Triplett in den Positionen -5 bis -3 mit dem Nukleotid-Triplett identisch ist, das in den Positionen -5 bis -3 des genomischen Targets vorhanden ist, und (iv) das Nukleotid-Triplett an den Positionen +3 bis +5 mit der umgekehrten Komplementärsequenz des Nukleotid-Triplett identisch ist, das an den Positionen -5 bis -3 des genomischen Targets vorhanden ist,
(h) Kombinieren, in einer einzigen Variante, der Mutation(en) an den Positionen 28 bis 40 und 44 bis 70 der beiden Varianten von Schritt (e) und Schritt (f), um eine neuartige homodimere I-*Cre*I-Variante zu erhalten, die eine Sequenz spaltet, wobei (i) das Nukleotid-Triplett in den Positionen +3 bis +5 mit dem Nukleotid-Triplett identisch ist, das in den Positionen +3 bis +5 des genomischen Targets in (c) vorhanden ist, (ii) das Nukleotid-Triplett in den Positionen -5 bis -3 mit der umgekehrten Komplementärsequenz des Nukleotid-Tripletts identisch ist, das in den Positionen +3 bis +5 des genomischen Targets vorhanden ist, (iii) das Nukleotid-Triplett in den Positionen +8 bis +10 der I-CreI-Stelle durch das Nukleotid-Triplett ersetzt worden ist, das in den Positionen +8 bis +10 des genomischen Targets vorhanden ist, und (iv) das Nukleotid-Triplett in den Positionen -10 bis -8 mit der umgekehrten Komplementärsequenz des Nukleotid-Tripletts in den Positionen +8 bis +10 des genomischen Targets identisch ist,
(i) Kombinieren der in den Schritten (g) und (h) erhaltenen Varianten, um Heterodimere zu bilden, und
(j) Auswählen der Heterodimere aus Schritt (i), die die DNA-Targetsequenz spalten kann, die aus der Gruppe bestehend aus den Sequenzen SEQ ID NO: 1 bis 24 und 45 bis 216 von einem menschlichen Xeroderma-pigmentosum-Gen ausgewählt ist.

2. Variante nach Anspruch 1, wobei die Substitutionen einen Ersatz der anfänglichen Aminosäuren durch die in der Gruppe bestehend aus A, D, E, G, H, K, N, P, Q, R, S, T, Y, L und V ausgewählten Aminosäuren darstellen.

3. Variante nach Anspruch 1 oder 2, die eine oder mehrere Substitutionen in den Positionen: 19, 24, 26, 42, 69, 75, 77, 80, 85, 87, 109, 133 und 161 von I-*Cre*I aufweist.

4. Variante nach einem der Ansprüche 1 bis 3, die die Substitution der Asparaginsäure in der Position 75 in einer ungeladenen Aminosäure aufweist.

5. Variante nach Anspruch 4, wobei die ungeladene Aminosäure ein Asparagin- oder ein Valinrest ist.

6. Variante nach einem der Ansprüche 1 bis 5, die durch ein Verfahren erhalten werden kann, das die wie in Anspruch 1 definierten Schritte a) bis j) sowie die folgenden zusätzlichen Schritte beinhaltet: willkürliche Mutagenese auf zumindest einem Monomer des in Schritt (i) gebildeten oder in Schritt (j) erhaltenen Heterodimers und Auswahl und/oder Screening der Heterodimere mit einer verbesserten Aktivität in Bezug auf das DNA-Target aus einem Xeroderma-pigmentosum-Gen im Vergleich zu dem anfänglichen Heterodimer, das in Schritt (i) gebildet oder in Schritt (j) erhalten wird.

7. Variante nach einem der Ansprüche 1 bis 6, bei der es sich um ein Heterodimer handelt, das ein DNA-Target spalten kann, das aus der Gruppe bestehend aus den Sequenzen SEQ-ID NO: 45 bis 57 von dem menschlichen *XPA*-Gen ausgewählt ist, wobei das erste und das zweite Monomer Aminosäuren in den Positionen 24, 26, 28, 30, 33, 38, 40, 42, 44, 68, 70, 75, 77 und/oder 80 von I-*Cre*I aufweisen, wie sie in der Tabelle XXII angegeben sind:
| Erstes Monomer | Zweites Monomer |
|---|---|
| 28K33S38R40D44K68R70G75N | 28R30D38R44K68A70S75N77I |
| 28K30G38H44Q68R70Q75N | 28K30G38K44Q68R70S75R77T80K |
| 28K33T38A40Q44N68K70S75R77N | 30D33R38G44N68K70S75R77N |
| 28K30G38H44R68Y70S75E77Y | 28K33N38Q40Q44K68R70E75N |
| 28K30N38Q44Q68A70N75N | 28Q33Y38R40K42R44Q70S75N77N |
| 30N33H38Q44K68R70E75N | 28K33R38A40Q44Q68R70S75N |
| 28K30N38Q44K68H70E75N | 28K33R38A40Q44Q68R70S75N |
| 28K30G38G44Q68R70G75N | 30D33R38G44Q68R70S75N |
| 28K30G38H44Q68R70S75R77T80K | 28K33T38A40Q44Q68R70G75N |
| 30N33H38Q44Q68A70N75N | 24I26Q28K30N33Y38Q40S44K68R70E75N |
| 28K30N38Q44K68A70N75N | 28K33R38A40Q44A68R70G75N |
| 28K33R38E40R44K68S70N75N | 28K30G38H44R68Y70S75E77Y |
| 28K33R38A40Q44N68R70N75N | 30D33R38G44A68N70N75N |

8. Variante nach einem der Ansprüche 1 bis 6, bei der es sich um ein Heterodimer handelt, das ein DNA-Target spalten kann, das aus der Gruppe bestehend aus den Sequenzen SEQ ID NO: 58 bis 86 von dem menschlichen XPB-Gen ausgewählt ist, wobei das erste und zweite Monomer Aminosäuren in den Positionen 24, 26, 28, 30, 33, 38, 40, 42, 44, 68, 70, 75, 77 und/oder 80 von I-*Cre*I aufweisen, wie sie in der Tabelle XXIII angegeben sind:
| Erstes Monomer | Zweites Monomer |
|---|---|
| 30R33G38S44K68S70N75N | 30N33H38Q44Q68R70S75R77T80K |
| 30N33H38A44K68R70E75N | 30D33R38T44K68S70N75N |
| 28K33N38Q40Q44R68R70R75N | 28K33R38A40Q44R68R70R75N |
| 28R30D38Q44E68R70A75N | 28Q33S38R40K44K68T70T75N |
| 30N33H38A44A68R70G75N | 28K33T38A40Q44Q68Y70S75R77Q |
| 30N33H38A44A68N70N75N | 30N33H38Q44R68Y70S75E77Y |
| 28K33S38Q40Q44R68R70R75N | 28K33R38Q40S44K68R70G75N |
| 28K33T38A40A44Q68R70S75R77T80K | 30N33H38Q44K68Y70S75Q77N |
| 30N33T38A42R44Q70S75N77N | 28R33A38Y40Q44K68R70E75N |
| 30D33R38G44K68A70N75N | 28K30G38H44R68R70R75N |
| 28R33A38Y40Q44Q68R70G75N | 30N33H38A44A68S70R75N |
| 28Q33Y38Q40K44A68N70N75N | 30D33R38G44Q68R70S75R77T80K |
| 30N33H38Q44K68Y70S75D77T | 23Q33Y38Q40K44K68Y70S75D77T |
| 28K33S38Q40Q44Q68R70S75N | 28K30G38H44K68H70E75N |
| 30N33H38A44A68R70S75N | 28K30G38H44A68N70N75N |
| 30D33R38T44K68S70N75N | 30D33R38G44R68Y70S75E77Y |
| 24I26Q2SK30N33Y38Q40S44K68H70E75N | 28K30N38Q44K68Y70S75D77T |
| 28K33T38A40A44K68Y70S75Q77N | 30N33H38Q44Q68R70G75N |
| 30N33H38A44N68K70S75R77N | 28R33A38Y44Q44R68Y70S75E77Y |
| 28Q33Y38Q40K44K68T70T75N | 24I26Q28K30N33Y38Q40S44E68R70A75N |
| 24I26Q28K30N33Y38Q40S44Q68R70N75N | 30D33R38T44A68R70S75Y77Y |
| 28K33T38A40Q44Q68R70Q75N | 28K30N38Q44Q68Y70S75R77Q |
| 28K33S38R40D44Y68D70S75R77T | 28K33S38R40D44K68T70T75N |
| 30N33H38A44R68Y70S75E77Y | 28R33A38Y40Q44Q68R70S75R77T80K |
| 28Q33Y38R40K44Q68R70S75R77T80K | 30R33G38S44R68R70R75N |
| 30R33G38S44Q68R70S75N | 28R30D38Q44K68A74N75N |
| 30D33R38T42R44Q70S77N | 30N33T38A44A68R70S75N |
| 30N33T38A44Q68R70S75N | 30N33H38Q44E68R70A75N |
| 30N33H38A44K68A70N75N | 30N33T38Q44A68S70R75N |

9. Variante nach einem der Ansprüche 1 bis 6, bei der es sich um ein Heterodimer handelt, das ein DNA-Target spalten kann, das aus der Gruppe bestehend aus den Sequenzen SEQ ID NO: 1 bis 24 von dem menschlichen *XPC*-Gen ausgewählt ist, wobei das erste und das zweite Monomer Aminosäuren in den Positionen 28, 30, 33, 38, 40, 42, 44, 68, 70, 75, 77, 80 und/oder 133 von I-*Cre*I aufweisen, wie sie in der Tabelle XXIV angegeben sind:
| Erstes Monomer | Zweites Monomer |
|---|---|
| 30D33R38G44R68Y70S75Y77T | 28K33R38E40R44R68Y70S75E77V |
| 30D33R38T44T68Y70S75R77T | 28K33R38N40Q44N68R70N75N |
| 28K33R38E40R44R68Y70S75E77I | 28Q33Y38R40K42R44Q70S77N |
| 28Q33S38R40K44Q68Y70S75N77Y | 28T33T38Q40R44T68E70S75R77R |
| 30D33R38T44N68R70S75Q77R | 28R33A38Y40Q44D68Y70S75S77R |
| 28K33R38Q40A44T68R70S75Y77T133V | 28K33R38E40R44K68Y70S75D77T |
| 28K33N38Q40Q44R68Y70S75E77I | 28K33T38A40Q44K68Q70S75N77R |
| 28E33R38R40K44Q68Y70S75N77Y | 28Q33S38R40K44A68R70S75R77L |
| 28K33T38A40Q44A68R70S75R77L | 28A33T38Q40R44R68S70S75E77R |
| 28K30N38Q44Q68R70S75R77T80K | 28T33T38Q40R44T68Y70S75R77V |
| 28K30N38Q44A68Y70S75Y77K | 28K33R38E40R44T66R70S75Y77T133V |
| 28K33R38Q40A44Q68R70N75N | 28K33R38A40Q44Q68R70S75N77K |
| 30N33H38Q44K68A70S75N77I | 28K33N38Q40Q44R68Y70S75E77V |
| 28Q33S38R40K44N68R70S75R77D | 28T33R38Q40R44Q68R70S75R77T80K |
| 28Q33R38R40K44T68Y70S75R77T | 28Q33Y38R40K44Y68D70S75R77V |
| 28K33T38A40A44T68E70S75R77R | 28K30N38Q44A68N70S75Y77R |
| 28Q33Y38Q40K44Q68R70S75R77T80K | 28K33R38Q40A44Q68R70N75N |
| 28K33R38A40Q44K68A70S75N77I | 28K33R38E40R44R68Y70S75Y77T |
| 28T33T38Q40R44Q68R70S75D77K | 28E33R38R40K44Q68R70S75R77T80K |
| 28R33A38Y40Q44Q68R70S75R77T80K | 28Q33Y38R40K44T68Y70S75R77T |
| 28Q33S38R40K42T44K70S75N77Y | 28R33A38Y40Q44A68R70S75E77R |
| 30D33R38T44A68Y70S75Y77K | 28Q33Y38R40K42R44Q70S75N77N |
| 28K33R38E40R44K68Q70S75N77R | 28Q33S38R40K44R68Y70S75E77V |
| 28K30N33H38Q40S44Q68R70R75N | 28K30N33R38A40Q44K68R70N75N |

10. Variante nach einem der Ansprüche 1 bis 6, bei der es sich um ein Heterodimer handelt, das ein DNA-Target spalten kann, das aus der Gruppe bestehend aus den Sequenzen SEQ ID NO: 87 bis 119 von dem menschlichen *XPD*-Gen ausgewählt ist, wobei das erste und das zweite Monomer Aminosäuren in den Positionen 28, 30, 33, 38, 40, 44, 68, 70, 75, 77 und/oder 80 von I-*Cre*I aufweisen, wie sie in der Tabelle XXV angegeben sind:
| Erstes Monomer | Zweites Monomer |
|---|---|
| 30A33D38H44K68R70E75N | 28K30G38K44K68S70N75N |
| 30N33T38Q44Q68R70G75N | 30N33H38Q44A68R70S75E77R |
| 30N33H38A44N68R70N75N | 30N33H38Q44Q68R70S75R77T80K |
| 28K33R38E40R44E68R70A75N | 28R33A38Y40Q44A68S70R75N |
| 28K33T38R40Q44K68R70E75N | 28Q33S38R40K44K68R70G75N |
| 30D33R38T44K68R70E75N | 28K30G38G44Q68Y70S75R77Q |
| 30D33R38T44K68R70E75N | 30R33G38S44A68R70S75N |
| 28K30G38H44A68R70N75N | 28K33S38R40D44A68N70N75N |
| 30N33H38Q44Y68D70S75R77T | 28R30D38R44A68N70N75N |
| 28K33R38Q40S44K68T70T75N | 30D33R38G44K68R70E75N |
| 28R33A38Y40Q44Q68R70Q75N | 28K30G38H44R68Y70S75E77Y |
| 33R38E44Q68R70G75N | 28K33R38E40R44K68R70E75N |
| 30R33G38S44K68R70E7SN | 30D33R38G44AS8R70N75N |
| 30R33G38S44A68R70S75N | 30D33R38G44K68R70G75N |
| 30N33H38A44K68R70G75N | 28Q33Y38R40K44K68A70N75N |
| 30N33T38A44N68R70N75N | 28K30G38K44Q68R70S75N |
| 28R33A38Y40Q44K68R70G75N | 28Q33Y38R40K44T68Y70S75R77V |
| 28Q33Y38R40K44K68Y70S75Q77N | 33T44Q68R70S75N |
| 28K30G38G44A68N70N75N | 28K33R38E40R44E68R70A75N |
| 28K33R38A40Q44R68R70R75N | 28K33R38E40R44T68Y70S75R77V |
| 28K33R38Q40S44K68R70E75N | 30R33G38S44A68R70S75E77R |
| 28Q33Y38R40K44D68R70N75N | 28K33R38A40Q44R68R70R75N |
| 30N33H38A44Q68R70G75N | 28Q33Y38R40K44A68S70R75N |
| 30D33R38T44A68R70N75N | 28K33R38E40R44A68R70S75N |
| 28Q33Y38R40K44E68R70A75N | 33R38E44N68R70N75N |
| 28K30G38H44A68R70S75N | 30N33T38Q44R68Y70S75E77Y |
| 28K33R38E40R44Q68R70G75N | 30N33T38A44R68R70R75N |
| 28Q33Y38Q40K44Q68R70S75R77T80K | 30R33G38S44G68Q70T75N |
| 30N33H38Q44D68R70N75N | 28K30G38K44G68Q70T75N |
| 28K33N38Q40Q44A68N70N75N | 28K30G38H44Q68R70G75N |
| 28K33R38E40R44K68R70E75N | 28K30G38H44A68R70S75N |
| 28Q33S38R40K44K68R70E75N | 28K33T38A40A44A68R70S75N |
| 28K33N38Q40Q44K68T70T75N | 28K30G38H44Q68A70N75N |

11. Variante nach einem der Ansprüche 1 bis 6, bei der es sich um ein Heterodimer handelt, das ein DNA-Target spalten kann, das aus der Gruppe bestehend aus den Sequenzen SEQ ID NO: 120 bis 166 von dem menschlichen *XPD*-Gen ausgewählt ist, wobei das erste und das zweite Monomer Aminosäuren in den Positionen 28, 30, 33, 38, 40, 44, 68, 70, 75, 77, 80 und/oder 133 von I-*Cre*I aufweisen, wie sie in der Tabelle XXVI angegeben sind:
| Erstes Monomer | Zweites Monomer |
|---|---|
| 28K30N38Q44K68R70E75N | 28Q33S38R40K44A68S70R75N |
| 28K30G38H44Q68R70N75N | 28K30G38K44Y68D70S75R77T |
| 28K30N38Q44D68Y70S75S77R | 28R33A38Y40Q44R68Y70S75E77Y |
| 30N33H38Q44Q68R70S75N | 30N33H38A44A68R70S75N |
| 28Q33S38R40K44K68T70G75N | 28K33R38Q40S44A68R70S75E77R |
| 28K30G38H44K68R70E75N | 30N33H38A44K68R70G75N |
| 28R33A38Y40Q44D68R70R75N | 28K33N38Q40Q44Q68R70S75R77T80K |
| 28K33R38E40R44R68R70R75N | 30Q33G38H44A68R70G75N |
| 30D33R38T44K68R70E75N | 28Q33Y38R40K44A68R70S75N |
| 44Q68R70Q75N | 33T44Q68Y70S75R77Q |
| 28K33T38R40Q44A68R70G75N | 28K33R38E40R44Q68R70S75R77T80K |
| 30D33R38G44K68S70N75N | 30D33R38T44A68S70R75N |
| 28Q33Y38Q40K44K68R70E75N | 28K30G38H44A68S70R75N |
| 30N33H38A44D68Y70S75S77R | 28K33R38E40R44Q68R70S75R77T80K |
| 28K33S38Q40Q44T68R70S75Y7TT133V | 30N33T38Q44Q68R70S75N |
| 30D33R38T44K68R70E75N | 28Q33Y38R40K44A68S70R75N |
| 30D33R38T44Q68R70S75N | 28K33R38Q40S44K68Y70S75D77T |
| 28R33A38Y40Q44K68T70G75N | 44N68R70R75N |
| 28K33R38FA0R44Q68R70G75N | 28K33T38R40Q44Q68Y70S75R77Q |
| 30N33H38Q44R68Y70S75E77Y | 28K33R38Q40S70S75N |
| 28R33A38Y40Q44T68Y70S75R77V | 30R33G38S44A68R70S75Y77Y |
| 28K33R38A40Q44K68R70E75N | 28K30G38H44N68R70R75N |
| 28K33R38Q40S44D68R70N75N | 28K33S38R40D44A68R70G75N |
| 30D33R38G44K68H70E75N | 28K30G38G44A68R70G75N |
| 30N33H38Q44R68R70R75N | 30N33T38A44K68R70E75N |
| 30D33R38T44K68T70G75N | 30R33G38S44Q68R70N75N |
| 28K30G38H44R68Y70S75E77Y | 28R33A38Y40Q44Q68R70S75R77T80K |
| 28R33A38Y40Q44A68R70S75N | 28K30G38H44Q68R70S75R77T80K |
| 30N33H38A44K68A70N75N | 28Q33Y38Q40K44Y68D70S75R77T |
| 28Q33Y38R40K44K68R70E75N | 28K33R38E40R44N68R70R75N |
| 28K33R38A40Q44K68Y70S75Q77N | 28K33S38Q40Q44K68R70E75N |
| 28Q33Y38R40K44D68R70R75N | 30N33H38A44A68R70N75N |
| 30D33R38G44A68N70N75N | 30D33R38T44K68R70E75N |
| 28K33N38Q40Q44D68R70N75N | 30D33R38T44A68R70S75N |
| 30N33H38A44Q68R70S75N | 30Q33G38H44A68N70N75N |
| 28R33A38Y40Q44K68R70E75N | 28K33R38Q40S44E68R70A75N |
| 28R30D38Q44K68R70E75N | 28K33S38R40D70S75N |
| 30A33D38H44K68H70E75N | 28K33T38A40A44N68R70R75N |
| 28K30N38Q44K68A70S75N77I | 28K33R38E40R44A68S70R75N |
| 28Q33Y38Q40K44K68R70E75N | 28Q33Y38Q40K44Q68R70S75R77T80K |
| 30D33R38G44N68R70A75N | 28Q33Y38Q40K44Q68R70S75R77T80K |
| 30N33H38Q44R68R70R75N | 28R33A38Y40Q44A68R70S75N |
| 28K33S38Q40Q44R68Y70S75E77I | 30N33T38A44A68R70S75N |
| 28K30G38K44N68R70N75N | 28K33R38E40R44D68Y70S75S77R |
| 28K33R38A40Q44K68R70E75N | 28K30N38Q44Q68Y70S75R77Q |
| 28K33T38R40Q44K68R70G75N | 28R33A38Y40Q44E68R70A75N |
| 28K33N38Q40Q44Q68Y70S75R77Q | 30D33R38T44Q68R70G75N |

12. Variante nach einem der Ansprüche 1 bis 6, bei der es sich um ein Heterodimer handelt, das ein DNA-Target spalten kann, das aus der Gruppe bestehend aus den Sequenzen SEQ ID NO: 167 bis 188 von dem menschlichen *XPF*-Gen ausgewählt ist, wobei das erste und das zweite Monomer Aminosäuren in den Positionen 28, 30, 33, 38, 40, 44, 68, 70, 75, 77 und/oder 80 von I-*Cre*I aufweisen, wie sie in der Tabelle XXVII angegeben sind:
| Erstes Monomer | Zweites Monomer |
|---|---|
| 30N33H38Q44T68Y70S75R77V | 30D33R38T44Q68R70G75N |
| 28K33S38R40A44K68S70N75N | 28E33R38R40K44Q68R70G75N |
| 28K33T38A40A44Q68R70N75N | 30D33R38G44Q68R70S75N |
| 28Q33538R40K44R68Y70575E77Y | 28K33T38A40Q44T68Y70S75R77T |
| 28R30D38Q44Q68R70G75N | 28R33A38Y40Q44Q68R70S75R77T80K |
| 28K30N38Q44A68R70S75Y77Y | 28K33T38R40Q44A68R70S75Y77Y |
| 28K33T38R40Q44Q68Y70S75R77Q | 28K30N38Q44K68Y70S75Q77N |
| 28K33T38A40Q44Q68R70S75N | 28R30D38Q44A68N70S75Y77R |
| 28K33S38Q40Q44Q68R70S75N | 28K33N38Q40Q44A68R70S75Y77Y |
| 28K33R38A40Q44E68R70A75N | 28K33R38A40Q44Q68R70S75R77T80K |
| 28K33T38A40A42T44K70S75N77Y | 28K33T38A40A44T68Y70S75R77V |
| 30N33T38Q44K68R70E75N | 28T33R38Q40R44Q68R70N75N |
| 28Q33Y38Q40K44Q68R70G75N | 28R30D38Q44T68Y70S75R77V |
| 28K33S38Q40Q44Q68R70S75N | 28K30N38Q44K68R70G75N |
| 28K33T38A40Q44Q68R70G75N | 28Q33Y38R40K44Q68R70R75E77R |
| 28Q33Y38R40K44Q68R70S75R77T80K | 28A33T38Q40R44Q68R70S75R77T80K |
| 30D33R38T44A68R70G75N | 28K33R38E40R44Q68R70G75N |
| 28Q33S38R40K44R68Y70S75D77N | 30D33R38T44K68R70E75N |
| 28K30G38G44T68Y70S75R77V | 28R33A38Y40Q44K68A70S75N77I |
| 28Q33Y38R40K44D68Y70S75S77R | 30D33R38T44E68R70A75N |
| 30N33T38A44Q68R70G75N | 28K30G38H44R68Y70S75E77Y |
| 28K30G38H44A68N70N75N | 30N33H38A44R68Y70S75E77V |

13. Variante nach einem der Ansprüche 1 bis 6, bei der es sich um ein Heterodimer handelt, das ein DNA-Target spalten kann, das aus der Gruppe bestehend aus den Sequenzen SEQ ID NO: 189 bis 216 von dem menschlichen *XPG*-Gen ausgewählt ist, wobei das erste und das zweite Monomer Aminosäuren in den Positionen 28, 30, 33, 38, 40, 42, 44, 68, 70, 75, 77, 80 und/oder 133 von I-*Cre*I aufweisen, wie sie in der Tabelle XXVIII angegeben sind:
| Erstes Monomer | Zweites Monomer |
|---|---|
| 30D33R38T44K68R70E75N | 30D33R38T44Q68N70R75N |
| 30D33R38T44E68R70A75N | 28T33T38Q40R44Q68Y70S75R77Q |
| 30N33Y38Q44Q68R70S75R77T80K | 28K33T38A40A44K68R70E75N |
| 28T33R38S40R44A68R70S75N | 30N33H38Q44N68R70S75R77D |
| 28K30G38H44Q68R70Q75N | 28T33R38Q40R44A68R70S75N |
| 28K33T38R40Q44N68R70A75N | 28T33R38Q40R44K68R70E75N |
| 28K30G38H44A68Q70N75N | 32T33C44Y68D70S75R77T |
| 28R30D38Q44T68R70S75Y77T133V | 30N33T38A44Q68R70S75N |
| 28Q33Y38Q40K44A68R70N75N | 28K30N38Q44Q68R70G75N |
| 28K33R38E40R44Q68R70S75N | 28K33R38Q40A44K68Q70S75N77R |
| 30A33D38H44K68G70T75N | 28K33T38A40Q44N68R70S75R77D |
| 32T33C44N68R70A75N | 28R33A38Y40Q44K68R70E75N |
| 2SK3QG38H44A68R70D75N | 30N33H38A44Q68R70S75N |
| 30R33G38S44K68T70S75N | 28R33A38Y40Q42T44K70S75N77Y |
| 28R33S38Y40Q44K68T70S75N | 28R33A38Y40Q44N68R70S75R77D |
| 30N33H38Q44Q68R70D75N | 28Q33S38R40K44K68H70E75N |
| 28R33S38Y40Q44Y68E70S75R77V | 28K30N38Q44K68R70E75N |
| 32T33C44A68R70S75N | 28T33T38Q40R44T68Y70S75R77T |
| 30N33H38A44D68Y70S75S77R | 28K33R38E40R44T68Y70S75R77T |
| 28R33A38Y40Q44N68R70N75N | 28R33A38Y40Q44Q68Y70S75N77Y |
| 30N33Y38Q44Q68S70K75N | 30D33R38G44A68N70S75Y77R |
| 28K33T38A40Q44E68R70A75N | 28R33A38Y40Q44S68Y70S75Y77V |
| 28A33T38Q40R44 E68R70A75N | 28K30G38G44A68N70N75N |
| 28K33R38A40Q44N68R70N75N | 28K33S38R40D42T44K70S75N77Y |
| 32T33C44Q68R70D75N | 28K33T38A40A44A68R70N75N |
| 32T33C44K68A70S75N | 30D33R38G44N6BR70N75N |
| 28Q33Y38R40K44A68R70S75N | 30D33R38T42T44K70S75N77Y |
| 30D33R38T44K68G70T75N | 30D33R38G44A68D70K75N |

14. Variante nach einem der Ansprüche 1 bis 6, bei der es sich um ein Heterodimer handelt, das ein DNA-Target spalten kann, das aus der Gruppe bestehend aus den Sequenzen SEQ ID NO: 1 bis 24 von dem menschlichen *XPC*-Gen ausgewählt ist, wobei das erste Monomer Aminosäuren in den Positionen 19, 28, 30, 33, 38, 40, 69, 70, 75 und/oder 87 von I-*Cre*I aufweist, die aus der Gruppe bestehend aus 28K30N33S38R40S70S75N, 28A30N33S38R40K70S75N, 19A28A30N33S38R40K70S75N, 19A28A30N33Y38R40K70S75N87L und 19A28A30N33S38R40K69G70S75N ausgewählt sind, und das zweite Monomer Aminosäuren in den Positionen 28, 30, 33, 38, 40, 44, 68, 70, 75, 85, 109 und/oder 161 of I-*Cre*I aufweist, die aus der Gruppe bestehend aus:
28E30N33Y38R40K44K68S70S75N, , 28E30N33Y38R40K44K68R70E75N85R109T, 28E30N33Y38R40K44K68R70E75N85R109T161F 28E30N32R33Y38Q40K44K68R70E75N85R109T, 28S30N33Y38R40K44K68S70S75N, 28S30N33Y38R40K44K68R70D75N, 28S30N33Y38R40K44K68A70S75N, 28K30G33Y38H40S44K68R70E75N, 28K30G33Y38H40S44K68A70G75N,28K30G33Y38R40S44K68R70E75N, 28K30G33Y38R40S44KL68T70H75N, 28K30G33Y38R40S44K68S70S75N und 28K30G33Y38R40S44K68T70S75N ausgewählt sind.

15. Einkettige chimäre Meganuklease, aufweisend zwei Monomere oder Kemdomänen von einer oder zwei I-*Cre*I-Varianten nach einem der Ansprüche 1 bis 14 oder eine Kombination aus beiden.

16. Polynukleotidenfragment, das eine Variante nach einem der Ansprüche 1 bis 14 oder eine einkettige chimäre Meganuklease nach Anspruch 15 verschlüsselt.

17. Ausdrucksvektor, aufweisend zumindest ein Polynukleotidenfragment nach Anspruch 16.

18. Ausdrucksvektor nach Anspruch 17, der zwei unterschiedliche Polynukleotidenfragmente aufweist, die jeweils eines der Monomere einer heterodimeren Variante, wie sie in einem der Ansprüche 7 bis 14 definiert ist, verschlüsselt.

19. Vektor nach Anspruch 18, der ein Target-Konstrukt beinhaltet, das eine DNA-Sequenz aufweist, die in ein menschliches Xeroderma-pigmentosum-Gen eingeführt werden soll, das von zumindest 50 bp der DNA-Sequenzen flankiert ist, die zumindest zu 95 % mit dem Bereich des menschlichen Xeroderma-pigmentosum-Gens identisch sind, das eine der Target-Sequenzen SEQ ID NO: 1 bis 24 und 45 bis 216 flankiert.

20. Vektor nach Anspruch 19, wobei die Sequenz, die eingeführt werden soll, eine Sequenz ist, die eine Mutation in einem Xeroderma-pigmentosum-Gen repariert.

21. Vektor nach Anspruch 20, wobei die Sequenz, die die Mutation repariert, die korrekte Sequenz des Xeroderma-pigmentosa-Gens ist.

22. Vektor nach Anspruch 20, wobei die Sequenz, die die Mutation repariert, die Exonen des Xeroderma-pigmentosum-Gens stromabwärts der genomischen Spaltstelle der Variante, in den Rahmen fusioniert, und eine Polyadenylationsstelle aufweist, um eine Transkription in 3' zu stoppen.

23. Vektor nach einem der Ansprüche 19 bis 22, wobei das Target-Konstrukt ein *XPA*-Genfragment aufweist, das eine Teilung in dem *XPA-Gen* reparieren kann und das zumindest die Nukleotiden von den Positionen 34 bis 233, 201 bis 400, 3493 bis 3692, 7627 bis 7826, 9994 bis 10193, 10151 bis 10350 12513 bis 12712, 12531 bis 12730, 21679 bis 21878, 21844 bis 22043, 21955 bis 22154, 22228 bis 22427 oder 22234 bis 22433 des *XPA*-Gens aufweist.

24. Vektor nach einem der Ansprüche 19 bis 22, wobei das Target-Konstrukt ein *XPB*-Genfragment aufweist, das eine Teilung in dem *XPB*-Gen reparieren kann und das zumindest die Nukleotiden von den Positionen: -40 bis 159, 357 bis 556, 1335 bis 1534, 1336 bis 1535, 1457 bis 1656, 3624 bis 3823, 4108 bis 4307, 5015 bis 5214, 5148 bis 5347, 5284 bis 5483, 5420 bis 5619, 7377 bis 7576, 13517 bis 13716, 13552 bis 13751, 13633 bis 13832, 14697 bis 14896, 14760 bis 14959, 14881 bis 15080, 21139 bis 21338, 21207 bis 21406, 22796 bis 22995, 32378 bis 32577, 33003 bis 33202, 34481 bis 34680, 34869 bis 35068, 34891 bis 35090, 36584 bis 36783, 36634 bis 36833 oder 36639 bis 36838 des *XPB*-Gens aufweist.

25. Vektor nach einem der Ansprüche 19 bis 22, wobei das Target-Konstrukt ein *XPC*-Genfragment aufweist, das eine Teilung in dem *XPC*-Gen reparieren kann und das zumindest die Nukleotiden von den Positionen: 105 to 304, 5704 to 5903, 7973 bis 8172, 9887 bis 10086, 10173 bis 10372, 11263 bis 11462, 13051 bis 13250, 13432 bis 13631, 18619 bis 18818, 19580 bis 19779, 20303 bis 20502, 20349 bis 20548, 20389 bis 20588, 21985 bis 22184, 21990 bis 22189, 22028 bis 22227, 22102 bis 22301, 26017 bis 26216, 29566 bis 29765, 29726 bis 29925, 30416 bis 30615, 31166 bis 31365 oder 32317 bis 32516 des *XPC*-Gens aufweist.

26. Vektor nach einem der Ansprüche 19 bis 22, wobei das Target-Konstrukt ein *XPD*-Genfragment aufweist, das eine Teilung in dem *XPD*-Gen reparieren kann und das zumindest die Nukleotiden von den Positionen: -87 bis 112, 812 bis 1011, 1319 bis 1518, 1324 bis 1523, 1426 bis 1625, 1717 bis 1916, 1867 bis 2066, 5473 bis 5672, 5585 bis 5784, 5637 bis 5836, 5920 bis 6119, 6050 bis 6249, 6290 bis 6489, 6392 bis 6591, 6472 bis 6671, 6581 bis 6780, 8830 bis 9029, 8943 bis 9142, 12661 bis 12860, 12991 bis 13190, 13084 bis 13283, 14614 bis 14813, 14817 bis 15016, 15528 bis 15727, 15878 bis 16077, 15936 bis 16135, 17023 bis 17222, 17350 bis 17549, 17365 bis 17564, 17572 bis 17771, 18347 bis 18546, 18370 bis 18569 oder 18641 bis 18840 des *XPD*-Gens aufweist.

27. Vektor nach einem der Ansprüche 19 bis 22, wobei das Target-Konstrukt ein *XPE*-Genfragment aufweist, das eine Teilung in dem *XPE*-Gen reparieren kann und das zumindest die Nukleotiden von den Positionen 10-209, 1295-1494, 2899-3098, 3488-3687, 3616-3815, 6093-6292, 6194-6393, 7034-7233, 7653-7852, 8753-8952, 9781-9980, 9966-10165, 10511-10710, 10665-10864, 11534-11733, 16439-16638, 16667-16866, 18268-18647, 18757-18956, 18863-19062, 19179-19378, 19266-19465, 19596-19795, 20714-20913, 20938-21137, 21099-21298, 22568-22767, 22732-22931, 23173-23372, 23181-23380, 23954-24153, 24000-24199, 29205-29404, 29651-29850, 30280-30479, 30355-30554, 30661-30860, 30685-30884, 32150-32349, 32753-32592, 32770-32969, 32811-33010, 32836-33035, 32841-33040, 33230-33429, 33369-33568 oder 33512-33711 des *XPE*-Gens aufweist.

28. Vektor nach einem der Ansprüche 19 bis 22, wobei das Target-Konstrukt ein *XPF*-Genfragment aufweist, das eine Teilung in dem *XPF*-Gen reparieren kann und das zumindest die Nukleotiden von den Positionen 244-443, 1731-1930, 6429-6628, 6486-6685, 7918-8117, 10500-10699, 10676-10875, 11885-12084, 11886-12085, 12176-12375, 14073-14272, 14110-14309, 15336-15535, 15431-15630, 15574-15773, 17673-17872, 17677-17876, 24486, 24685, 27496-27695, 27822-28021, 27827-28026 oder 27963-28162 des *XPF*-Gens aufweist.

29. Vektor nach einem der Ansprüche 19 bis 22, wobei das Target-Konstrukt ein XPG-Genfragment aufweist, das eine Teilung in dem XPG-Gen reparieren kann und das zumindest die Nukleotiden von den Positionen: 158-357, 5956-6155, 7961-7890, 8044-8243, 9977-10176, 12201-12400, 12289-12488, 15230-15429, 15803-16002, 16041-16240, 16146-16345, 16174-16373, 16174-16373, 16394-16593, 16553-16732, 16887-17086, 19487-19686, 19727-19926, 19782-19981, 20207-20406, 20500-20699, 21890-22089, 22117-22316, 25876-26075, 26450-26649, 26832-27031, 27258-27457, 29180-29379 oder 29456-29655 des XPG-Gens aufweist.

30. Vektor nach Anspruch 20 oder 22, wobei die Sequenz, die die Mutation repariert, von einem Xeroderma-pigmentosum-Genfragment flankiert ist, wie es in einem der Ansprüche 23 bis 29 definiert ist.

31. Vektor nach Anspruch 20 oder 21, wobei die Sequenz, die die Mutation repariert, in dem Xeroderma-pigmentosum-Genfragment, wie es in einem der Ansprüche 23 bis 29 definiert ist, beinhaltet ist.

32. Zusammensetzung, aufweisend zumindest eine Variante nach einem der Ansprüche 1 bis 14, eine einkettige chimäre Meganuklease nach Anspruch 15 und/oder zumindest einen Ausdrucksvektor nach einem der Ansprüche 17 bis 31.

33. Zusammensetzung nach Anspruch 32, die ein Target-DNA-Konstrukt aufweist, das eine Sequenz aufweist, die eine Mutation in einem menschlichen Xeroderma-pigmentosum-Gen, das durch Sequenzen flankiert ist, die dem Bereich des Gens entsprechen, der eine der Target-Sequenzen SEQ ID NO: 1 bis 24 und 45 bis 216 umgibt, repariert.

34. Zusammensetzung nach Anspruch 33, wobei das Target-DNA-Konstrukt in einem rekombinanten Vektor beinhaltet ist.

35. Produkte, die einen Ausdrucksvektor nach Anspruch 17 oder 18 und einen Vektor enthalten, der ein Target-Konstrukt, wie es in einem der Ansprüche 19 bis 31 definiert ist, als eine kombinierte Zubereitung für eine gleichzeitige, gesonderte oder sequentielle Verwendung zum Verhindern, Bessern oder Heilen einer Krankheit, die Xeroderma pigmentosum zugeordnet ist, bei einem Individuum, das diese benötigt, beinhaltet.

36. Verwendung von zumindest einer Variante nach einem der Ansprüche 1 bis 14, einer einkettigen chimären Meganuklease nach Anspruch 15 und/oder eines Ausdrucksvektors nach einem der Ansprüche 19 bis 31, für die Zubereitung eines Medikaments zum Verhindern, Bessern oder Heilen einer Krankheit, die Xeroderma pigmentosum zugeordnet ist, bei einem Individuum, das diese benötigt.

37. Wirtszelle, aufweisend ein Polynukleotid nach Anspruch 16 oder einen Vektor nach einem der Ansprüche 19 bis 31.

38. Nichtmenschliches transgenes Tier, aufweisend ein oder zwei Polynukleotidfragmente, wie sie in Anspruch 16 oder Anspruch 18 definiert sind.

39. Transgene Pflanze, aufweisend ein oder zwei Polynukleotidfragmente, wie sie in Anspruch 16 oder 18 definiert sind.

40. Verwendung von zumindest einer Variante nach einem der Ansprüche 1 bis 14, einer einkettigen chimären Meganuklease nach Anspruch 15, eines Vektors nach einem der Ansprüche 17 bis 31, zu nicht therapeutischen Zwecken in der Gentechnik.

41. Verwendung nach Anspruch 40, wobei die Variante, die einkettige chimäre Meganuklease, der Vektor einem Target-DNA-Konstrukt, wie es in einem der Ansprüche 19 bis 31 definiert ist, zugeordnet ist.

## Revendications

1. Variant d'I-CreI, **caractérisé en ce qu'**il possède au moins deux substitutions, une dans chacun des deux sous-domaines fonctionnels du domaine de base LAGLIDADG situés de la position 28 à la position 40 et de la position 44 à la position 70 de la séquence d'acides aminés SEQ ID N° 217 d'I-CreI, ledit variant étant capable de cliver une séquence d'ADN cible choisie dans le groupe consistant en les séquences SEQ ID N° 1 à 24 et 45 à 216 provenant d'un gène de la xérodermie pigmentaire humaine, et pouvant être obtenu par un procédé comprenant les étapes de :
(a) construction d'une première série de variants d'I-CreI, ayant au moins une substitution aux positions 28, 30, 32, 33, 38 et/ou 40 d'un premier sous-domainé fonctionnel du domaine de base LAGLIDADG situé de la position 28 à la position 40 d'I-*Cre*I,
(b) construction d'une deuxième série de variants d'I-CreI ayant au moins une substitution aux positions 44, 68 et/ou 70 d'un deuxième sous-domaine fonctionnel du domaine de base LAGLIDADG situé de la position 44 à la position 70 d'I-*Cre*I,
(c) sélection des variants provenant de la première série de l'étape (a), qui sont capables de cliver un site I-*Cre*I mutant, dans lequel (i) le triplet nucléotidique aux positions -10 à -8 du site I-*Cre*I a été remplacé par le triplet nucléotidique qui est présent aux positions -10 à -8 d'un ADN génomique cible choisi dans le groupe consistant en les séquences SEQ ID N° 1 à 24 et 45 à 216 provenant d'un gène de la xérodermie pigmentaire humaine, et (ii) le triplet nucléotidique aux positions +8 à +10 a été remplacé par la séquence complémentaire inverse du triplet nucléotidique qui est présent aux positions -10 à -8 de ladite cible génomique,
(d) sélection des variants provenant de la deuxième série de l'étape (b), qui sont capables de cliver un site I-CreI mutant, dans lequel (i) le triplet nucléotidique aux positions -5 à -3 du site I-CreI a été remplacé par le triplet nucléotidique qui est présent aux positions -5 à -3 de ladite cible génomique de (c), et (ii), le triplet nucléotidique aux positions +3 à +5 a été remplacé par la séquence complémentaire inverse du triplet nucléotidique qui est présent aux positions -5 à -3 de ladite cible génomique,
(e) sélection des variants provenant de la première série de l'étape (a), qui sont capables de cliver un site I-CreI mutant, dans lequel (i) le triplet nucléotidique aux positions +8 à +10 du site I-CreI a été remplacé par le triplet nucléotidique qui est présent sur les positions +8 à +10 de ladite cible génomique de (c), et (ii) le triplet nucléotidique aux positions -10 à -8 a été remplacé par la séquence complémentaire inverse du triplet nucléotidique qui est présent aux positions +8 à +10 de ladite cible génomique,
(f) sélection des variants provenant de la deuxième série de l'étape (b), qui sont capables de cliver un site I-CreI mutant, dans lequel (i) le triplet nucléotidique aux positions +3 à +5 du site I-CreI a été remplacé par le triplet nucléotidique qui est présent aux positions +3 à +5 de ladite cible génomique de (c), et (ii) le triplet nucléotidique aux positions -5 à -3 a été remplacé par la séquence complémentaire inverse du triplet nucléotidique qui est présent aux positions +3 à +5 de ladite cible génomique,
(g) combinaison, en un variant unique, de la ou des mutations aux positions 28 à 40 et 44 à 70 de deux variants provenant de l'étape (c) et de l'étape (d), pour obtenir un nouveau variant d'I-*Cre*I homodimère, qui clive une séquence dans laquelle (i) le triplet nucléotidique aux positions -10 à -8 est identique au triplet nucléotidique qui est présent aux positions -10 à -8 de ladite cible génomique de (c), (ii) le triplet nucléotidique aux positions +8 à +10 est identique à la séquence complémentaire inverse du triplet nucléotidique qui est présent aux positions -10 à -8 de ladite cible génomique, (iii) le triplet nucléotidique aux positions -5 à -3 est identique au triplet nucléotidique qui est présent aux positions -5 à -3 de ladite cible génomique, et (iv) le triplet nucléotidique aux positions +3 à +5 est identique à la séquence complémentaire inverse du triplet nucléotidique qui est présent aux positions -5 à -3 de ladite cible génomique,
(h) combinaison, en un variant unique, de la ou des mutations aux positions 28 à 40 et 44 à 70 de deux variants provenant de l'étape (e) et de l'étape (f), pour obtenir un nouveau variant d'I-*Cre*I homodimère, qui clive une séquence dans laquelle (i) le triplet nucléotidique aux positions +3 à +5 est identique au triplet nucléotidique qui est présent aux positions +3 à +5 de ladite cible génomique de (c), (ii) le triplet nucléotique aux positions -5 à -3 est identique à la séquence complémentaire inverse du triplet nucléotidique qui est présent aux positions +3 à +5 de ladite cible génomique, (iii) le triplet nucléotidique aux positions +8 à +10 du site I-CreI a été remplacé par le triplet nucléotidique qui est présent aux positions +8 à +10 de ladite cible génomique, et (iv) le triplet nucléotidique aux positions -10 à -8 est identique à la séquence complémentaire inverse du triplet nucléotidique aux positions +8 à +10 de ladite cible génomique,
(i) combinaison des variants obtenus dans les étapes (g) et (h) pour former des hétérodimères, et
(j) sélection des hétérodimères de l'étape (i) qui sont capables de cliver ladite séquence d'ADN cible choisie dans le groupe consistant en les séquences SEQ ID N° 1 à 24 et 45 à 216, provenant d'un gène de la xérodermie pigmentaire humaine.

2. Variant selon la revendication 1, dans lequel lesdites substitutions sont le remplacement des acides aminés initiaux par des acides aminés choisis dans le groupe consistant en A, D, E, G, H, K, N, P, Q, R, S, T, Y, L et V.

3. Variant selon la revendication 1 ou 2, qui comprend une ou plusieurs substitutions aux positions 19, 24, 26, 42, 69, 75, 77, 80, 85, 87, 109, 133 et 161 d'I-CreI.

4. Variant selon l'une quelconque des revendications 1 à 3, qui comprend la substitution de l'acide aspartique à la position 75 par un acide aminé non chargé.

5. Variant selon la revendication 4, dans lequel ledit acide aminé non chargé est un résidu d'asparagine ou de valine.

6. Variant selon l'une quelconque des revendications 1 à 5, qui peut être obtenu par un procédé comprenant les étapes a) à j) telles que définies dans la revendication 1, et les étapes additionnelles de mutagenèse aléatoire sur au moins un monomère de l'hétérodimère formé dans l'étape (i) ou obtenu dans l'étape (j), et la sélection et/ou le criblage des hétérodimères ayant une activité améliorée vis-à-vis dudit ADN cible provenant d'un gène de la xérodermie pigmentaire, par comparaison avec ledit hétérodimère initial formé dans l'étape (i) ou obtenu dans l'étape (j).

7. Variant selon l'une quelconque des revendications 1 à 6, qui est un hétérodimère capable de cliver un ADN cible choisi dans le groupe consistant en les séquences SEQ ID N° 45 à 57 provenant du gène *XPA* humain, dans lequel le premier et le deuxième monomères ont des acides aminés aux positions 24, 26, 28, 30, 33, 38, 40, 42, 44, 68, 70, 75, 77 et/ou 80 d'I-CreI, qui sont tels qu'indiqués dans le Tableau XXII :
| Premier monomère | Deuxième monomère |
|---|---|
| 28K33S38R40D44K68R70G75N | 28R30DF38R44K68A70S75N77I |
| 28K30G38H44Q68R70Q75N | 28K30G38K44Q68R70S75R77T80K |
| 28K33T38A40Q44N68K70S75R77N | 30D33R38G44N68K70S75R77N |
| 28K30G38H44R68Y70S75E77Y | 28K33N38Q40Q44K68R70E75N |
| 28K30N38Q44Q68A70N75N | 28Q33Y38R40K42R44Q70S75N77N |
| 30N33H38Q44K68R70E75N | 28K33R38A40Q44Q68R70S75N |
| 28K30N38Q44K68H70E75N | 28K33R38A40Q44Q68R70S75N |
| 28K30G38G44Q68R70G75N | 30D33R38G44Q68R70S75N |
| 28K30G38H44Q68R70S75R77T80K | 28K33T38A40Q44Q68R70G75N |
| 30N33H38Q44Q68A70N75N | 24126Q28K30N33Y38Q40S44K68R70E75N |
| 28K30N38Q44K68A70N75N | 28K33R38A40Q44A68R70G75N |
| 28K33R38E40R44K68S70N75N | 28K30G38H44R68Y70S75E77Y |
| 28K33R38A40Q44N68R70N75N | 30D33R38G44A68N70N75N |

8. Variant selon l'une quelconque des revendications 1 à 6, qui est un hétérodimère capable de cliver un ADN cible choisi dans le groupe consistant en les séquences SEQ ID N° 58 à 86 provenant du gène *XPB* humain, dans lequel le premier et le deuxième monomères ont des acides aminés aux positions 24, 26, 28, 30, 33, 38, 40, 42, 44, 68, 70, 75, 77 et/ou 80 d'I-CreI, qui sont tels qu'indiqués dans le Tableau XXIII :
| Premier monomère | Deuxième monomère |
|---|---|
| 30R33G38S44K68S70N75N | 30N33H38Q44Q68R70S75R77T80K |
| 30N33H3aA4₄K68R70E75N | 30D33R38T44K68S70N75N |
| 28K33N38Q40Q44R68R70R75N | 28K33R38A40Q44R68R70R75N |
| 28R30D38Q44E68R70A75N | 28Q33S38R40K44K68T70T75N |
| 30N33H38A44A68R70G75N | 28K33T38A40Q44068Y70S75R77Q |
| 30N33H38A44A68N70N75N | 30N33H38Q44R68Y70S75E77Y |
| 28K33S38Q40Q44R68R70R75N | 28K33R38Q40S44K68R70G75N |
| 28K33T38A40A44Q68R70S75R77T80K | 30N33H38Q44K68Y70S75Q77N |
| 30N33T38A42R44Q70S7SN77N | 28R33A38Y40Q44K68R70E75N |
| 30D33R38G44K68A70N75N | 28K30G38H44R68R70R75N |
| 20R33A38Y40Q44Q68R70G75N | 30N33H38A44A68S70R75N |
| 28Q33Y38Q40K44A68N70N75N | 30D33R38G44Q68R70S75R77T80K |
| 30N33H38Q44K68Y70S75D77T | 28Q33Y38Q40K44K68Y70S75D77T |
| 28K33S38Q40Q44Q68R70S75N | 28K30G38H44K68H70E75N |
| 30N33H38A44A68R70S75N | 28K30G38H44A68N70N75N |
| 30D33R38T44K68S70N75N | 30D33R38G44R68Y70S75E77Y |
| 24I26Q28K30N33Y38Q40S44K68H70E75N | 28K30N38Q44K68Y70S75D77T |
| 28K33T38A40A44K68Y70S75Q77N | 30N33H38Q44Q68R70G75N |
| 30N33H38A44N68K70S75R77N | 28R33A38Y40Q44R68Y70S75E77Y |
| 28Q33Y38Q40K44K68T70T75N | 24I26Q28K30N33Y38Q40S44E68R70A75N |
| 24I26Q28K30N33Y38Q40S44Q68R70N75N | 30D33R38T44A68R70S75Y77Y |
| 28K33T38A44Q44Q68R70Q75N | 28K30N38Q44Q68Y70S75R77Q |
| 28K33S38R40D44Y68D70S75R77T | 28K33S38R40D44K68T70T75N |
| 30N33H38A44R68Y70S75E77Y | 28R33A38Y40Q44Q68R70S75R77T80K |
| 28Q33Y38R40K44Q68R70S75R77T80K | 30R33G38S44R68R70R75N |
| 30R33G38S44Q68R70S75N | 28R30D38Q44K68A70N75N |
| 30D33R38T42R44Q70S77N | 30N33T38A44A68R70S75N |
| 30N33T38A44Q68R70S75N | 30N33H38Q44E68R70A75N |
| 30N33H38A44K68A70N75N | 30N33T38Q44A68S70R75N |

9. Variant selon l'une quelconque des revendications 1 à 6, qui est un hétérodimère capable de cliver un ADN cible choisi dans le groupe consistant en les séquences SEQ ID N° 1 à 24 provenant du gène *XPC* humain, dans lequel le premier et le deuxième monomères ont des acides aminés aux positions 28, 30, 33, 38, 40, 42, 44, 68, 70, 75, 77, 80 et/ou 133 d'I-*Cre*I, qui sont tels qu'indiqués dans le Tableau XXIV :
| Premier monomère | Deuxième monomère |
|---|---|
| 30D33R38G44R68Y70S75Y77T | 28K33R38E40R44R68Y70S75E77V |
| 30D33R38T44T68Y70S75R77T | 28K33R38N40Q44N68R70N75N |
| 28K33R38E40R44R68Y70S75E77I | 28Q33Y38R40K42R44Q70S77N |
| 28Q33S38R40K44Q68Y70S75N77Y | 28T33T38Q40R44T68E70S75R77R |
| 30D33R38T44N68R70S75Q77R | 28R33A38Y40Q44D68Y70S75S77R |
| 28K33R38Q40A44T68R70S75Y77T133V | 28K33R38E40R44K68Y70S75D77T |
| 28K33N38Q40Q44R68Y70S75E77I | 28K33T38A40Q44K68Q70S75N77R |
| 28E33R38R40K44Q68Y70S75N77Y | 28Q33S38R40K44A68R70S75R77L |
| 28K33T38A40Q44A68R70S75R77L | 28A33T38Q40R44R68S70S75E77R |
| 28K30N38Q44Q68R70S75R77T80K | 28T33T38Q40R44T68Y70S75R77V |
| 28K30N38Q44A68Y70S75Y77K | 28K33R38E40R44T68R70S75Y77T133V |
| 28K33R38Q40A44Q68R70N75N | 28K33R38A40Q44Q68R70S75N77K |
| 30N33H38Q44K68A70S75N77I | 28K33N38Q40Q44R68Y70S75E77V |
| 28Q33S38R40K44N68R70S75R77D | 28T33R3840R44Q68R70S75R77T80K |
| 28Q33A38R40K44T68Y70S75R77T | 28Q33Y38R40K44Y68D70S75R77V |
| 28K33T38A40A44T68E70S75R77R | 28K30N380Q44A68N70S75Y77R |
| 28Q33Y38Q40K44Q68R70S75R77T80K | 28K33R38Q40A44Q68R70N75N |
| 28K33R38A40Q44K68A70S75N77I | 28K33R38E40R44R68Y70S75Y77T |
| 28T33T38Q40R44Q68R70S75D77K | 28E33R38R40K44Q68R70S75R77T80K |
| 28R33A38Y40Q44Q68R70S75R77T80K | 28Q33Y38R40K44T68Y70S75R77T |
| 28Q33S38R40K42T44K70S75N77Y | 28R33A38Y40Q44A68R70S75E77R |
| 30D33R38T44A68Y70S75Y77K | 28Q33Y38R40K42R44Q70S75N77N |
| 28K33R38E40R44K68Q70S75N77R | 28Q33S38R40K44R68Y70S75E77V |
| 28K30N33H38Q40S44Q68R70R75N | 28K30N33R38A40Q44K68R70N75N |

10. Variant selon l'une quelconque des revendications 1 à 6, qui est un hétérodimère capable de cliver un ADN cible choisi dans le groupe consistant en les séquences SEQ ID N° 87 à 119 provenant du gène *XPD* humain, dans lequel le premier et le deuxième monomères ont des acides aminés aux positions 28, 30, 33, 38, 40, 44, 68, 70, 75, 77 et/ou 80 d'I-CreI, qui sont tels qu'indiqués dans le Tableau XXV :
| Premier monomère | Deuxième monomère |
|---|---|
| 30A33D38H44K68R70E75N | 28K30G38K44K68S70N75N |
| 30N33T38Q44Q68R70G75N | 30N33H38Q44A68R70S75E77R |
| 30N33H38A44N68R70N75N | 30N33H38Q44Q68R70S75R77T80K |
| 28K33R38E40R44E68R70A75N | 28R33A38Y40Q44A68S70R75N |
| 28K33T38R40Q44K68R70E75N | 28Q33S38R40K44K68R70G75N |
| 30D33R38T44K68R70E75N | 28K30G38G44Q68Y70S75R77Q |
| 30D33R38T44K68R70E75N | 30R33G38S44A68R70S75N |
| 28K30G38H44A68R70N75N | 28K33S38R40D44A68N70N75N |
| 30N33H38Q44Y68D70S75R77T | 28R30D38R44A68N70N75N |
| 28K33R38Q40S44K68T70T75N | 30D33R38G44K68R70E75N |
| 28R33A38Y40Q44Q68R70Q75N | 28K30G38H44R68Y70S75E77Y |
| 33R38E44Q68R70G75N | 28K33R38E40R44K68R70E75N |
| 30R33G38S44K68R70E75N | 30D33R36G44A68R70N75N |
| 30R33G38S44A68R70S75N | 30D33R38G44K68R70G75N |
| 30N33H38A44K68R70G75N | 28Q33Y38R40K44K68A70N75N |
| 30N33T38A44N68R70N75N | 28K30G38K44Q68R70S75N |
| 28R33A38Y40Q44K68R70G75N | 28Q33Y38R40K44T68Y70S75R77V |
| 28Q33Y38R40K44K68Y70S75Q77N | 33T44Q68R70S75N |
| 28K30G38G44A68N70N75N | 20K33R38E40R44E68R70A75N |
| 28K33R38A40Q44R68R70R75N | 28K33R38E40R44T68Y70S75R77V |
| 28K33R38Q40S44K68R70E75N | 30R33G38S44A68R70S75E77R |
| 28Q33Y38R40K44D68R70N75N | 28K33R38A40Q44R68R70R75N |
| 30N33H38A44Q68R70G75N | 28Q33Y38R40K44A68S70R75N |
| 30D33R38T44A68R70N75N | 28K33R38E40R44A68R70S75N |
| 28Q33Y38R40K44E68R70A75N | 33R38E44N68R70N75N |
| 28K30G38H44A68R70S75N | 30N33T38Q44R68Y70S75E77Y |
| 28K33R38E40R44Q68R70G75N | 30N33T38A44R68R70R75N |
| 28Q33Y38Q40K44Q66R70S75R77T80K | 30R33G38S44G68Q70T75N |
| 30N33H38Q44D68R70N75N | 28K30G38K44G68Q70T75N |
| 28K33N38Q40Q44A68N70N75N | 28K30G38H44Q68R70G75N |
| 28K33R38E40R44K68R70E75N | 28K30G38H44A68R70S75N |
| 28Q33S38R40K44K68R70E75N | 28K33T38A40A44A68R70S75N |
| 28K33N38Q40K68T70T75N | 28K30G38H44Q68A70N75N |

11. Variant selon l'une quelconque des revendications 1 à 6, qui est un hétérodimère capable de cliver un ADN cible choisi dans le groupe consistant en les séquences SEQ ID N° 120 à 166 provenant du gène *XPE* humain, dans lequel le premier et le deuxième monomères ont des acides aminés aux positions 28, 30, 33, 38, 40, 44, 68, 70, 75, 77, 80 et/ou 133 d'I-*Cre*I, qui sont tels qu'indiqués dans le Tableau XXVI :
| Premier monomère | Deuxième monomère |
|---|---|
| 28K30N38Q44K68R70E75N | 28Q33S38R40K44A68S70R75N |
| 28K30G38H44Q88R70N75N | 28K30G38K44Y68D70ST5R77T |
| 28K30N38Q44D68Y70S75S77R | 28R33A38Y40Q44R68Y70S75E77Y |
| | 30N33H38A44A68R70S75N |
| 28Q33S38R40K44K68T70G75N | 28K33R38Q40S44A68R70S75E77R |
| 28K30G38H44K68R70E75N | 30N33H38A44K68R70G75N |
| 28R33A38Y40Q44D68R70R75N | 28K33N38Q40Q68R70S75R77T80K |
| 28K33R38E40R44R68R70R75N | 30Q33G38H44A68R70G75N |
| 30D33R38T44K68A70E75N | 28Q33Y38R40K44A68R70S75N |
| 44Q68R70Q75N | 33T44Q68Y70S75R77Q |
| 28K33T38R40Q44A68R70G75N | 28K33R38E40R44Q68R70S75R77T80K |
| 30D33R38G44K68S70N75N | 30D33R38T44A68S70R75N |
| 28Q33Y38Q40K44K68R70E75N | 28K30G38M44A68S70R75N |
| 30N33H38A44D68Y70S75S77R | 28K33R38E40R44Q68R70S75R77T80K |
| 28K33S38Q40Q44T68R70S75Y77T1333V | 30N33T38Q44Q88R70S75N |
| 30D33R38T44K68R70E75N | 28Q33Y38R40K44A68S70R75N |
| 30D33R38T44Q68R70S75N | 28K33R38Q40S44K6BY70S75D77T |
| 28R33A38Y40Q44K68T70G75N | 44N68R70R75N |
| 28K33R38E40R44Q68R70G75N | 28K33T38R40Q44Q68Y70S75R77Q |
| 30N33H38Q44R68Y70S75E77Y | 28K33R38Q40S70S75N |
| 28R33A38Y40Q44T68Y70S75R77V | 30R33G38S44AA68R70S75Y77Y |
| 28K33R38A40Q44K68R70E75N | 28K30G38H44N68R70R75N |
| 28K33R38Q40S44D68R70N75N | 28K33S38R40D44A68R70G75N |
| 30D33R36G44K68H70E75N | 28K30G38G44A68R70G75N |
| 30N33H38Q44R68R70R75N | 30N33T38A44K68R70E75N |
| 30D33R38T44K68T70G75N | 30R33G38S44Q68R70N75N |
| 28K30G38H44R68Y70S75E77Y | 28R33A38Y40Q44Q68R70S75R77T80K |
| 28R33A38Y40Q44A68R70S75N | 28K30G38H44Q68R70S75R77T80K |
| 30N33H38A44K68A70N75N | 28Q33Y38Q40K44Y68D70S75R77T |
| 28Q33Y38R40K44K68R70E75N | 28K33R38E40R44N68R70R75N |
| 28K33R38A40Q44K68Y70S75Q77N | 28K33S38Q40Q44K68R70E75N |
| 28Q33Y38R40K44D68R70R75N | 30N33H38A44A68R70N75N |
| 30D33R38G44A68N70N75N | 30D33R38T44K68R70E75N |
| 28K33N38Q40Q44D68R70N75N | 30D33R38T44A68R70S75N |
| 30N33H38A44Q68R70S75N | 30Q33G38H44A68N70N75N |
| 28R33A38Y40Q44K68R70E75N | 28K33R38Q40S44E88R70A75N |
| 28R30D38Q44K68R70E75N | 28K33S38R40D70S75N |
| 30A33D38H44K68H70E75N | 28K33T38A40A44N68R70R75N |
| 28K30N38Q44K68A70S75N77I | 28K33R38E40R44A68S70R75N |
| 28Q33Y38Q40K44K68R70E75N | 28Q33Y38Q40K44Q68R70S75R77T80K |
| 30D33R38G44N68R70A75N | 28Q33Y38Q40K44Q68R70S75R77T80K |
| 30N33H38Q44R68R70R75N | 28R33A38Y40Q44A68R70S75N |
| 28K33S38Q40Q44R68Y70S75E77I | 30N33T38A44A68R70S75N |
| 28K30G38K44N68R70N75N | 28K33R38E40R44D68Y70S75S77R |
| 28K33R38A40Q44K68R70E75N | 28K30N38Q44Q68Y70S75R77Q |
| 28K33T38R40Q44K68R70G75N | 28R33A38Y40Q44E68R70A75N |
| 28K33N38Q40Q44Q68Y70S75R77Q | 30D33R38T44Q68R70G75N |

12. Variant selon l'une quelconque des revendications 1 à 6, qui est un hétérodimère capable de cliver un ADN cible choisi dans le groupe consistant en les séquences SEQ ID N° 167 à 188 provenant du gène *XPF* humain, dans lequel le premier et le deuxième monomères ont des acides aminés aux positions 28, 30, 33, 38, 40, 44, 68, 70, 75, 77 et/ou 80 d'I-*Cre*I, qui sont tels qu'indiqués dans le Tableau XXVII :
| Premier monomère | Deuxième monomère |
|---|---|
| 30N33H38Q44T68Y70S75R77V | 30D33R38T44Q68R70G75N |
| 28K33S38R4044K68S70N75N | 28E33R38R40K44Q68R70G75N |
| 28K33T38A40A44Q68R70N75N | 30D33R38G44Q68R70S75N |
| 28Q33S38R40K44R68Y70S75E77Y | 28K33T38A40Q44T68Y70S75R77T |
| 28R30D38Q44Q68R70G75N | 28R33A38Y40Q44Q66R70S75R77T80K |
| 28K30N38Q44A68R70S75Y77Y | 28K33T38R40Q44A68R70S75Y77Y |
| 28K33T38R40Q44Q68Y70S75R77Q | 28K3GN38044K68Y7CS75Q77N |
| 28K33T38A40Q44Q68R70S75N | 28R30D38Q44A68N70S75Y77R |
| 28K33S38Q40Q44Q68R70S75N | 28K33N38Q40Q44A68R70S75Y77Y |
| 28K33R38A40Q44E68R70A75N | 28K33R38A40Q44Q68R70S75R77T80K |
| 28K33T38A40A42T44K70S75N77Y | 28K33T38A40A44T68Y70S75R77V |
| 30N33T38Q44K68R70E75N | 28T33R38Q40R44Q68R70N75N |
| 28Q33Y38Q40K44Q68R70G75N | 28R30D38Q44T68Y70S75R77V |
| 28K33S38Q40Q44Q68R70S75N | 28K30N38Q44K68R70G75N |
| 28K33T38A40Q44Q68R70G75N | 28Q33Y38R40K44Q68R70R75E77R |
| 28Q33Y38R40K44Q68R70S75R77T80K | 28A33T38Q40R44Q68R70S75R77T80K |
| 30D33R38T44A68R70G75N | 28K33R38E40R44Q68R70G75N |
| 28Q33S38R40K44R68Y70S75D77N | 30D33R38T44K68R70E75N |
| 28K30G38G44T68Y70S75R77V | 28R33A38Y40Q44K68A70S75N77I |
| 28Q33Y38R40K44D68Y70S75S77R | 30D33R38T44E68R70A75N |
| 30N33T38A44Q68R70G75N | 28K30G38H44R68Y70S7577Y |
| 28K30G38H44A68N70N75N | 30N33H38A44R68Y70S75E77V |

13. Variant selon l'une quelconque des revendications 1 à 6, qui est un hétérodimère capable de cliver un ADN cible choisi dans le groupe consistant en les séquences SEQ ID N° 189 à 216 provenant du gène *XPG* humain, dans lequel le premier et le deuxième monomères ont des acides aminés aux positions 28, 30, 33, 38, 40, 42, 44, 68, 70, 75, 77, 80 et/ou 133 d'I-CreI, qui sont tels qu'indiqués dans le Tableau XXVIII :
| Premier monomère | Deuxième monomère |
|---|---|
| 30D33R38T44K68R70E75N | 30D33R38T44Q68N70R75N |
| 30D33R38T44E68R70A75N | 28T33T38Q40R44Q68Y70S75R77Q |
| 30N33Y38Q44Q68R70S75R77T80K | 28K33T38A40A44K68R70E75N |
| 28T33R38S40R44A68R70S75N | 30N33H38Q44N68R70S75R77D |
| 28K30G38H44Q68R70Q75N | 28T33R38Q40R44A68R70S75N |
| 28K33T38R40Q44N68R70A75N | 28T33R38Q40R44K68R70E75N |
| 28K30G38H44A6SQ70N75N | 32T33C44Y68D70S75R77T |
| 28R30D38Q44T68R70S75Y77T133V | 30N33T38A44Q68R70S75N |
| 28Q33Y38Q40K44A68R70N75N | 28K30N38Q44Q68R70G75N |
| 28K33R38E40R44Q68R70S75N | 28K33R38Q40A44K68Q70S75N77R |
| 30A33D38H44K68G70T75N | 28K33T38A40Q44N68R70S75R77D |
| 32T33C44N68R70A75N | 28R33A38Y40Q44K68R70E75N |
| 28K30G38H44A68R70D75N | 30N33H38A44Q68R70S75N |
| 30R33G38S44K68T70S75N | 28R33A38Y40Q42T44K70S75N77Y |
| 28R33S38Y40Q44K68T7DS75N | 28R33A38Y40Q44N68R70S75R77D |
| 30N33H38Q44Q68R70D75N | 28Q33S38R40K44K68H70E75N |
| 28R33S38Y40Q44Y68E70S75R77V | 28K30N38Q44K68R70E75N |
| 32T33C-44A68R70S75N | 28T33T38Q40R44T68Y70S75R77T |
| 30N33H38A44D68Y70S75S77R | 28K33R38E40R44T68Y70S75R77T |
| 28R33A38Y40Q44N68R70N75N | 28R33A38Y40Q44Q68Y70S75N77Y |
| 30N33Y38Q44Q68S70K75N | 30D33R38G44A68N70S75Y77R |
| 28K33T38A40Q44E68R70A75N | 28R33A38Y40Q44S68Y70S75Y77V |
| 28A33T38Q40R44E68R70A75N | 28K30G38G44A68N70N75N |
| 28K33R38A40Q44N68R70N75N | 28K33S38R40D42T44K70S75N77Y |
| 32T33C44Q68R70D75N | 28K33T38A4DA44A68R70N75N |
| 32T33C44K68A70S75N | 30D33R38G44N68R70N75N |
| 28Q33Y38R40K44A68R70S75N | 30D33R38T42T44K70S75N77Y |
| 30D33R38T44K68G70T75N | 30D33R38G44A68D70K75N |

14. Variant selon l'une quelconque des revendications 1 à 6, qui est un hétérodimère capable de cliver un ADN cible choisi dans le groupe consistant en les séquences SEQ ID N° 1 à 24 provenant du gène *XPC* humain, dans lequel le premier monomère a des acides aminés aux positions 19, 28, 30, 33, 38, 40, 69, 70, 75 et/ou 87 d'I-CreI qui sont choisis dans le groupe consistant en :
28K30N33S38R40S70S75N, 28A30N33S38R40K70S75N, 19A28A30N33S38R40K70S75N, 19A28A30N33Y38R40K70S75N87L, et 19A28A30N33S38R40K69G70S75N,
et le deuxième monomère a des acides aminés aux positions 28, 30, 33, 38, 40, 44, 68, 70, 75, 85, 109
et/ou 161 d'I-*Cre*I, qui sont choisis dans le groupe consistant en :
28E30N33Y38R40K44K68S70S75N, 28E30N33Y38R40K44K68R70E7SN85R109T, 28E30N33Y38R40K44K68R70E7SN8SR109T161F 28E30N32R33Y38Q40K44K68R70E75N85R109T, 28S30N33Y38R40K44K68S70S75N, 28S30N33Y38R40K44K68R70D75N, 28S30N33Y38R40K44K68A70S75N,28K30G33Y38H40S44K68R70E75N, 28K30G33Y38H40S44K68A70G75N, 28K30G33Y38R40S44K68R70E75N, 28K30G33Y38R40S44K68T70H75N, 28K30G33Y38R40S44K68S70S75N, et 28K30G33Y38R40S44K68T70S75N.

15. Méganucléase chimérique à chaîne unique comprenant deux monomères ou domaines de base d'un ou deux variants d'I-CreI selon l'une quelconque des revendications 1 à 14, ou une combinaison des deux.

16. Fragment polynucléotidique codant pour un variant selon l'une quelconque des revendications 1 à 14 ou une méganucléase chimérique à chaîne unique selon la revendication 15.

17. Vecteur d'expression comprenant au moins un fragment polynucléotidique selon la revendication 16.

18. Vecteur d'expression selon la revendication 17, qui comprend deux fragments polynucléotidiques différents, chacun codant pour l'un des monomères d'un variant hétérodimérique tel que défini dans l'une quelconque des revendications 7 à 14.

19. Vecteur selon la revendication 18, qui comprend une construction de ciblage comprenant une séquence d'ADN devant être introduite dans un gène de la xérodermie pigmentaire humaine flanquée par au moins 50 pb de séquences d'ADN ayant une identité d'au moins 95 % avec la région du gène de la xérodermie pigmentaire humaine flanquant l'une des séquences cibles SEQ ID N° 1 à 24 et 45 à 216.

20. Vecteur selon la revendication 19, dans lequel ladite séquence devant être introduite est une séquence qui répare une mutation dans le gène de la xérodermie pigmentaire.

21. Vecteur selon la revendication 20, dans lequel la séquence qui répare ladite mutation est la séquence correcte dudit gène de la xérodermie pigmentaire.

22. Vecteur selon la revendication 20, dans lequel la séquence qui répare ladite mutation comprend les exons dudit gène de la xérodermie pigmentaire, en aval du site génomique de clivage du variant, fusionnés en phase, et un site de polyadénylation pour stopper la transcription en 3'.

23. Vecteur selon l'une quelconque des revendications 19 à 22, dans lequel ladite construction de ciblage comprend un fragment du gène *XPA* qui est capable de réparer un clivage dans le gène *XPA* et qui comprend au moins les nucléotides des positions 34 à 233, 201 à 400, 3493 à 3692, 7627 à 7826, 9994 à 10193, 10151 à 10350, 12513 à 12712, 12531 à 12730, 21679 à 21878, 21844 à 22043, 21955 à 22154, 22228 à 22427 ou 22234 à 22433 dudit gène *XPA.*

24. Vecteur selon l'une quelconque des revendications 19 à 22, dans lequel ladite construction de ciblage comprend un fragment de gène *XPB* qui est capable de réparer un clivage dans le gène *XPB* et qui comprend au moins les nucléotides des positions -40 à 159, 357 à 556, 1335 à 1534, 1336 à 1535, 1457 à 1656, 3624 à 3823, 4108 à 4307, 5015 à 5214, 5148 à 5347, 5284 à 5483, 5420 à 5619, 7377 à 7576, 13517 à 13716, 13552 à 13751, 13633 à 13832, 14697 à 14896, 14760 à 14959, 14881 à 15080, 21139 à 21338, 21207 à 21406, 22796 à 22995, 32378 à 32577, 33003 à 33202, 34481 à 34680, 34869 à 35068, 34891 à 35090, 36584 à 36783, 36634 à 36833, ou 36639 à 36838 dudit gène *XPB.*

25. Vecteur selon l'une quelconque des revendications 19 à 22, dans lequel ladite construction de ciblage comprend un fragment du gène *XPC* qui est capable de réparer un clivage dans le gène *XPC* et qui comprend au moins les nucléotides des positions 105 à 304, 5704 à 5903, 7973 à 8172, 9887 à 10086, 10173 à 10372, 11263 à 11462, 13051 à 13250, 13432 à 13631, 18619 à 18818, 19580 à 19779, 20303 à 20502, 20349 à 20548, 20389 à 20588, 21985 à 22184, 21990 à 22189, 22028 à 22227, 22102 à 22301, 26017 à 26216, 29566 à 29765, 29726 à 29925, 30416 à 30615, 31166 à 31365, ou 32317 à 32516 dudit gène *XPC.*

26. Vecteur selon l'une quelconque des revendications 19 à 22, dans lequel ladite construction de ciblage comprend un fragment du gène *XPD* qui est capable de réparer un clivage dans le gène *XPD* et qui comprend au moins les nucléotides des positions -87 à 112, 812 à 1011, 1319 à 1518, 1324 à 1523, 1426 à 1625, 1717 à 1916, 1867 à 2066, 5473 à 5672, 5585 à 5784, 5637 à 5836, 5920 à 6119, 6050 à 6249, 6290 à 6489, 6392 à 6591, 6472 à 6671, 6581 à 6780, 8830 à 9029, 8943 à 9142, 12661 à 12860, 12991 à 13190, 13084 à 13283, 14614 à 14813, 14817 à 15016, 15528 à 15727, 15878 à 16077, 15936 à 16135, 17023 à 17222, 17350 à 17549, 17365 à 17564, 17572 à 17771, 18347 à 18546, 18370 à 18569, ou 18641 à 18840 dudit gène *XPD.*

27. Vecteur selon l'une quelconque des revendications 19 à 22, dans lequel ladite construction de ciblage comprend un fragment du gène *XPE* qui est capable de réparer un clivage dans le gène *XPE* et qui comprend au moins les nucléotides des positions :
10-209, 1295-1494, 2899-3098, 3488-3687, 3616-3815, 6093-6292, 6194-6393, 7034-7233, 7653-7852, 8753-8952, 9781-9980, 9966-10165, 10511-10710, 10665-10864, 11534-11733, 16439-16638, 16667-16866, 18268-18647, 18757-18956, 18863-19062, 19179-19378, 19266-19465, 19596-19795, 20714-20913, 20938-21137, 21099-21298, 22568-22767, 22732-22931, 23173-23372, 23181-23380, 23954-24153, 24000-24199, 29205-29404, 29651-29850, 30280-30479, 30355-30554, 30661-30860, 30685-30884, 32150-32349, 32753-32592, 32770-32969, 32811-33010, 32836-33035, 32841-33040, 33230-33429, 33369-33568, ou 33512-33711 dudit gène *XPE.*

28. Vecteur selon l'une quelconque des revendications 19 à 22, dans lequel ladite construction de ciblage comprend un fragment du gène *XPF* qui est capable de réparer un clivage dans le gène *XPF* et qui comprend au moins les nucléotides des positions :
244-443, 1731-1930, 6429-6628, 6486-6685, 7918-8117, 10500-10699, 10676-10875, 11885-12084, 11886-12085, 12176-12375, 14073-14272, 14110-14309, 15336-15535, 15431-15630, 15574-15773, 17673-17872, 17677-17876, 24486, 24685, 27496-27695, 27822-28021, 27827-28026,ou 27963-28162 dudit gène *XPF*.

29. Vecteur selon l'une quelconque des revendications 19 à 22, dans lequel ladite construction de ciblage comprend un fragment du gène *XPG* qui est capable de réparer un clivage dans le gène *XPG* et qui comprend au moins les positions 158-357, 5956-6155, 7961-7890, 8044-8243, 9977-10176, 12201-12400, 12289-12488, 15230-15429, 15803-16002, 16041-16240, 16146-16345, 16174-16373, 16174-16373, 16394-16593, 16553-16732, 16887-17086, 19487-19686, 19727-19926, 19782-19981, 20207-20406, 20500-20699, 21890-22089, 22117-22316, 25876-26075, 26450-26649, 26832-27031, 27258-27457, 29180-29379, ou 29456-29655 dudit gène *XPG.*

30. Vecteur selon les revendications 20 ou 22, dans lequel la séquence qui répare ladite mutation est flanquée par un fragment du gène de la xérodermie pigmentaire tel que défini dans l'une quelconque des revendications 23 à 29.

31. Vecteur selon la revendication 20 ou 21, dans lequel la séquence qui répare ladite mutation est incluse dans ledit fragment du gène de la xérodermie pigmentaire tel que défini dans l'une quelconque des revendications 23 à 29.

32. Composition comprenant au moins un variant selon l'une quelconque des revendications 1 à 14, une méganucléase chimérique à chaîne unique selon la revendication 15 et/ou au moins un vecteur d'expression selon l'une quelconque des revendications 17 à 31.

33. Composition selon la revendication 32, qui comprend une construction d'ADN de ciblage comprenant une séquence qui répare une mutation dans un gène de la xérodermie pigmentaire humaine, flanquée de séquences homologues de la région dudit gène entourant l'une des séquences cibles SEQ ID N° 1 à 24 et 45 à 216.

34. Composition selon la revendication 33, dans laquelle ladite construction d'ADN de ciblage est incluse dans un vecteur recombinant.

35. Produits contenant un vecteur d'expression selon la revendication 17 ou 18 et un vecteur qui comprend une construction de ciblage telle que définie dans l'une quelconque des revendications 19 à 31, en tant que préparation combinée pour une utilisation simultanée, séparée ou séquentielle dans le but de prévenir, d'améliorer ou de guérir une maladie associée à la xérodermie pigmentaire chez un individu qui en a besoin.

36. Utilisation d'au moins un variant selon l'une quelconque des revendications 1 à 14, d'une méganucléase chimère monocaténaire selon la revendication 15 et/ou d'un vecteur d'expression selon l'une quelconque des revendications 19 à 31 pour la préparation d'un médicament destiné à la prévention, à l'amélioration ou à la guérison d'une maladie associée à la xérodermie pigmentaire chez un individu qui en a besoin.

37. Cellule hôte comprenant un polynucléotide selon la revendication 16 ou un vecteur selon l'une quelconque des revendications 19 à 31.

38. Animal transgénique non humain comprenant un ou plusieurs fragments polynucléotidiques tels que définis dans la revendication 16 ou la revendication 18.

39. Plante transgénique comprenant un ou plusieurs fragments polynucléotidiques tels que définis dans la revendication 16 ou la revendication 18.

40. Utilisation d'au moins un variant selon l'une quelconque des revendications 1 à 14, d'une méganucléase chimérique à chaîne unique selon la revendication 15, d'un vecteur selon l'une quelconque des revendications 17 à 31, pour la manipulation génétique du génome, à des fins non thérapeutiques.

41. Utilisation selon la revendication 40, pour laquelle ledit variant, ladite méganucléase chimérique à chaîne unique, ledit vecteur, est associé à une construction d'ADN de ciblage telle que définie dans l'une quelconque des revendications 19 à 31.
